(19) Europäisches Patentamt
European Patent Office
Office européen des brevets

(11) EP 4 581 641 B1

(12) **EUROPÄISCHE PATENTSCHRIFT**

(45) Veröffentlichungstag und Bekanntmachung des Hinweises auf die Patenterteilung:
**06.05.2026 Patentblatt 2026/19**

(21) Anmeldenummer: **23758344.8**

(22) Anmeldetag: **23.08.2023**

(51) Internationale Patentklassifikation (IPC):
***G16H 30/40*** *(2018.01)* ***G16H 50/20*** *(2018.01)*
***G16H 50/70*** *(2018.01)*

(52) Gemeinsame Patentklassifikation (CPC):
**G16H 30/40; G16H 50/20; G16H 50/70**

(86) Internationale Anmeldenummer:
**PCT/EP2023/073103**

(87) Internationale Veröffentlichungsnummer:
**WO 2024/046832 (07.03.2024 Gazette 2024/10)**

(54) **ERZEUGEN VON SYNTHETISCHEN RADIOLOGISCHEN AUFNAHMEN**

GENERATION OF SYNTHETIC RADIOLOGICAL RECORDINGS

PRODUCTION D'ENREGISTREMENTS RADIOLOGIQUES SYNTHÉTIQUES

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **30.08.2022 EP 22192907**
**01.03.2023 EP 23159289**

(43) Veröffentlichungstag der Anmeldung:
**09.07.2025 Patentblatt 2025/28**

(73) Patentinhaber: **Bayer Aktiengesellschaft**
**51373 Leverkusen (DE)**

(72) Erfinder:
- **LENGA, Matthias**
  **51373 Leverkusen (DE)**
- **BALTRUSCHAT, Ivo Matteo**
  **51373 Leverkusen (DE)**
- **KREIS, Felix Karl**
  **51373 Leverkusen (DE)**

(74) Vertreter: **BIP Patents**
**c/o Bayer Intellectual Property GmbH**
**Alfred-Nobel-Straße 50**
**40789 Monheim am Rhein (DE)**

(56) Entgegenhaltungen:
**EP-A1- 3 875 979** **WO-A1-2021/197996**
**US-B2- 10 997 716**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

TECHNISCHES GEBIET

Die vorliegende Offenbarung betrifft das technische Gebiet der Radiologie, insbesondere der Unterstützung von Radiologen bei radiologischen Untersuchungen mit Methoden der künstlichen Intelligenz. Die vorliegende Erfindung befasst sich mit dem Trainieren eines Modells des maschinellen Lernens und der Nutzung des trainierten Modells zur Vorhersage einer synthetischen Repräsentation eines Untersuchungsbereichs eines Untersuchungsobjekts.

EINLEITUNG

WO2021/197996A1 offenbart ein Verfahren zur Erzeugung radiologischer synthetischer Repräsentationen eines Untersuchungsbereichs eines Untersuchungsobjekts. Auf der Grundlage gemessener radiologischer Aufnahmen eines Untersuchungsbereichs, die Blutgefäße im Untersuchungsbereich mit zeitlich abnehmender Kontrastintensität zeigen, erzeugt das Verfahren synthetische radiologische Aufnahmen des Untersuchungsbereichs, die Blutgefäße mit konstanter Kontrastintensität zeigen.

US 10997716B2 offenbart ein Verfahren zur diagnostischen Bildgebung mit reduzierter Kontrastmittelgabe. Das Verfahren verwendet ein Deep-Learning-Netzwerk, das mit kontrastfreien und kontrastarmen Bildern als Eingabe für das Deep-Learning-Netzwerk und kontrastreichen Bildern als Referenz-Grundwahrheitsbilder trainiert wurde. Das trainierte Deep-Learning-Netzwerk wird dann verwendet, um aus den aufgenommenen Bildern ohne Kontrastmittel und mit geringer Kontrastmittelgabe ein synthetisches Bild mit voller Kontrastmittelgabe vorherzusagen.

EP3875979A1 offenbart ein Verfahren, eine Vorrichtung, ein System und ein Computerprogrammprodukt zur Ermittlung eines optimierten Zeitpunktes zum Starten der Aufnahme und Erfassung einer MRT-Aufnahme nach der Applikation eines Kontrastmittels.

Die zeitliche Verfolgung von Vorgängen innerhalb des Körpers eines Menschen oder eines Tieres mit bildgebenden Verfahren spielt unter anderem bei der Diagnose und/oder Therapie von Krankheiten eine wichtige Rolle.

Als ein Beispiel sei die Detektion und Differentialdiagnose fokaler Leberläsionen mittels dynamischer kontrastverstärkender Magnetresonanztomographie (MRT) mit einem hepatobiliären Kontrastmittel genannt.

Ein hepatobiliäres Kontrastmittel wie beispielsweise Primovist® kann zur Detektion von Tumoren in der Leber eingesetzt werden. Die Blutversorgung des gesunden Lebergewebes erfolgt in erster Linie über die Pfortader (Vena portae), während die Leberarterie (Arteria hepatica) die meisten Primärtumoren versorgt. Nach intravenöser Bolusinjektion eines Kontrastmittels lässt sich dementsprechend eine Zeitverzögerung zwischen der Signalanhebung des gesunden Leberparenchyms und des Tumors beobachten.

Neben malignen Tumoren findet man in der Leber häufig gutartige Läsionen wie Zysten, Hämangiome und fokal noduläre Hyperplasien (FNH). Für eine sachgerechte Therapieplanung müssen diese von den malignen Tumoren differenziert werden. Primovist® kann zur Erkennung gutartiger und bösartiger fokaler Leberläsionen eingesetzt werden. Es liefert mittels T1-gewichteter MRT Informationen über den Charakter dieser Läsionen. Bei der Differenzierung nutzt man die unterschiedliche Blutversorgung von Leber und Tumor und den zeitlichen Verlauf der Kontrastverstärkung.

Bei der durch Primovist® erzielten Kontrastverstärkung während der Anflutungsphase beobachtet man typische Perfusionsmuster, die Informationen für die Charakterisierung der Läsionen liefern. Die Darstellung der Vaskularisierung hilft, die Läsionstypen zu charakterisieren und den räumlichen Zusammenhang zwischen Tumor und Blutgefäßen zu bestimmen.

Bei T1-gewichteten MRT-Aufnahmen führt Primovist® 10-20 Minuten nach der Injektion (in der hepatobiliären Phase) zu einer deutlichen Signalverstärkung im gesunden Leberparenchym, während Läsionen, die keine oder nur wenige Hepatozyten enthalten, z.B. Metastasen oder mittelgradig bis schlecht differenzierte hepatozelluläre Karzinome (HCCs), als dunklere Bereiche auftreten.

Die zeitliche Verfolgung der Verteilung des Kontrastmittels bietet also eine gute Möglichkeit der Detektion und Differentialdiagnose fokaler Leberläsionen; allerdings zieht sich die Untersuchung über eine vergleichsweise lange Zeitspanne hin. Über diese Zeitspanne sollten Bewegungen des Patienten weitgehend vermieden werden, um Bewegungsartefakte in den MRT-Aufnahmen zu minimieren. Die lang andauernde Bewegungseinschränkung kann für einen Patienten unangenehm sein.

In der Offenlegungsschrift WO2021/052896A1 wird vorgeschlagen, eine oder mehrere MRT-Aufnahmen während der hepatobiliären Phase nicht messtechnisch zu erzeugen, sondern auf Basis von MRT-Aufnahmen aus einer oder mehreren vorangegangenen Phasen zu berechnen (vorherzusagen), um die Aufenthaltsdauer des Patienten im MRT-Scanner zu verkürzen.

In dem in der Offenlegungsschrift WO2021/052896A1 beschriebenen Ansatz wird ein Modell des maschinellen Lernens trainiert, auf Basis von MRT-Aufnahmen eines Untersuchungsbereichs vor und/oder unmittelbar nach der Applikation eines Kontrastmittels eine MRT-Aufnahme des Untersuchungsbereichs zu einem späteren Zeitpunkt vorherzusagen.

Es hat sich gezeigt, dass die so vorhergesagten radiologischen Aufnahmen Artefakte aufweisen können. Es kann sein, dass feine Strukturen nicht richtig wiedergegeben werden oder dass Strukturen in Bereichen der synthetischen radiologischen Aufnahme auftreten, die das repräsentierte Gewebe nicht aufweist (siehe z.B.: K. Schwarz et al.: On the Frequency Bias of Generative Models, https://doi.org/10.48550/arXiv.2111.02447).

ZUSAMMENFASSUNG

Diesem und weiteren Problemen widmet sich die vorliegende Offenbarung.

Ein erster Gegenstand der vorliegenden Offenbarung ist ein computer-implementiertes Verfahren (Vorhersageverfahren) zum Erzeugen einer synthetischen radiologischen Aufnahme mit Hilfe des trainierten Modells des maschinellen Lernens. Das Vorhersageverfahren umfasst:

- Bereitstellen eines trainieren Modells des maschinellen Lernens,

    o wobei das trainierte Modell des maschinellen Lernens anhand von Trainingsdaten trainiert worden ist, auf Basis mindestens einer Eingabe-Repräsentation eines Untersuchungsbereichs eines Untersuchungsobjekts eine synthetische Repräsentation des Untersuchungsbereichs des Untersuchungsobjekts zu erzeugen,

    o wobei die Trainingsdaten für jedes Untersuchungsobjekt einer Vielzahl von Untersuchungsobjekten i) mindestens eine Eingabe-Repräsentation des Untersuchungsbereichs des Untersuchungsobjekts, ii) eine Ziel-Repräsentation des Untersuchungsbereichs des Untersuchungsobjekts und iii) eine transformierte Ziel-Repräsentation umfassen,

        ▪ wobei die mindestens eine Eingabe-Repräsentation den Untersuchungsbereich des jeweiligen Untersuchungsobjekts in einer ersten Zeitspanne vor oder nach einer Applikation eines Kontrastmittels repräsentiert,

        ▪ wobei die Ziel-Repräsentation den Untersuchungsbereich des jeweiligen Untersuchungsobjekts in einer zweiten Zeitspanne nach der Applikation des Kontrastmittels repräsentiert, wobei zweite Zeitspanne der ersten Zeitspanne zeitlich nachgelagert ist,

        ▪ wobei die transformierte Ziel-Repräsentation zumindest einen Teil des Untersuchungsbereichs des jeweiligen Untersuchungsobjekts im Frequenzraum repräsentiert, falls die Ziel-Repräsentation den Untersuchungsbereichs des jeweiligen Untersuchungsobjekts im Ortsraum repräsentiert, oder im Ortsraum repräsentiert, falls die Ziel-Repräsentation den Untersuchungsbereich des jeweiligen Untersuchungsobjekts im Frequenzraum repräsentiert,

    o wobei das Trainieren des Modells des maschinellen Lernens ein Reduzieren von Abweichungen zwischen i) zumindest einem Teil der synthetischen Repräsentation und zumindest einem Teil der Ziel-Repräsentation und ii) zwischen zumindest einem Teil einer transformierten synthetischen Repräsentation und zumindest einem Teil der transformierten Ziel-Repräsentation umfasst,

- Empfangen mindestens einer Eingabe-Repräsentation des Untersuchungsbereichs eines neuen Untersuchungsobjekts, wobei die mindestens eine Eingabe-Repräsentation des Untersuchungsbereichs des neuen Untersuchungsobjekts den Untersuchungsbereich in einer ersten Zeitspanne vor und/oder nach einer Applikation eines Kontrastmittels repräsentiert,

- Eingeben der mindestens einen Eingabe-Repräsentation des Untersuchungsbereichs des neuen Untersuchungsobjekts in das trainierte Modell des maschinellen Lernens,

- Empfangen einer synthetischen Repräsentation des Untersuchungsbereichs des neuen Untersuchungsobjekts von dem Modell des maschinellen Lernens,

- Ausgeben und/oder Speichern der synthetischen Repräsentation des Untersuchungsbereichs des neuen Untersuchungsobjekts und/oder Übermitteln der synthetischen Repräsentation des Untersuchungsbereichs des neuen Untersuchungsobjekts an ein separates Computersystem.

Ein weiterer Gegenstand der vorliegenden Offenbarung ist ein Computersystem umfassend

- eine Empfangseinheit,
- eine Steuer- und Recheneinheit und
- eine Ausgabeeinheit,

  - wobei die Steuer- und Recheneinheit konfiguriert ist, ein trainiertes Modell des maschinellen Lernens bereitzustellen,

    o wobei das trainierte Modell des maschinellen Lernens anhand von Trainingsdaten trainiert worden ist, auf Basis mindestens einer Eingabe-Repräsentation eines Untersuchungsbereichs eines Untersuchungsobjekts eine synthetische Repräsentation des Untersuchungsbereichs des Untersuchungsobjekts zu erzeugen,

    o wobei die Trainingsdaten für jedes Untersuchungsobjekt einer Vielzahl von Untersuchungsobjekten i) mindestens eine Eingabe-Repräsentation des Untersuchungsbereichs des Untersuchungsobjekts, ii) eine Ziel-Repräsentation des Untersuchungsbereichs des Untersuchungsobjekts und iii) eine transformierte Ziel-Repräsentation umfassen,

      ▪ wobei die mindestens eine Eingabe-Repräsentation den Untersuchungsbereich des jeweiligen Untersuchungsobjekts in einer ersten Zeitspanne vor oder nach einer Applikation eines Kontrastmittels repräsentiert,

      ▪ wobei die Ziel-Repräsentation den Untersuchungsbereich des jeweiligen Untersuchungsobjekts in einer zweiten Zeitspanne nach der Applikation des Kontrastmittels repräsentiert, wobei zweite Zeitspanne der ersten Zeitspanne zeitlich nachgelagert ist,

      ▪ wobei die transformierte Ziel-Repräsentation zumindest einen Teil des Untersuchungsbereichs des jeweiligen Untersuchungsobjekts im Frequenzraum repräsentiert, falls die Ziel-Repräsentation den Untersuchungsbereichs des jeweiligen Untersuchungsobjekts im Ortsraum repräsentiert, oder im Ortsraum repräsentiert, falls die Ziel-Repräsentation den Untersuchungsbereich des jeweiligen Untersuchungsobjekts im Frequenzraum repräsentiert,

    o wobei das Trainieren des Modells des maschinellen Lernens ein Reduzieren von Abweichungen zwischen i) zumindest einem Teil der synthetischen Repräsentation und zumindest einem Teil der Ziel-Repräsentation und ii) zwischen zumindest einem Teil einer transformierten synthetischen Repräsentation und zumindest einem Teil der transformierten Ziel-Repräsentation umfasst,

  - wobei die Steuer- und Recheneinheit konfiguriert ist, die Empfangseinheit zu veranlassen, mindestens eine Eingabe-Repräsentation eines Untersuchungsbereichs eines neuen Untersuchungsobjekts zu empfangen, wobei die mindestens eine Eingabe-Repräsentation des Untersuchungsbereichs des neuen Untersuchungsobjekts den Untersuchungsbereich in einer ersten Zeitspanne vor und/oder nach einer Applikation eines Kontrastmittels repräsentiert,

  - wobei die Steuer- und Recheneinheit konfiguriert ist, die mindestens eine Eingabe-Repräsentation des Untersuchungsbereichs des neuen Untersuchungsobjekts in ein trainiertes Modell des maschinellen Lernens einzugeben,

  - wobei die Steuer- und Recheneinheit konfiguriert ist, von dem Modell des maschinellen Lernens eine synthetische Repräsentation des Untersuchungsbereichs des neuen Untersuchungsobjekts zu empfangen,

  - wobei die Steuer- und Recheneinheit konfiguriert ist, die Ausgabeeinheit zu veranlassen, die synthetische Repräsentation des Untersuchungsbereichs des neuen Untersuchungsobjekts auszugeben und/oder zu speichern und/oder an ein separates Computersystem zu übermitteln.

Ein weiterer Gegenstand der vorliegenden Offenbarung ist ein Computerprogrammprodukt umfassend ein Computerprogramm, das in einen Arbeitsspeicher eines Computersystems geladen werden kann und dort das Computersystem dazu veranlasst, folgende Schritte ausführen:

- Bereitstellen eines trainieren Modells des maschinellen Lernens,

  o wobei das trainierte Modell des maschinellen Lernens anhand von Trainingsdaten trainiert worden ist, auf Basis mindestens einer Eingabe-Repräsentation eines Untersuchungsbereichs eines Untersuchungsobjekts eine synthetische Repräsentation des Untersuchungsbereichs des Untersuchungsobjekts zu erzeugen,

  o wobei die Trainingsdaten für jedes Untersuchungsobjekt einer Vielzahl von Untersuchungsobjekten i) mindestens eine Eingabe-Repräsentation des Untersuchungsbereichs des Untersuchungsobjekts, ii) eine Ziel-Repräsentation des Untersuchungsbereichs des Untersuchungsobjekts und iii) eine transformierte Ziel-Repräsentation umfassen,

    ▪ wobei die mindestens eine Eingabe-Repräsentation den Untersuchungsbereich des jeweiligen Untersuchungsobjekts in einer ersten Zeitspanne vor oder nach einer Applikation eines Kontrastmittels repräsentiert,

    ▪ wobei die Ziel-Repräsentation den Untersuchungsbereich des jeweiligen Untersuchungsobjekts in einer zweiten Zeitspanne nach der Applikation des Kontrastmittels repräsentiert, wobei zweite Zeitspanne der ersten Zeitspanne zeitlich nachgelagert ist,

    ▪ wobei die transformierte Ziel-Repräsentation zumindest einen Teil des Untersuchungsbereichs des jeweiligen Untersuchungsobjekts im Frequenzraum repräsentiert, falls die Ziel-Repräsentation den Untersuchungsbereichs des jeweiligen Untersuchungsobjekts im Ortsraum repräsentiert, oder im Ortsraum repräsentiert, falls die Ziel-Repräsentation den Untersuchungsbereich des jeweiligen Untersuchungsobjekts im Frequenzraum repräsentiert,

  o wobei das Trainieren des Modells des maschinellen Lernens ein Reduzieren von Abweichungen zwischen i) zumindest einem Teil der synthetischen Repräsentation und zumindest einem Teil der Ziel-Repräsentation und ii) zwischen zumindest einem Teil einer transformierten synthetischen Repräsentation und zumindest einem Teil der transformierten Ziel-Repräsentation umfasst,

- Empfangen mindestens einer Eingabe-Repräsentation des Untersuchungsbereichs eines neuen Untersuchungsobjekts, wobei die mindestens eine Eingabe-Repräsentation des Untersuchungsbereichs des neuen Untersuchungsobjekts den Untersuchungsbereich in einer ersten Zeitspanne vor und/oder nach einer Applikation eines Kontrastmittels repräsentiert,

- Eingeben der mindestens einen Eingabe-Repräsentation des Untersuchungsbereichs des neuen Untersuchungsobjekts in das trainierte Modell des maschinellen Lernens,

- Empfangen einer synthetischen Repräsentation des Untersuchungsbereichs des neuen Untersuchungsobjekts von dem Modell des maschinellen Lernens,

- Ausgeben und/oder Speichern der synthetischen Repräsentation des Untersuchungsbereichs des neuen Untersuchungsobjekts und/oder Übermitteln der synthetischen Repräsentation des Untersuchungsbereichs des neuen Untersuchungsobjekts an ein separates Computersystem.

Ein weiterer Gegenstand der vorliegenden Offenbarung ist eine Verwendung eines Kontrastmittels in einem radiologischen Untersuchungsverfahren, wobei das radiologische Untersuchungsverfahren umfasst:

- Bereitstellen eines trainieren Modells des maschinellen Lernens,

  ◦ wobei das trainierte Modell des maschinellen Lernens anhand von Trainingsdaten trainiert worden ist, auf Basis mindestens einer Eingabe-Repräsentation eines Untersuchungsbereichs eines Untersuchungsobjekts eine synthetische Repräsentation des Untersuchungsbereichs des Untersuchungsobjekts zu erzeugen,

  ◦ wobei die Trainingsdaten für jedes Untersuchungsobjekt einer Vielzahl von Untersuchungsobjekten i) mindestens eine Eingabe-Repräsentation des Untersuchungsbereichs des Untersuchungsobjekts, ii) eine Ziel-Repräsentation des Untersuchungsbereichs des Untersuchungsobjekts und iii) eine transformierte Ziel-Repräsentation umfassen,

▪ wobei die mindestens eine Eingabe-Repräsentation den Untersuchungsbereich des jeweiligen Untersuchungsobjekts in einer ersten Zeitspanne vor oder nach einer Applikation des Kontrastmittels repräsentiert,

▪ wobei die Ziel-Repräsentation den Untersuchungsbereich des jeweiligen Untersuchungsobjekts in einer zweiten Zeitspanne nach der Applikation des Kontrastmittels repräsentiert, wobei zweite Zeitspanne der ersten Zeitspanne zeitlich nachgelagert ist,

▪ wobei die transformierte Ziel-Repräsentation zumindest einen Teil des Untersuchungsbereichs des jeweiligen Untersuchungsobjekts im Frequenzraum repräsentiert, falls die Ziel-Repräsentation den Untersuchungsbereichs des jeweiligen Untersuchungsobjekts im Ortsraum repräsentiert, oder im Ortsraum repräsentiert, falls die Ziel-Repräsentation den Untersuchungsbereich des jeweiligen Untersuchungsobjekts im Frequenzraum repräsentiert,

◦ wobei das Trainieren des Modells des maschinellen Lernens ein Reduzieren von Abweichungen zwischen i) zumindest einem Teil der synthetischen Repräsentation und zumindest einem Teil der Ziel-Repräsentation und ii) zwischen zumindest einem Teil einer transformierten synthetischen Repräsentation und zumindest einem Teil der transformierten Ziel-Repräsentation umfasst,

- Empfangen mindestens einer Eingabe-Repräsentation des Untersuchungsbereichs eines neuen Untersuchungsobjekts, wobei die mindestens eine Eingabe-Repräsentation des Untersuchungsbereichs des neuen Untersuchungsobjekts den Untersuchungsbereich in einer ersten Zeitspanne vor und/oder nach einer Applikation eines Kontrastmittels repräsentiert,

- Eingeben der mindestens einen Eingabe-Repräsentation des Untersuchungsbereichs des neuen Untersuchungsobjekts in das trainierte Modell des maschinellen Lernens,

- Empfangen einer synthetischen Repräsentation des Untersuchungsbereichs des neuen Untersuchungsobjekts von dem Modell des maschinellen Lernens,

- Ausgeben und/oder Speichern der synthetischen Repräsentation des Untersuchungsbereichs des neuen Untersuchungsobjekts und/oder Übermitteln der synthetischen Repräsentation des Untersuchungsbereichs des neuen Untersuchungsobjekts an ein separates Computersystem.

Ein weiterer Gegenstand der vorliegenden Offenbarung ist ein Kit umfassend ein Kontrastmittel und ein Computerprogrammprodukt umfassend ein Computerprogramm, das in einen Arbeitsspeicher eines Computersystems geladen werden kann und dort das Computersystem dazu veranlasst, folgende Schritte ausführen:

- Bereitstellen eines trainieren Modells des maschinellen Lernens,

◦ wobei das trainierte Modell des maschinellen Lernens anhand von Trainingsdaten trainiert worden ist, auf Basis mindestens einer Eingabe-Repräsentation eines Untersuchungsbereichs eines Untersuchungsobjekts eine synthetische Repräsentation des Untersuchungsbereichs des Untersuchungsobjekts zu erzeugen,

◦ wobei die Trainingsdaten für jedes Untersuchungsobjekt einer Vielzahl von Untersuchungsobjekten i) mindestens eine Eingabe-Repräsentation des Untersuchungsbereichs des Untersuchungsobjekts, ii) eine Ziel-Repräsentation des Untersuchungsbereichs des Untersuchungsobjekts und iii) eine transformierte Ziel-Repräsentation umfassen,

▪ wobei die mindestens ein Eingabe-Repräsentation den Untersuchungsbereich des jeweiligen Untersuchungsobjekts in einer ersten Zeitspanne vor oder nach einer Applikation des Kontrastmittels repräsentiert,

▪ wobei die Ziel-Repräsentation den Untersuchungsbereich des jeweiligen Untersuchungsobjekts in einer zweiten Zeitspanne nach der Applikation des Kontrastmittels repräsentiert, wobei zweite Zeitspanne der ersten Zeitspanne zeitlich nachgelagert ist,

▪ wobei die transformierte Ziel-Repräsentation zumindest einen Teil des Untersuchungsbereichs des

jeweiligen Untersuchungsobjekts im Frequenzraum repräsentiert, falls die Ziel-Repräsentation den Untersuchungsbereichs des jeweiligen Untersuchungsobjekts im Ortsraum repräsentiert, oder im Ortsraum repräsentiert, falls die Ziel-Repräsentation den Untersuchungsbereich des jeweiligen Untersuchungsobjekts im Frequenzraum repräsentiert,

◦ wobei das Trainieren des Modells des maschinellen Lernens ein Reduzieren von Abweichungen zwischen i) zumindest einem Teil der synthetischen Repräsentation und zumindest einem Teil der Ziel-Repräsentation und ii) zwischen zumindest einem Teil einer transformierten synthetischen Repräsentation und zumindest einem Teil der transformierten Ziel-Repräsentation umfasst,

- Empfangen mindestens einer Eingabe-Repräsentation des Untersuchungsbereichs eines neuen Untersuchungsobjekts, wobei die mindestens eine Eingabe-Repräsentation des Untersuchungsbereichs des neuen Untersuchungsobjekts den Untersuchungsbereich in einer ersten Zeitspanne vor und/oder nach einer Applikation eines Kontrastmittels repräsentiert,

- Eingeben der mindestens einen Eingabe-Repräsentation des Untersuchungsbereichs des neuen Untersuchungsobjekts in das trainierte Modell des maschinellen Lernens,

- Empfangen einer synthetischen Repräsentation des Untersuchungsbereichs des neuen Untersuchungsobjekts von dem Modell des maschinellen Lernens,

- Ausgeben und/oder Speichern der synthetischen Repräsentation des Untersuchungsbereichs des neuen Untersuchungsobjekts und/oder Übermitteln der synthetischen Repräsentation des Untersuchungsbereichs des neuen Untersuchungsobjekts an ein separates Computersystem.

Weitere Gegenstände und Ausführungsformen finden sich in der nachfolgenden Beschreibung, den Patentansprüchen und den Zeichnungen.

## KURZE BESCHREIBUNG DER ZEICHNUNGEN

Fig. 1 zeigt beispielhaft und schematisch den Prozess des Trainierens des Modells des maschinellen Lernens.

Fig. 2 zeigt beispielhaft und schematisch eine Verwendung eines trainierten Modells des maschinellen Lernens zur Vorhersage.

Fig. 3 zeigt beispielhaft und schematisch eine weitere Ausführungsform des Trainierens des Modells des maschinellen Lernens.

Fig. 4 zeigt beispielhaft und schematisch eine weitere Ausführungsform des Trainierens des Modells des maschinellen Lernens.

Fig. 5 zeigt beispielhaft und schematisch eine weitere Ausführungsform des Trainierens des Modells des maschinellen Lernens.

Fig. 6 zeigt beispielhaft und schematisch eine weitere Ausführungsform des Trainierens des Modells des maschinellen Lernens.

Fig. 7 zeigt beispielhaft und schematisch eine weitere Ausführungsform des Trainierens des Modells des maschinellen Lernens.

Fig. 8 zeigt beispielhaft und schematisch eine weitere Ausführungsform des Trainierens des Modells des maschinellen Lernens.

Fig. 9 zeigt beispielhaft und schematisch eine weitere Verwendung eines trainierten Modells des maschinellen Lernens zur Vorhersage.

Fig. 10 zeigt beispielhaft und schematisch ein Computersystem gemäß der vorliegenden Offenbarung.

Fig. 11 zeigt beispielhaft und schematisch eine weitere Ausführungsform des Computersystems gemäß der vorliegenden Offenbarung.

Fig. 12 zeigt schematisch in Form eines Ablaufdiagramms eine Ausführungsform des Verfahrens zum Trainieren eines Modells des maschinellen Lernens.

Fig. 13 zeigt schematisch in Form eines Ablaufdiagramms eine Ausführungsform des Verfahrens zum Erzeugen einer synthetischen Repräsentation eines Untersuchungsbereichs eines Untersuchungsobjekts mit Hilfe des trainierten Modells des maschinellen Lernens.

AUSFÜHRLICHE BESCHREIBUNG

Die Erfindung wird nachstehend näher erläutert, ohne zwischen den Erfindungsgegenständen (Vorhersageverfahren, Computersystem, Computerprogrammprodukt, Verwendung, Kit) zu unterscheiden. Vielmehr sollen die folgenden Ausführungen sinngemäß für alle Gegenstände der Erfindung Vorhersageverfahren, Computersystem, Computerprogrammprodukt, Verwendung, Kit) gelten, unabhängig davon, in welchem Zusammenhang sie gemacht werden.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Die Erfindung wird an einigen Stellen in Bezug auf Zeichnungen näher erläutert. Dabei sind in den Zeichnungen konkrete Ausführungsformen mit konkreten Merkmalen und Merkmalskombinationen dargestellt, die in erster Linie der Veranschaulichung dienen; die Erfindung soll nicht so verstanden werden, dass sie auf die in den Zeichnungen dargestellten Merkmale und Merkmalskombinationen beschränkt ist. Ferner sollen Aussagen, die bei der Beschreibung der Zeichnungen in Bezug auf Merkmale und Merkmalskombinationen getroffen werden, allgemein gelten, das heißt auch auf andere Ausführungsformen übertragbar und nicht auf die gezeigten Ausführungsformen beschränkt sein.

Mit Hilfe der vorliegenden Erfindung können Repräsentationen eines Untersuchungsbereichs eines Untersuchungsobjekts vorhergesagt werden. Eine solche vorhergesagte Repräsentation wird in dieser Offenbarung auch als synthetische Repräsentation bezeichnet.

Das "Untersuchungsobjekt" ist üblicherweise ein Lebewesen, vorzugsweise ein Säugetier, ganz besonders bevorzugt ein Mensch.

Der "Untersuchungsbereich" ist ein Teil des Untersuchungsobjekts, zum Beispiel ein Organ oder ein Teil eines Organs wie beispielsweise die Leber, das Gehirn, das Herz, die Niere, die Lunge, der Magen, der Darm, die Bauchspeicheldrüse, die Schilddrüse, die Prostata, die Brust oder ein Teil der genannten Organe oder mehrere Organe oder ein anderer Teil des Körpers.

Der "Untersuchungsbereich" ist ein Teil des Untersuchungsobjekts, zum Beispiel ein Organ oder ein Teil eines Organs wie beispielsweise die Leber, das Gehirn, das Herz, die Niere, die Lunge, der Magen, der Darm, die Bauchspeicheldrüse, die Schilddrüse, die Prostata, die Brust oder ein Teil der genannten Organe oder mehrere Organe oder ein anderer Teil des Körpers.

In einer Ausführungsform umfasst der Untersuchungsbereich eine Leber oder einen Teil einer Leber oder der Untersuchungsbereich ist eine Leber oder ein Teil einer Leber eines Säugetiers, vorzugsweise eines Menschen.

In einer weiteren Ausführungsform umfasst der Untersuchungsbereich ein Gehirn oder einen Teil eines Gehirns oder der Untersuchungsbereich ist ein Gehirn oder ein Teil eines Gehirns eines Säugetiers, vorzugsweise eines Menschen.

In einer weiteren Ausführungsform umfasst der Untersuchungsbereich ein Herz oder ein Teil eines Herzes oder der Untersuchungsbereich ist ein Herz oder ein Teil eines Herzes eines Säugetiers, vorzugsweise eines Menschen.

In einer weiteren Ausführungsform umfasst der Untersuchungsbereich einen Thorax oder einen Teil eines Thorax oder der Untersuchungsbereich ist ein Thorax oder ein Teil eines Thorax eines Säugetiers, vorzugsweise eines Menschen.

In einer weiteren Ausführungsform umfasst der Untersuchungsbereich einen Magen oder einen Teil eines Magens oder der Untersuchungsbereich ist ein Magen oder ein Teil eines Magens eines Säugetiers, vorzugsweise eines Menschen.

In einer weiteren Ausführungsform umfasst der Untersuchungsbereich eine Bauchspeicheldrüse oder einen Teil

einer Bauchspeicheldrüse oder der Untersuchungsbereich ist eine Bauchspeicheldrüse oder ein Teil einer Bauchspeicheldrüse eines Säugetiers, vorzugsweise eines Menschen.

In einer weiteren Ausführungsform umfasst der Untersuchungsbereich eine Niere oder einen Teil einer Niere oder der Untersuchungsbereich ist eine Niere oder ein Teil einer Niere eines Säugetiers, vorzugsweise eines Menschen.

In einer weiteren Ausführungsform umfasst der Untersuchungsbereich einen oder beide Lungenflügel oder einen Teil eines Lungenflügels Säugetiers, vorzugsweise eines Menschen.

In einer weiteren Ausführungsform umfasst der Untersuchungsbereich eine Brust oder einen Teil einer Brust oder der Untersuchungsbereich ist eine Brust oder ein Teil einer Brust eines weiblichen Säugetiers, vorzugsweise eines weiblichen Menschen.

In einer weiteren Ausführungsform umfasst der Untersuchungsbereich eine Prostata oder einen Teil einer Prostata oder der Untersuchungsbereich ist eine Prostata oder ein Teil einer Prostata eines männlichen Säugetiers, vorzugsweise eines männlichen Menschen.

Der Untersuchungsbereich, auch Aufnahmevolumen (engl.: *field of view,* FOV) genannt, stellt insbesondere ein Volumen dar, welches in radiologischen Aufnahmen abgebildet wird. Der Untersuchungsbereich wird typischerweise durch einen Radiologen, beispielsweise auf einer Übersichtsaufnahme (engl.: *localizer)* festgelegt. Selbstverständlich kann der Untersuchungsbereich alternativ oder zusätzlich auch automatisch, beispielsweise auf Grundlage eines ausgewählten Protokolls, festgelegt werden.

Eine "Repräsentation des Untersuchungsbereichs" ist üblicherweise das Ergebnis einer radiologischen Untersuchung.

Die "Radiologie" ist das Teilgebiet der Medizin, das sich mit der Anwendung vorwiegend elektromagnetischer Strahlen und (unter Einbezug etwa der Ultraschalldiagnostik) mechanischer Wellen zu diagnostischen, therapeutischen und/oder wissenschaftlichen Zwecken befasst. Neben Röntgenstrahlen kommen auch andere ionisierende Strahlungen wie Gammastrahlung oder Elektronen zum Einsatz. Da ein wesentlicher Einsatzzweck die Bildgebung ist, werden auch andere bildgebende Verfahren wie die Sonographie und die Magnetresonanztomographie (Kernspintomographie) zur Radiologie gerechnet, obwohl bei diesen Verfahren keine ionisierende Strahlung zum Einsatz kommt. Der Begriff "Radiologie" im Sinne der vorliegenden Erfindung umfasst damit insbesondere die folgenden Untersuchungsmethoden: Computertomographie, Magnetresonanztomographie, Sonographie.

In einer bevorzugten Ausführungsform der vorliegenden Erfindung handelt es sich bei der radiologischen Untersuchung um eine magnetresonanztomographische oder computertomographische Untersuchung. Ganz besonders bevorzugt handelt es sich bei der radiologischen Untersuchung um eine magnetresonanztomographische Untersuchung.

Die Magnetresonanztomographie, abgekürzt MRT oder MRI (engl. MRI: *Magnetic Resonance Imaging*), ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MRT-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten MRT-Daten liegen zunächst als Rohdaten im Frequenzraum vor (so genannte k-Raum-Daten), und können durch anschließende inverse Fourier-Transformation in den Ortsraum (Bildraum) transformiert werden.

Eine Repräsentation des Untersuchungsbereichs im Sinne der vorliegenden Offenbarung kann eine MRT-Aufnahme, ein CT-Aufnahme, ein Ultraschallbild oder dergleichen sein oder die Repräsentation des Untersuchungsbereichs kann aus einer oder mehreren MRT-Aufnahmen, CT-Aufnahmen, Ultraschallbildern oder dergleichen erzeugt werden.

Bei radiologischen Untersuchungen werden häufig Kontrastmittel zur Kontrastverstärkung eingesetzt.

"Kontrastmittel" sind Substanzen oder Gemische von Substanzen, die die Darstellung von Strukturen und Funktionen des Körpers bei radiologischen Untersuchungen verbessern.

In der Computertomographie werden meist iodhaltige Lösungen als Kontrastmittel eingesetzt. In der Magnetresonanztomographie (MRT) werden üblicherweise superparamagnetische Substanzen (z.B. Eisenoxidnanopartikel, superparamagnetische Eisen-Platin-Partikel (SIPPs)) oder paramagnetische Substanzen (z.B. Gadolinium-Chelate, Mangan-Chelate) als Kontrastmittel verwendet. Im Fall der Sonografie werden üblicherweise Flüssigkeiten, die gasgefüllte Mikrobläschen (*microbubbles*) enthalten, intravenös verabreicht. Beispiele für Kontrastmittel sind in der Literatur zu finden (siehe z.B. A. S. L. Jascinth et al.: Contrast Agents in computed tomography: A Review, Journal of Applied Dental and Medical Sciences, 2016, Vol. 2, Issue 2, 143 - 149; H. Lusic et al.: X-ray-Computed Tomography Contrast Agents, Chem. Rev. 2013, 113, 3, 1641-1666; https://www.radiology.wisc.edu/wpcontent/uploads/2017/10/contrast-agents-tuto

rial.pdf, M. R. Nough et al.: Radiographie and magnetic resonances contrast agents: Essentials and tips for safe practices, World J Radiol. 2017 Sep 28; 9(9): 339-349; L. C. Abonyi et al.: Intravascular Contrast Media in Radiography: Historical Development & Review of Risk Factors for Adverse Reactions, South American Journal of Clinical Research, 2016, Vol. 3, Issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362).

MRT-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren. Superparamagnetische führen zu einer überwiegenden T2-Verkürzung, während paramagnetische Kontrastmittel im Wesentlichen zu einer T1-Verkürzung führen. Die Wirkung dieser Kontrastmittel ist indirekt, da das Kontrastmittel selbst kein Signal abgibt, sondern nur die Signalintensität in seiner Umgebung beeinflusst. Ein Beispiel für ein superparamagnetisches Kontrastmittel sind Eisenoxidnanopartikel (SPIO, engl.: *superparamagnetic iron oxide).* Beispiele für paramagnetische Kontrastmittel sind Gadolinium-Chelate wie Gadopentetat-Dimeglumin (Handelsname: Magnevist® u.a.), Gadotersäure (Dotarem®, Dotagita®, Cyclolux®), Gadodiamid (Omniscan®), Gadoteridol (ProHance®), Gadobutrol (Gadovist®) und Gadoxetsäure (Primovist®/Eovist®).

Das radiologische Untersuchungsverfahren kann ein magnetresonanztomographisches Untersuchungsverfahren und das Kontrastmittel ein MRT-Kontrastmittel sein. Das heißt, die mindestens eine Eingabe-Repräsentation kann mindestens eine MRT-Aufnahme und die synthetische Repräsentation kann eine synthetische MRT-Aufnahme sein.

Das radiologische Untersuchungsverfahren kann ein computertomographisches Untersuchungsverfahren und das Kontrastmittel ein CT-Kontrastmittel sein. Das heißt, die mindestens eine Eingabe-Repräsentation kann mindestens eine CT-Aufnahme und die synthetische Repräsentation kann eine synthetische CT-Aufnahme sein.

Es ist auch möglich, dass die mindestens eine Eingabe-Repräsentation mindestens eine MRT-Aufnahme und mindestens eine CT-Aufnahme umfasst.

Es ist auch möglich, dass das radiologische Untersuchungsverfahren ein computertomographisches Untersuchungsverfahren und das Kontrastmittel ein MRT-Kontrastmittel ist.

In einer bevorzugten Ausführungsform ist das Kontrastmittel ein MRT-Kontrastmittel (unabhängig davon, ob es in einem magnetresonanztomographischen Untersuchungsverfahren oder in einem computertomographischen Verfahren verwendet wird).

Bei dem MRT-Kontrastmittel kann es sich um ein extrazelluläres Kontrastmittel handeln. Als extrazelluläre Kontrastmittel werden niedermolekulare, wasserlösliche Verbindungen bezeichnet, die sich nach intravenöser Gabe in den Blutgefäßen und im interstitiellen Raum verteilen. Sie werden nach einer gewissen, vergleichsweise kurzen Zeitspanne der Zirkulation im Blutkreislauf über die Nieren ausgeschieden. Zu den extrazellulären MRT-Kontrastmitteln zählen beispielsweise die Gadolinium-Chelate Gadobutrol (Gadovist®), Gadoteridol (Prohance®), Gadotersäure (Dotarem®), Gadopentetsäure (Magnevist®) und Gadodiamid (Omnican®).

Bei dem MRT-Kontrastmittel kann es sich um ein intrazelluläres Kontrastmittel handeln. Intrazelluläre Kontrastmittel werden zu gewissen Anteilen in die Zellen von Geweben aufgenommen und anschließend wieder ausgeschieden. Intrazelluläre MRT-Kontrastmittel auf der Basis der Gadoxetsäure zeichnen sich beispielsweise dadurch aus, dass sie anteilig spezifisch von Leberzellen, den Hepatozyten, aufgenommen werden, sich im Funktionsgewebe (Parenchym) anreichern und die Kontraste in gesundem Lebergewebe verstärken, bevor sie anschließend über Galle in die Faeces ausgeschieden werden. Beispiele für derartige Kontrastmittel auf der Basis der Gadoxetsäure sind in US 6,039,931A beschrieben; sie sind kommerziell zum Beispiel unter den Markennamen Primovist® und Eovist® erhältlich. Ein weiteres MRT-Kontrastmittel mit einer geringeren Aufnahme in die Hepatozyten ist Gadobenate Dimeglumine (Multihance®).

Gadoxetat-Dinatrium (GD, Primovist®) gehört zur Gruppe der intrazellulären Kontrastmittel. Es ist zur Verwendung in der MRT der Leber zugelassen, um Läsionen bei Patienten mit bekannter oder vermuteter fokaler Lebererkrankung zu erkennen und zu charakterisieren. GD weist mit seiner lipophilen Ethoxybenzyl-Einheit eine zweiphasige Verteilung auf: zunächst eine Verteilung im intravaskulären und interstitiellen Raum nach Bolusinjektion, gefolgt von einer selektiven Aufnahme durch Hepatozyten. GD wird über die Nieren und den hepatobiliären Weg (50:50 dualer Ausscheidungsmechanismus) in etwa gleichen Mengen unverändert aus dem Körper ausgeschieden. GD wird aufgrund seiner selektiven Anreicherung im gesunden Lebergewebe auch als hepatobiliäres Kontrastmittel bezeichnet.

GD ist in einer Dosis von 0,1 ml / kg Körpergewicht (BW) (0,025 mmol / kg BW Gd) zugelassen. Die empfohlene Verabreichung von GD umfasst eine unverdünnte intravenöse Bolusinjektion mit einer Flussrate von ungefähr 2 ml / Sekunde, gefolgt von einer Spülung der i.v. Kanüle mit einer physiologischen Kochsalzlösung. Ein Standardprotokoll für die Leberbildgebung unter Verwendung von GD besteht aus mehreren Planungs- und Vorkontrastsequenzen. Nach i.v. Bolusinjektion des Kontrastmittels, werden üblicherweise dynamische Aufnahmen während der arteriellen (ungefähr 30 Sekunden nach der Injektion, p.i.), portalvenösen (ungefähr 60 Sekunden p.i.) und der Übergangsphase (ungefähr 2-5 Minuten p.i.) aufgenommen. Die Übergangsphase zeigt typischerweise bereits einen gewissen Anstieg der Lebersignalintensität aufgrund der beginnenden Aufnahme des Mittels in Hepatozyten. Zusätzliche T2-gewichtete und diffusionsgewichtete (DWI) Aufnahmen können nach der dynamischen Phase und vor der späten hepatobiliären Phase

erzeugt werden.

In einer Ausführungsform handelt es sich bei dem Kontrastmittel um Gadoxetat-Dinatrium.

In einer Ausführungsform der vorliegenden Offenbarung handelt es sich bei dem Kontrastmittel um ein Mittel, das Gadolinium(III) 2-[4,7,10-tris(carboxymethyl)-1,4,7,10-tetrazacyclododec-1-yl]essigsäure (auch als Gadolinium-DOTA oder Gadotersäure bezeichnet) umfasst.

In einer weiteren Ausführungsform handelt es sich bei dem Kontrastmittel um ein Mittel, das Gadolinium(III) Ethoxybenzyl-diethylenetriaminepentaessigsäure (Gd-EOB-DTPA) umfasst; vorzugsweise umfasst das Kontrastmittel das Dinatriumsalz der Gadolinium(III)-ethoxybenzyl-diethylenetriaminpentaessigsäure (auch als Gadoxetsäure bezeichnet).

In einer Ausführungsform der vorliegenden Offenbarung handelt es sich bei dem Kontrastmittel um ein Mittel, das Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat (auch als Gadopiclenol bezeichnet, siehe z.B. WO2007/042504 sowie WO2020/030618 und/oder WO2022/013454) umfasst.

In einer Ausführungsform der vorliegenden Offenbarung handelt es sich bei dem Kontrastmittel um ein Mittel, das Dihydrogen[(±)-4-carboxy-5,8,11-tris(carboxymethyl)-1-phenyl-2-oxa-5,8,11-triazatridecan-13-oato(5-)]gadolinat(2-) (auch als Gadobensäure bezeichnet) umfasst.

In einer Ausführungsform der vorliegenden Offenbarung handelt es sich bei dem Kontrastmittel um ein Mittel, das Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetat (auch als Gadoquatrane bezeichnet) umfasst (siehe z.B. J. Lohrke et al.: Preclinical Profile of Gadoquatrane: A Novel Tetrameric, Macrocyclic High Relaxivity Gadolinium-Based Contrast Agent. Invest Radiol., 2022, 1, 57(10): 629-638; WO2016193190).

In einer Ausführungsform der vorliegenden Offenbarung handelt es sich bei dem Kontrastmittel um ein Mittel, das einen $Gd^{3+}$-Komplex einer Verbindung der Formel (I)

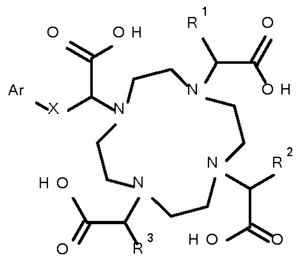

(I) ,

umfasst, wobei

Ar eine Gruppe ausgewählt aus

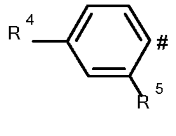

und

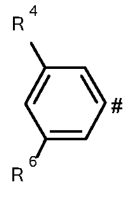

darstellt,

wobei # die Anknüpfung zu X darstellt,

X eine Gruppe darstellt, die aus

$CH_2$, $(CH_2)_2$, $(CH_2)_3$, $(CH_2)_4$ und $*-(CH_2)_2-O-CH_2-^{\#}$ ausgewählt wird,

wobei * die Anknüpfung zu Ar darstellt und $^{\#}$ die Anknüpfung zum Essigsäurerest darstellt,

$R^1$, $R^2$ and $R^3$ unabhängig voneinander ein Wasserstoffatom oder eine Gruppe ausgewählt aus $C_1$-$C_3$-Alkyl, $-CH_2OH$, $-(CH_2)_2OH$ und $-CH_2OCH_3$ darstellen,

$R^4$ eine Gruppe ausgewählt aus $C_2$-$C_4$-Alkoxy, $(H_3C-CH_2)-O-(CH_2)_2-O-$, $(H_3C-CH_2)-O-(CH_2)_2-O-(CH_2)_2-O-$ und $(H_3C-CH_2)-O-(CH_2)_2-O-(CH_2)_2-O-(CH_2)_2-O-$ darstellt,

$R^5$ ein Wasserstoffatom darstellt,
und

$R^6$ ein Wasserstoffatom darstellt,

oder ein Stereoisomer, Tautomer, Hydrat, Solvat oder Salz davon, oder eine Mischung davon.

In einer Ausführungsform der vorliegenden Offenbarung handelt es sich bei dem Kontrastmittel um ein Mittel, das einen $Gd^{3+}$-Komplex einer Verbindung der Formel (II)

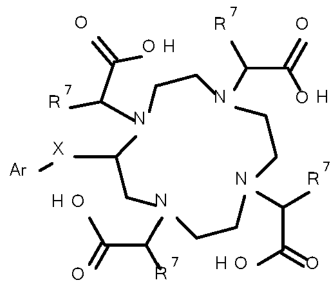

(II) ,

umfasst, wobei

Ar eine Gruppe ausgewählt aus

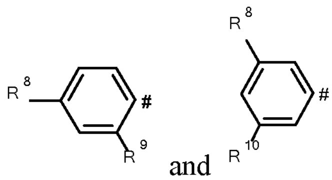

darstellt,

wobei # die Anknüpfung zu X darstellt,

X eine Gruppe darstellt, die aus $CH_2$, $(CH_2)_2$, $(CH_2)_3$, $(CH_2)_4$ and $*-(CH_2)_2-O-CH_2-^{\#}$ ausgewählt wird, wobei * die Anknüpfung zu Ar darstellt und # die Anknüpfung zum Essigsäurerest darstellt,

$R^7$ ein Wasserstoffatom oder eine Gruppe ausgewählt aus $C_1$-$C_3$-Alkyl, $-CH_2OH$, $-(CH_2)_2OH$ und $-CH_2OCH_3$ darstellt;

$R^8$ eine Gruppe ausgewählt aus $C_2$-$C_4$-Alkoxy, $(H_3C-CH_2O)-(CH_2)_2-O-$, $(H_3C-CH_2O)-(CH_2)_2-O-(CH_2)_2-O-$ und $(H_3C-CH_2O)-(CH_2)_2-O-(CH_2)_2-O-(CH_2)_2-O-$ darstellt;

$R^9$ und $R^{10}$ unabhängig voneinander ein Wasserstoffatom darstellen;

oder ein Stereoisomer, Tautomer, Hydrat, Solvat oder Salz davon, oder eine Mischung davon.

Der Begriff "$C_1$-$C_3$-Alkyl" bedeutet eine lineare oder verzweigte, gesättigte, einwertige Kohlenwasserstoffgruppe

mit 1, 2 oder 3 Kohlenstoffatomen, z.B. Methyl, Ethyl, n-Propyl und Isopropyl. Der Begriff "$C_2$-$C_4$-Alkyl" bedeutet eine lineare oder verzweigte, gesättigte, einwertige Kohlenwasserstoffgruppe mit 2, 3 oder 4 Kohlenstoffatomen.

Der Begriff "$C_2$-$C_4$-Alkoxy" bedeutet eine lineare oder verzweigte, gesättigte, einwertige Gruppe der Formel ($C_2$-$C_4$-Alkyl)-O-, in der der Begriff "$C_2$-$C_4$-Alkyl" wie oben definiert ist, z. B. ein Methoxy, Ethoxy, n-Propoxy oder Isopropoxygruppe.

In einer Ausführungsform der vorliegenden Offenbarung handelt es sich bei dem Kontrastmittel um ein Mittel, das Gadolinium 2,2',2"-(10-{1-carboxy-2-[2-(4-ethoxyphenyl)ethoxy]ethyl}-1,4,7,10-tetraazacyclododecan-1,4,7-triyl) triacetat umfasst (siehe z.B. WO2022/194777, Beispiel 1).

In einer Ausführungsform der vorliegenden Offenbarung handelt es sich bei dem Kontrastmittel um ein Mittel, das Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 2).

In einer Ausführungsform der vorliegenden Offenbarung handelt es sich bei dem Kontrastmittel um ein Mittel, das Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 4).

In einer Ausführungsform der vorliegenden Offenbarung handelt es sich bei dem Kontrastmittel um ein Mittel, das Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat) umfasst (siehe z.B. WO2022/194777, Beispiel 15).

In einer Ausführungsform der vorliegenden Offenbarung handelt es sich bei dem Kontrastmittel um ein Mittel, das Gadolinium 2,2',2"-{10-[(1S)-4-(4-butoxyphenyl)-1-carboxybutyl]-1,4,7,10-tetraazacyclododecan-1,4,7-triyl}triacetat umfasst (siehe z.B. WO2022/194777, Beispiel 31).

In einer Ausführungsform der vorliegenden Offenbarung handelt es sich bei dem Kontrastmittel um ein Mittel, das Gadolinium-2,2',2"-{(2S)-10-(carboxymethyl)-2-[4-(2-ethoxyethoxy)benzyl]-1,4,7,10-tetraazacyclododecane-1,4,7-triyl}triacetat umfasst.

In einer Ausführungsform der vorliegenden Offenbarung handelt es sich bei dem Kontrastmittel um ein Mittel, das Gadolinium-2,2',2"-[10-(carboxymethyl)-2-(4-ethoxybenzyl)-1,4,7,10-tetraazacyclododecane-1,4,7-triyl]triacetat umfasst.

In einer Ausführungsform der vorliegenden Offenbarung handelt es sich bei dem Kontrastmittel um ein Mittel, das Gadolinium(III) 5,8-bis(carboxylatomethyl)-2-[2-(methylamino)-2-oxoethyl]-10-oxo-2,5,8,11-tetraazadodecan-1-carboxylat-Hydrat (auch als Gadodiamid bezeichnet) umfasst.

In einer Ausführungsform der vorliegenden Offenbarung handelt es sich bei dem Kontrastmittel um ein Mittel, das Gadolinium(III) 2-[4-(2-hydroxypropyl)-7,10-bis(2-oxido-2-oxoethyl)-1,4,7,10-tetrazacyclododec-1-yl]acetat (auch als Gadoteridol bezeichnet) umfasst.

In einer Ausführungsform der vorliegenden Offenbarung handelt es sich bei dem Kontrastmittel um ein Mittel, das Gadolinium(III) 2,2',2"-(10-((2R,3S)-1,3,4-trihydroxybutan-2-yl)-1,4,7,10-tetraazacyclododecan-1,4,7-triyl)triacetat (auch als Gadobutrol oder Gd-DO3A-butrol bezeichnet) umfasst.

Eine Repräsentation des Untersuchungsbereichs im Sinne der vorliegenden Offenbarung kann eine Repräsentation im Ortsraum (Bildraum) oder eine Repräsentation im Frequenzraum sein.

In einer Repräsentation im Ortsraum, in dieser Beschreibung auch als Ortsraumdarstellung oder Ortsraum-Repräsentation bezeichnet, wird der Untersuchungsbereich üblicherweise durch eine Vielzahl an Bildelementen (Pixeln oder Voxel) repräsentiert, die beispielsweise rasterförmig angeordnet sein können, wobei jedes Bildelement einen Teil des Untersuchungsbereichs repräsentiert. Ein in der Radiologie weit verbreitetes Format zur Speicherung und Verarbeitung von Repräsentationen im Ortsraum ist das DICOM-Format. DICOM (Digital Imaging and Communications in Medicine) ist ein offener Standard zur Speicherung und zum Austausch von Informationen im medizinischen Bilddatenmanagement.

In einer Repräsentation im Frequenzraum, in dieser Beschreibung auch als Frequenzraumdarstellung oder Frequenzraum-Repräsentation bezeichnet, wird der Untersuchungsbereich durch eine Überlagerung von Grundschwingungen repräsentiert. Beispielsweise kann der Untersuchungsbereich durch eine Summe von Sinus- und Kosinus-Funktionen mit verschiedenen Amplituden, Frequenzen und Phasen repräsentiert werden. Die Amplituden und Phasen können als Funktion der Frequenzen beispielsweise in einer zwei- oder dreidimensionalen Darstellung aufgetragen werden. Üblicherweise wird die niedrigste Frequenz (Ursprung) in das Zentrum gelegt. Je weiter man sich von diesem Zentrum entfernt, desto höher sind die Frequenzen. Jeder Frequenz kann eine Amplitude, mit der die Frequenz in der Frequenzraumdarstellung vertreten ist, und eine Phase, die angibt, inwieweit die jeweilige Schwingung gegenüber einer Sinus- oder Kosinus-Schwingung verschoben ist, zugeordnet werden.

Eine Repräsentation im Ortsraum lässt sich beispielsweise durch eine Fourier-Transformation in eine Repräsentation im Frequenzraum überführen (transformieren). Umgekehrt lässt sich eine Repräsentation im Frequenzraum beispielsweise durch eine inverse Fourier-Transformation in eine Repräsentation im Ortsraum überführen (transformieren).

Details über Ortsraumdarstellungen und Frequenzraumdarstellungen und ihre jeweilige Umwandlung ineinander sind in zahlreichen Publikationen beschrieben, siehe z.B.: https://see.stanford.edu/materials/lsoftaee261/book-fall-07.pdf.

Mit Hilfe eines trainierten Modells des maschinellen Lernens kann auf Basis mindestens einer Repräsentation eines Untersuchungsbereichs eines Untersuchungsobjekts eine synthetische Repräsentation des Untersuchungsbereichs des Untersuchungsobjekts erzeugt werden. Die mindestens eine Repräsentation, auf deren Basis das (trainierte) Modell des maschinellen Lernens die synthetische Repräsentation erzeugt, wird in dieser Beschreibung auch als Eingabe-Repräsentation bezeichnet.

Die synthetische Repräsentation repräsentiert üblicherweise denselben Untersuchungsbereich desselben Untersuchungsobjekts wie die mindestens eine Eingabe-Repräsentation, auf deren Erzeugung die synthetische Repräsentation basiert.

Die mindestens eine Eingabe-Repräsentation repräsentiert den Untersuchungsbereich eines Untersuchungsobjekts in einer ersten Zeitspanne vor und/oder nach einer Applikation eines Kontrastmittels. Die synthetische Repräsentation repräsentiert den Untersuchungsbereich in einer zweiten Zeitspanne nach der Applikation des Kontrastmittels.

Die zweite Zeitspanne ist der ersten Zeitspanne zeitlich nachgelagert, d.h. sie ist später als die erste Zeitspanne. Die zweite Zeitspanne kann unmittelbar auf die erste Zeitspanne folgen; es ist aber auch möglich, dass zwischen der ersten Zeitspanne und der zweiten Zeitspanne ein zeitlicher Abstand liegt.

Die mindestens eine Eingabe-Repräsentation kann beispielsweise eine erste Eingabe-Repräsentation des Untersuchungsbereichs umfassen, die den Untersuchungsbereich vor einer Applikation eines Kontrastmittels repräsentiert, und eine zweite Eingabe-Repräsentation umfassen, die den Untersuchungsbereich nach der Applikation eines Kontrastmittels repräsentiert. Die erste Eingabe-Repräsentation und die zweite Eingabe-Repräsentation repräsentieren den Untersuchungsbereich in einer ersten Zeitspanne. Die Zeitspanne beginnt zu einem ersten Zeitpunkt vor der Applikation des Kontrastmittels und endet zu einem zweiten Zeitpunkt nach der Applikation des Kontrastmittels. Die synthetische Repräsentation repräsentiert den Untersuchungsbereich zu einem dritten Zeitpunkt in einer zweiten Zeitspanne nach der Applikation des Kontrastmittels.

Die mindestens eine Eingabe-Repräsentation kann auch eine erste Eingabe-Repräsentation des Untersuchungsbereichs umfassen, die den Untersuchungsbereich zu einem ersten Zeitpunkt in einer Zeitspanne nach einer Applikation eines Kontrastmittels repräsentiert, und eine zweite Eingabe-Repräsentation umfassen, die den Untersuchungsbereich zu einem zweiten Zeitpunkt in der ersten Zeitspanne nach der Applikation des Kontrastmittels repräsentiert. Die synthetische Repräsentation kann den Untersuchungsbereich zu einem dritten Zeitpunkt in einer zweiten Zeitspanne nach der Applikation des Kontrastmittels repräsentieren.

Es ist auch denkbar, dass die mindestens eine Eingabe-Repräsentation mehr als zwei Eingabe-Repräsentationen des Untersuchungsbereichs in einer ersten Zeitspanne vor und/oder nach der Applikation eines Kontrastmittels umfasst, z.B. drei oder vier oder fünf oder eine andere Anzahl. Jede der Eingabe-Repräsentationen kann den Untersuchungsbereich zu einem anderen Zeitpunkt vor und/oder nach der Applikation des Kontrastmittels repräsentieren. Es ist aber auch denkbar, dass zwei oder mehr Eingabe-Repräsentationen den Untersuchungsbereich zu demselben Zeitpunkt repräsentieren.

Es ist auch denkbar, dass die mindestens eine Eingabe-Repräsentation nur eine Eingabe-Repräsentation des Untersuchungsbereichs in einer ersten Zeitspanne vor oder nach der Applikation eines Kontrastmittels ist.

Es ist auch denkbar, dass die mindestens eine Eingabe-Repräsentation den Untersuchungsbereich in einer ersten Zeitspanne vor und/oder nach einer ersten Applikation eines Kontrastmittels und die synthetische Repräsentation den Untersuchungsbereich vor und/oder nach einer zweiten Applikation eines Kontrastmittels repräsentiert. Das Kontrastmittel der ersten Applikation und das Kontrastmittel der zweiten Applikation können gleich oder verschieden sein.

In einer Ausführungsform wird ein Modell des maschinellen Lernens trainiert, eine synthetische Repräsentation einer Leber oder eines Teil einer Leber eines Untersuchungsobjekts in der hepatobiliären Phase einer MRT-Untersuchung auf Basis von mindestens einer Eingabe-Repräsentation vorherzusagen, wobei die mindestens eine Eingabe-Repräsentation die Leber oder den Teil der Leber des Untersuchungsobjekts zu einem oder mehreren früheren Zeitpunkten repräsentiert. Die mindestens eine Eingabe-Repräsentation kann die Leber oder den Teil der Leber beispielsweise vor der Applikation eines hepatobiliären Kontrastmittels und/oder in der arteriellen Phase nach der Applikation des hepatobiliären Kontrastmittels und/oder in der portalvenösen Phasen nach der Applikation des hepatobiliären Kontrastmittels und/oder in der Übergangsphase nach der Applikation des hepatobiliären Kontrastmittels repräsentieren.

Nach der intravenösen Applikation des hepatobiliären Kontrastmittels in Form eines Bolus in eine Armvene erreicht das Kontrastmittel die Leber zunächst über die Arterien. Diese sind in den entsprechenden MRT-Aufnahmen kontrastverstärkt dargestellt. Die Phase, in der die Leberarterien in MRT-Aufnahmen kontrastverstärkt dargestellt sind, wird als "arterielle Phase" bezeichnet.

Anschließend erreicht das Kontrastmittel die Leber über die Lebervenen. Während der Kontrast in den Leberarterien bereits abnimmt, erreicht der Kontrast in den Lebervenen ein Maximum. Die Phase, in der die Lebervenen in MRT-Aufnahmen kontrastverstärkt dargestellt sind, wird als "portalvenöse Phase" bezeichnet. Diese Phase kann bereits während der arteriellen Phase beginnen und sich mit dieser überlagern.

An die portalvenöse Phase schließt sich die "Übergangsphase" (engl. *transitional phase*) an, in der der Kontrast in den Leberarterien weiter absinkt, der Kontrast in den Lebervenen ebenfalls absinkt. Bei Verwendung eines hepatobiliären Kontrastmittels steigt der Kontrast in den gesunden Leberzellen in der Übergangsphase allmählich an.

Die arterielle Phase, die portalvenöse Phase und die Übergangsphase werden zusammenfassend auch als "dynamische Phase" bezeichnet.

10-20 Minuten nach seiner Injektion führt ein hepatobiliäres Kontrastmittel zu einer deutlichen Signalverstärkung im gesunden Leberparenchym. Diese Phase wird als "hepatobiliäre Phase" bezeichnet. Das Kontrastmittel wird aus den Leberzellen nur langsam ausgeschieden; dementsprechend kann die hepatobiliäre Phase zwei Stunden und mehr andauern.

Die genannten Phasen sind beispielsweise in den folgenden Publikationen näher beschrieben: J. Magn. Reson. Imaging, 2012, 35(3): 492-511, doi:10.1002/jmri.22833; Clujul Medical, 2015, Vol. 88 no. 4: 438-448, DOI: 10.15386/cjmed-414; Journal of Hepatology, 2019, Vol. 71: 534-542, http://dx.doi.org/10.1016/j.jhep.2019.05.005).

Dadurch, dass eine Repräsentation der Leber in der hepatobiliären Phase in Form einer synthetischen Repräsentation mittels eines Models des maschinellen erzeugt und nicht gemessen wird, ist die Zeit, die ein Untersuchungsobjekt in einem MRT-Scanner verbringen muss, geringer. Dies gilt analog auch für andere Untersuchungen, die sich über eine Zeitspanne hinziehen. Die mit Hilfe des trainierten Modells des maschinellen Lernens der vorliegenden Offenbarung erzeugten synthetischen Repräsentationen weisen eine höhere Qualität auf als die synthetischen Repräsentationen, die nach dem in WO2021/052896A1 beschriebenen Verfahren erzeugt werden. Zudem erlaubt es der hier beschriebene Ansatz, beim Trainieren des Modells des maschinellen Lernens einen gezielten Fokus auf definierte Frequenzbereiche zu legen und damit zu steuern, welche Informationen die synthetischen Repräsentationen besonders genau darstellen sollen.

Ein "Modell des maschinellen Lernens" kann als eine computerimplementierte Datenverarbeitungsarchitektur verstanden werden. Das Modell kann Eingabedaten empfangen und Ausgabedaten auf der Grundlage dieser Eingabedaten und Modellparametern liefern. Das Modell kann durch Training eine Beziehung zwischen den Eingabedaten und den Ausgabedaten erlernen. Beim Training können Modellparameter angepasst werden, um eine gewünschte Ausgabe für eine bestimmte Eingabe zu liefern.

Beim Trainieren eines solchen Modells werden dem Modell Trainingsdaten präsentiert, aus denen es lernen kann. Das trainierte Modell des maschinellen Lernens ist das Ergebnis des Trainingsprozesses. Die Trainingsdaten umfassen neben Eingabedaten die korrekten Ausgabedaten (Zieldaten), die das Modell auf Basis der Eingabedaten erzeugen soll. Beim Trainieren werden Muster erkannt, die die Eingabedaten auf die Zieldaten abbilden.

Im Trainingsprozess werden die Eingabedaten der Trainingsdaten in das Modell eingegeben, und das Modell erzeugt Ausgabedaten. Die Ausgabedaten werden mit den Zieldaten verglichen. Modellparameter werden so verändert, dass die Abweichungen zwischen den Ausgabedaten und den Zieldaten auf ein (definiertes) Minimum reduziert werden. Zur Modifizierung der Modellparameter im Hinblick auf eine Reduzierung der Abweichungen kann ein Optimierungsverfahren wie beispielsweise ein Gradientenverfahren verwendet werden.

Die Abweichungen können mit Hilfe einer Fehlerfunktion (engl.: *loss function*) quantifiziert werden. Eine solche Fehlerfunktion kann verwendet werden, um einen Fehler (engl.: *loss*) für ein gegebenes Paar von Ausgabedaten und Zieldaten zu berechnen. Das Ziel des Trainingsprozesses kann darin bestehen, die Parameter des Modells des maschinellen Lernens so zu verändern (anzupassen), dass der Fehler für alle Paare des Trainingsdatensatzes auf ein (definiertes) Minimum reduziert wird.

Handelt es sich bei den Ausgabedaten und den Zieldaten beispielsweise um Zahlen, kann die Fehlerfunktion die absolute Differenz zwischen diesen Zahlen sein. In diesem Fall kann ein hoher absoluter Fehler bedeuten, dass ein oder mehrere Modellparameter in hohem Maße geändert werden müssen.

Bei Ausgabedaten in Form von Vektoren können beispielsweise Differenzmetriken zwischen Vektoren wie der mittlere quadratische Fehler, ein Kosinusabstand, eine Norm des Differenzvektors wie ein euklidischer Abstand, ein Tschebyscheff-Abstand, eine Lp-Norm eines Differenzvektors, eine gewichtete Norm oder eine andere Art von Differenzmetrik zweier Vektoren als Fehlerfunktion gewählt werden.

Bei höherdimensionalen Ausgaben, wie z.B. zweidimensionalen, dreidimensionalen oder höherdimensionalen Ausgaben, kann z.B. eine elementweise Differenzmetrik verwendet werden. Alternativ oder zusätzlich können die Ausgabedaten vor der Berechnung eines Fehlerwertes transformiert werden, z.B. in einen eindimensionalen Vektor.

Im vorliegenden Fall wird das Modell des maschinellen Lernens anhand von Trainingsdaten trainiert, eine synthetische Repräsentation eines Untersuchungsbereichs eines Untersuchungsobjekts auf Basis von mindestens einer Eingabe-Repräsentation des Untersuchungsbereichs des Untersuchungsobjekts zu erzeugen.

Die Trainingsdaten umfassen für jedes Untersuchungsobjekt einer Vielzahl von Untersuchungsobjekten einen Satz an Eingabedaten und Zieldaten.

Der Begriff Vielzahl bedeutet mindestens zehn, vorzugsweise mehr als einhundert.

Jeder Satz an Eingabedaten und Zieldaten umfasst mindestens eine Eingabe-Repräsentation eines Untersuchungsbereichs des Untersuchungsobjekts als Eingabedaten. Jeder Satz an Eingabedaten und Zieldaten umfasst

ferner eine Ziel-Repräsentation des Untersuchungsbereichs des Untersuchungsobjekts und eine transformierte Ziel-Repräsentation als Zieldaten.

Der Untersuchungsbereich ist üblicherweise für alle Untersuchungsobjekte der gleiche.

Jede Eingabe-Repräsentation repräsentiert den Untersuchungsbereich des jeweiligen Untersuchungsobjekts in einer ersten Zeitspanne vor oder nach einer Applikation eines Kontrastmittels.

Jede Ziel-Repräsentation repräsentiert den Untersuchungsbereich des jeweiligen Untersuchungsobjekts in einer zweiten Zeitspanne nach der Applikation des Kontrastmittels.

Jede transformierte Ziel-Repräsentation repräsentiert zumindest einen Teil des Untersuchungsbereichs des jeweiligen Untersuchungsobjekts im Frequenzraum, falls die Ziel-Repräsentation den Untersuchungsbereichs des jeweiligen Untersuchungsobjekts im Ortsraum repräsentiert, oder im Ortsraum, falls die Ziel-Repräsentation den Untersuchungsbereich des jeweiligen Untersuchungsobjekts im Frequenzraum repräsentiert.

Mit anderen Worten: für den Fall, dass eine Ziel-Repräsentation den Untersuchungsbereich des Untersuchungsobjekts im Ortsraum repräsentiert, repräsentiert die transformierte Ziel-Repräsentation zumindest einen Teil des Untersuchungsbereichs des Untersuchungsobjekts im Frequenzraum; für den Fall, dass eine Ziel-Repräsentation den Untersuchungsbereich des Untersuchungsobjekts im Frequenzraum repräsentiert, repräsentiert die transformierte Ziel-Repräsentation zumindest einen Teil des Untersuchungsbereichs des Untersuchungsobjekts im Ortsraum.

Eine transformierte Ziel-Repräsentation im Frequenzraum kann aus einer Ziel-Repräsentation im Ortsraum beispielsweise durch Fourier-Transformation erzeugt werden.

Eine transformierte Ziel-Repräsentation im Ortsraum kann aus einer Ziel-Repräsentation im Frequenzraum beispielsweise durch inverse Fourier-Transformation erzeugt werden.

Beim Trainieren des Modells des maschinellen Lernens wird für jedes Untersuchungsobjekt der Vielzahl an Untersuchungsobjekten die mindestens eine Eingabe-Repräsentation des Untersuchungsbereichs dem Modell des maschinellen Lernens zugeführt. Das Modell erzeugt auf Basis der mindestens einen Eingabe-Repräsentation des Untersuchungsbereichs und auf Basis von Modellparametern eine synthetische Repräsentation.

Für den Fall, dass die dem Modell zugeführte mindestens eine Eingabe-Repräsentation den Untersuchungsbereich im Ortsraum repräsentiert, repräsentiert die synthetische Repräsentation den Untersuchungsbereich vorzugsweise (aber nicht notwendigerweise) ebenfalls im Ortsraum.

Für den Fall, dass die dem Modell zugeführte mindestens eine Eingabe-Repräsentation den Untersuchungsbereich im Frequenzraum repräsentiert, repräsentiert die synthetische Repräsentation den Untersuchungsbereich vorzugsweise (aber nicht notwendigerweise) ebenfalls im Frequenzraum.

Das Modell des maschinellen Lernens kann konfiguriert sein und trainiert werden, auf Basis der mindestens einen Eingabe-Repräsentation des Untersuchungsbereichs eine synthetische Repräsentation des Untersuchungsbereichs zu erzeugen, die i) den Untersuchungsbereich im Frequenzraum repräsentiert falls die mindestens eine Repräsentation den Untersuchungsbereich im Ortsraum repräsentiert, oder ii) den Untersuchungsbereich im Ortsraum repräsentiert falls die mindestens eine Repräsentation den Untersuchungsbereich im Frequenzraum repräsentiert. Mit anderen Worten: das Modell des maschinellen Lernens kann konfiguriert sein und trainiert werden, (unter anderem) eine Transformation vom Orts- in den Frequenztraum oder umgekehrt auszuführen.

Von oder zu der synthetischen Repräsentation wird eine transformierte synthetische Repräsentation erzeugt. Für den Fall, dass die synthetische Repräsentation den Untersuchungsbereich im Ortsraum in einer zweiten Zeitspanne nach einer Applikation eines Kontrastmittels repräsentiert, repräsentiert die transformierte synthetische Repräsentation zumindest einen Teil des Untersuchungsbereichs im Frequenzraum in der zweiten Zeitspanne nach der Applikation des Kontrastmittels. Für den Fall, dass die synthetische Repräsentation den Untersuchungsbereich im Frequenzraum in einer zweiten Zeitspanne nach einer Applikation eines Kontrastmittels repräsentiert, repräsentiert die transformierte synthetische Repräsentation zumindest einen Teil des Untersuchungsbereichs im Ortsraum in der zweiten Zeitspanne nach der Applikation des Kontrastmittels. Die Erzeugung der transformierten synthetischen Repräsentation kann durch Transformation der synthetischen Repräsentation erfolgen und/oder das Modell des maschinellen Lernens kann konfiguriert sein und trainiert werden, eine transformierte synthetische Repräsentation auf Basis der mindestens einen Eingabe-Repräsentation zu erzeugen.

Mittels einer Fehlerfunktion werden die Abweichungen i) zwischen zumindest einem Teil der synthetischen Repräsentation und zumindest einem Teil der Ziel-Repräsentation und ii) zwischen zumindest einem Teil der transformierten synthetischen Repräsentation und zumindest einem Teil der transformierten Ziel-Repräsentation quantifiziert.

Die Fehlerfunktion kann zwei Terme aufweisen, einen ersten Term zur Quantifizierung der Abweichungen zwischen zumindest einem Teil der synthetischen Repräsentation und zumindest einem Teil der Ziel-Repräsentation und einen zweiten Term zur Quantifizierung der Abweichungen zwischen zumindest einem Teil der transformierten synthetischen Repräsentation und zumindest einem Teil der transformierten Ziel-Repräsentation. Die Terme können in der Fehlerfunktion beispielsweise addiert werden. Die Terme können in der Fehlerfunktion unterschiedlich gewichtet werden. Die nachfolgende Gleichung gibt ein Beispiel für eine (Gesamt-)Fehlerfunktion $L$ zur Quantifizierung der Abweichungen an:

$$L = \lambda_1 \cdot L_1 + \lambda_2 \cdot L_2 \quad \text{(Gl. 1)}$$

Dabei ist $L$ die (Gesamt-)Fehlerfunktion, $L_1$ ein Term, der die Abweichungen zwischen der synthetischen Repräsentation und der Ziel-Repräsentation repräsentiert, $L_2$ ein Term, der die Abweichungen zwischen der transformierten synthetischen Repräsentation und der transformierten Ziel-Repräsentation quantifiziert, und $\lambda_1$ und $\lambda_2$ Gewichtsfaktoren, die beispielsweise Werte zwischen 0 und 1 annehmen können und den beiden Termen ein unterschiedliches Gewicht in der Fehlerfunktion geben. Es ist möglich, dass die Gewichtsfaktoren während des Trainierens des Modells des maschinellen Lernens konstant gehalten werden oder variiert werden.

Beispiele für Fehlerfunktionen, die zur Ausführung der vorliegenden Erfindung verwendet werden können, sind L1-Fehlerfunktion (L1 *loss*), L2-Fehlerfunktion (L2 *loss*), Lp-Fehlerfunktion (Lp loss), Strukturähnlichkeitsindexmaß (*structural similarity index measure* (SSIM)), VGG-Fehlerfunktion (VGG loss), Wahrnehmungs-Fehlerfunktion (*perceptual loss*) oder eine Kombination der oben genannten Funktionen oder andere Fehlerfunktionen. Weitere Einzelheiten zu Fehlerfunktionen sind z.B. in der wissenschaftlichen Literatur zu finden (siehe z. B.: R. Mechrez et al.: The Contextual Loss for Image Transformation with Non-Aligned Data, 2018, arXiv:1803.02077v4; H. Zhao et al.: Loss Functions for Image Restoration with Neural Networks, 2018, arXiv:1511.08861v3; D. Fuoli et al.: Fourier Space Losses for Efficient Perceptual Image Super-Resolution, arXiv:2106.00783v1).

Fig. 1 zeigt beispielhaft und schematisch den Prozess des Trainierens des Modells des maschinellen Lernens. Das Trainieren erfolgt mit Hilfe von Trainingsdaten. In Fig. 1 sind Trainingsdaten TD für ein Untersuchungsobjekt gezeigt. Die Trainingsdaten TD umfassen als Eingabedaten eine erste Eingabe-Repräsentation R1 eines Untersuchungsbereichs des Untersuchungsobjekts und eine zweite Eingabe-Repräsentation R2 des Untersuchungsbereichs des Untersuchungsobjekts. Die erste Eingabe-Repräsentation R1 und die zweite Eingabe-Repräsentation R2 repräsentieren den Untersuchungsbereich im Ortsraum. Die erste Eingabe-Repräsentation R1 repräsentiert den Untersuchungsbereich in einer ersten Zeitspanne, z.B. zu einem ersten Zeitpunkt vor einer Applikation eines Kontrastmittels. Die zweite Eingabe-Repräsentation R2 repräsentiert den Untersuchungsbereich ebenfalls in der ersten Zeitspanne, zum Beispiel zu einem zweiten Zeitpunkt nach der Applikation eines Kontrastmittels.

Die Trainingsdaten TD umfassen ferner eine Ziel-Repräsentation TR als Zieldaten. Die Ziel-Repräsentation TR repräsentiert den Untersuchungsbereich ebenfalls im Ortsraum. Die Ziel-Repräsentation TR repräsentiert den Untersuchungsbereich in einer zweiten Zeitspanne, beispielsweise zu einem dritten Zeitpunkt nach der Applikation des Kontrastmittels. Die zweite Zeitspanne folgt zeitlich der ersten Zeitspanne, d.h. der erste Zeitpunkt ist früher als der zweite Zeitpunkt und der zweite Zeitpunkt ist früher als der dritte Zeitpunkt.

Das Modell des maschinellen Lernens MLM wird trainiert, auf Basis der ersten Eingabe-Repräsentation R1 und der zweiten Eingabe-Repräsentation R2 sowie auf Basis von Modellparametern MP die Ziel-Repräsentation TR vorherzusagen. Die erste Eingabe-Repräsentation R1 und die zweite Eingabe-Repräsentation R2 werden dem Modell des maschinellen Lernens als Eingabedaten zugeführt. Das Modell des maschinellen Lernens ist konfiguriert, eine synthetische Repräsentation SR zu erzeugen.

Von der synthetischen Repräsentation SR wird in dem in Fig. 1 gezeigten Beispiel mittels einer Transformation T (z.B. einer Fourier-Transformation) eine transformierte synthetische Repräsentation $SR^T$ erzeugt. Analog wird von der Ziel-Repräsentation TR mittels der Transformation T eine transformierte Ziel-Repräsentation $TR^T$ erzeugt. Die transformierte synthetische Repräsentation $SR^T$ und die transformierte Ziel-Repräsentation $TR^T$ sind Frequenzraumdarstellungen des Untersuchungsbereichs in der zweiten Zeitspanne nach der Applikation des Kontrastmittels.

Eine erste Fehlerfunktion $L_1$ wird verwendet, um die Abweichungen zwischen der synthetischen Repräsentation SR und der Ziel-Repräsentation TR zu quantifizieren. Eine zweite Fehlerfunktion $L_2$ wird verwendet, um die Abweichungen zwischen der transformierten synthetischen Repräsentation $SR^T$ und der transformierten Ziel-Repräsentation $TR^T$ zu quantifizieren. Die mittels der Fehlerfunktionen $L_1$ und $L_2$ berechneten Fehler werden in einer Gesamtfehlerfunktion $L$ zu einem Gesamtfehler zusammengefasst (z.B. durch Addition mit oder ohne Gewichtung).

Modellparameter MP werden im Hinblick auf eine Reduzierung des Gesamtfehlers modifiziert. Die Reduzierung des Gesamtfehlers kann mit Hilfe eines Optimierungsverfahrens erfolgen, beispielsweise mit Hilfe eines Gradientenverfahrens.

Der Prozess wird für eine Vielzahl an Untersuchungsobjekten wiederholt, bis der Gesamtfehler ein vordefiniertes (gewünschtes) Minimum erreicht hat und/oder bis sich der Gesamtfehler durch Modifizieren von Modellparametern nicht weiter reduzieren lässt.

Das trainierte Modell des maschinellen Lernens kann dann zur Vorhersage verwendet werden. Dies ist beispielhaft und schematisch in Fig. 2 dargestellt: Mindestens eine Eingabe-Repräsentation (R1*, R2*) des Untersuchungsbereichs eines neuen Untersuchungsobjekts wird dem trainierten Modell $MLM^t$ des maschinellen Lernens zugeführt. Das trainierte Modell $MLM^t$ des maschinellen Lernens erzeugt eine synthetische Repräsentation SR* des Untersuchungsbereichs des neuen Untersuchungsobjekts. Dabei bedeutet der Begriff "neu", das Eingabedaten von dem neuen Untersuchungsobjekt üblicherweise nicht bereits beim Trainieren und/oder Validieren des Modells des maschinellen Lernens verwendet wurden. Die erzeugte synthetische Repräsentation SR* kann ausgegeben, gespeichert und/oder

an ein separates Computersystem übermittelt werden.

In einer Ausführungsform der vorliegenden Offenbarung erfolgt die Quantifizierung der Abweichungen zwischen der transformierten synthetischen Repräsentation und der transformierten Ziel-Repräsentation nur auf Basis von Anteilen der genannten Repräsentationen. Dies ist beispielhaft und schematisch in Fig. 3 dargestellt.

Der in Fig. 3 dargestellte Prozess entspricht dem Prozess, der in Fig. 1 dargestellt ist, mit folgenden Unterschieden:

- Die transformierte Ziel-Repräsentation $TR^T$ wird auf einen definierten Anteil $TR^{T,P}$ reduziert. Die Repräsentation $TR^{T,P}$ ist ein Teil der transformierten Ziel-Repräsentation TR. Die Repräsentation $TR^{T,P}$ kann durch eine Funktion P aus der transformierten Ziel-Repräsentation $TR^T$ erzeugt werden, indem beispielsweise alle Werte von Frequenzen außerhalb des vordefinierten Teils auf Null gesetzt werden.

- Analog wird die transformierte synthetische Repräsentation $SR^T$ auf einen definierten Anteil $SR^{T,P}$ reduziert.

- Die Anteile, auf die die transformierte Ziel-Repräsentation $TR^T$ und die transformierte synthetische Repräsentation $SR^T$ reduziert werden, korrespondieren üblicherweise miteinander, d.h. sie betreffen üblicherweise dieselben Frequenzen. In dem in Fig. 3 dargestellten Prozess ist sowohl die die transformierte Ziel-Repräsentation $TR^T$ als auch die transformierte synthetische Repräsentation $SR^T$ auf jeweils einen Anteil reduziert worden, der die niedrigen Frequenzen in einem Bereich (z.B. Rechteck oder Quadrat) um das Zentrum herum beinhaltet (siehe die gestrichelten weißen Rahmen). In diesem Bereich sind überwiegend Kontrastinformationen kodiert).

In dem in Fig. 3 dargestellten Beispiel wird also nicht der gesamte Frequenzraum bei der Berechnung mittels der Fehlerfunktion $L_2$ betrachtet. Die Repräsentationen im Frequenzraum werden auf einen Bereich mit niedrigen Frequenzen reduziert; die höheren Frequenzen werden verworfen. In dem Bereich der niedrigen Frequenzen sind überwiegend Kontrastinformationen kodiert, während im Bereich der höheren Frequenzen überwiegend Informationen zu Feinstrukturen kodiert sind. Das bedeutet, dass das Modell des maschinellen Lernens in dem in Fig. 3 dargestellten Beispiel zusätzlich zu der Erzeugung der synthetischen Repräsentation SR trainiert wird, insbesondere die niedrigen Frequenzen korrekt wiederzugeben. Damit wird beim Trainieren ein Fokus auf Kontrastinformationen gelegt.

Es sei angemerkt, dass Fig. 3 nicht so verstanden werden soll, dass die transformierte Ziel-Repräsentation $TR^T$ und die transformierte synthetische Repräsentation $SR^T$ beschnitten werden müssen, um sie auf den definierten Anteil $TR^{T,P}$ bzw. den definierten Anteil $SR^{T,P}$ zu reduzieren. Es ist ebenso möglich, zur Quantifizierung der Abweichungen zwischen dem Anteil $SR^{T,P}$ und dem Anteil $TR^{T,P}$ mittels der Fehlerfunktion $L_2$ nur die Bereiche $SR^{TP}$ und $TR^{T,P}$ innerhalb der Repräsentationen $SR^T$ und $TR^T$ zu berücksichtigen. Der Begriff "reduzieren" ist also so zu verstehen, dass für die Ermittlung der Abweichungen zwischen der transformierten synthetischen Repräsentation $SR^T$ und der transformierten Ziel-Repräsentation $TR^T$ nur die Bereiche $SR^{T,P}$ und $TR^{T,P}$ innerhalb der Repräsentationen $SR^T$ und $TR^T$ berücksichtigt werden. Dies gilt analog auch für Fig. 4, Fig. 5, Fig. 6, Fig. 7 und Fig. 8.

Fig. 4 zeigt ein weiteres Beispiel, bei dem nicht der gesamte Frequenzraum betrachtet, sondern ein Fokus auf einen definierten Frequenzbereich gelegt wird. Der in Fig. 4 dargestellte Prozess entspricht dem Prozess, der in Fig. 3 dargestellt ist, mit dem Unterschied, dass die Funktion *P* dafür sorgt, dass die transformierte Ziel-Repräsentation $TR^T$ und die transformierte synthetische Repräsentation $SR^T$ jeweils auf einen Anteil mit höheren Frequenzen reduziert werden, während niedrige Frequenzen verworfen werden (die niedrigen Frequenzwerte werden im vorliegenden Beispiel auf den Wert Null gesetzt, der durch die Farbe schwarz repräsentiert wird). Das bedeutet, dass das Modell des maschinellen Lernens in dem in Fig. 4 dargestellten Beispiel zusätzlich zu der Erzeugung der synthetischen Repräsentation SR trainiert wird, insbesondere die höheren Frequenzen korrekt wiederzugeben und damit ein Fokus auf die korrekte Wiedergabe von Feinstrukturen gelegt wird. Wie bereits in Bezug zu Fig. 3 erläutert, müssen die niedrigen Frequenzwerte nicht zwingend auf Null gesetzt werden; ebenso ist es möglich, dass bei der Quantifizierung der Abweichungen zwischen der transformierten synthetischen Repräsentation $SR^T$ und der transformierten Ziel-Repräsentation $TR^T$ mittels der Fehlerfunktion $L_2$ nur die nicht schwarz dargestellten Bereiche berücksichtigt werden. Dies gilt analog auch für Fig. 8.

Es sei ferner angemerkt, dass der Teil, auf den der Frequenzraum bei der Fehlerberechnung reduziert wird, auch andere als die in Fig. 3, Fig. 4 und Fig. 8 gezeigten Formen und Größen annehmen kann. Ferner können mehrere Teile ausgewählt und die Frequenzraum-Darstellungen auf mehrere Teile (Bereiche) beschränkt (reduziert) werden. So ist es beispielsweise möglich, den Frequenzraum in verschiedene Bereiche zu unterteilen und für mehrere oder alle Bereiche Frequenzraum-Darstellungen des Untersuchungsbereichs zu erzeugen, in denen jeweils nur die Frequenzen des jeweiligen Bereichs auftreten. Auf diese Weise können unterschiedliche Frequenzbereiche unterschiedlich stark beim Trainieren gewichtet/berücksichtigt werden.

In den in Fig. 3 und Fig. 4 gezeigten Beispielen handelt es sich bei den Repräsentationen R1, R2, TR und SR um Repräsentationen im Ortsraum. Es ist ebenso möglich, dass eine oder mehrere oder alle dieser Repräsentationen Frequenzraum-Darstellungen sind.

Sind die transformierte synthetische Repräsentation $SR^T$ und die transformierte Ziel-Repräsentation $TR^T$ Repräsentationen im Ortsraum, kann ebenfalls ein Teil (oder mehrere Teile) in den Repräsentationen ausgewählt und die Repräsentationen können auf diesen Teil (diese Teile) reduziert werden. In einem solchen Fall wird also bei der Fehlerberechnung Fokus auf Merkmale im Ortsraum gelegt, die sich in dem ausgewählten Teil (den ausgewählten Teilen) wiederfinden.

Fig. 5, Fig. 6, Fig. 7 und Fig. 8 zeigen weitere Ausführungsformen des Trainingsverfahrens.

In der in Fig. 5 gezeigten Ausführungsform umfassen die Trainingsdaten für jedes Untersuchungsobjekt mindestens eine Eingabe-Repräsentation R1 eines Untersuchungsbereichs und eine Ziel-Repräsentation TR des Untersuchungsbereichs. Die mindestens eine Eingabe-Repräsentation R1 repräsentiert den Untersuchungsbereich in einer ersten Zeitspanne vor oder nach der Applikation eines Kontrastmittels, und die Ziel-Repräsentation TR repräsentiert den Untersuchungsbereich in einer zweiten Zeitspanne nach der Applikation des Kontrastmittels.

Die mindestens eine Eingabe-Repräsentation R1 und die Ziel-Repräsentation TR können jeweils eine Repräsentation im Ortsraum oder eine Repräsentation im Frequenzraum sein. In dem in Fig. 5 gezeigten Beispiel sind die Eingabe-Repräsentation R1 und die Ziel-Repräsentation TR Repräsentationen im Ortsraum. Das Modell des maschinellen Lernens MLM wird trainiert, die Ziel-Repräsentation TR auf Basis der mindestens einen Eingabe-Repräsentation R1 vorherzusagen. Mittels einer Transformation T wird auf Basis der Eingabe-Repräsentation R1 eine transformierte Eingabe-Repräsentation R2 erzeugt. Analog wird mittels der Transformation T eine transformierte Ziel-Repräsentation $TR^T$ auf Basis der Ziel-Repräsentation TR erzeugt. In dem in Fig. 5 gezeigten Beispiel sind die transformierte Eingabe-Repräsentation R2 und die transformierte Ziel-Repräsentation $TR^T$ Repräsentationen des Untersuchungsbereichs im Frequenzraum. Die transformierte Eingabe-Repräsentation R2 repräsentiert den Untersuchungsbereich (wie die Eingabe-Repräsentation R1) in der ersten Zeitspanne; die transformierte Ziel-Repräsentation $TR^T$ repräsentiert den Untersuchungsbereich (wie die Ziel-Repräsentation TR) in der zweiten Zeitspanne.

In Fig. 5 ist dargestellt, dass die Eingabe-Repräsentation R1 durch eine inverse Transformation $T^{-1}$ auf Basis der transformierten Eingabe-Repräsentation R2 gewonnen werden kann. Analog kann eine Ziel-Repräsentation TR durch die inverse Transformation $T^{-1}$ auf Basis der transformierten Ziel-Repräsentation $TR^T$ gewonnen werden. Die inverse Transformation $T^{-1}$ ist die zu der Transformation $T$ inverse Transformation, was durch den hochgestellten Index -1 gekennzeichnet ist. Der Hinweis auf die inverse Transformation soll verdeutlichen, dass die Trainingsdaten zumindest eine Eingabe-Repräsentation (R1 oder R2) und eine Ziel-Repräsentation (TR oder $TR^T$) enthalten müssen; die jeweils andere (R2 oder R1, bzw. $TR^T$ oder TR) kann durch Transformation oder inverse Transformation aus der vorliegenden Repräsentation gewonnen werden. Dies gilt generell und nicht nur für die in Fig. 5 dargestellte Ausführungsform.

In der in Fig. 5 gezeigten Ausführungsform werden sowohl die Eingabe-Repräsentation R1 als auch die transformierte Eingabe-Repräsentation R2 dem Modell des maschinellen Lernens MLM zugeführt. Das Modell des maschinellen Lernens MLM ist konfiguriert, sowohl eine synthetische Repräsentation SR als auch eine transformierte synthetische Repräsentation $SR^T$ zu erzeugen.

Mittels einer ersten Fehlerfunktion $L_1$ werden die Abweichungen zwischen zumindest einem Teil der synthetischen Repräsentation SR und zumindest einem Teil der Ziel-Repräsentation TR quantifiziert. Mittels einer zweiten Fehlerfunktion $L_2$ werden die Abweichungen zwischen zumindest einem Teil der transformierten synthetischen Repräsentation $SR^T$ und zumindest einem Teil der transformierten Ziel-Repräsentation $TR^T$ quantifiziert. Die in Fig. 5 in den transformierten Repräsentationen eingezeichneten gestrichelten weißen Rahmen sollen zum Ausdruck bringen, dass die Berechnung des Fehlers mit der Fehlerfunktion wie in Bezug zu Fig. 3 und Fig. 4 beschrieben nicht auf der gesamten Frequenzraumdarstellung beruhen muss, sondern dass die transformierten Repräsentationen auf einen Frequenzbereich (oder mehrere Frequenzbereiche) reduziert werden können. Analog können die Ortsraumdarstellungen SR und TR bei der Fehlerberechnung auf einen Teil (oder mehrere Teile) reduziert werden (ebenfalls dargestellt durch die gestrichelten weißen Rahmen). Dies gilt analog auch für die übrigen Ausführungsformen und nicht nur für die in Fig. 5 dargestellte Ausführungsform.

Die mittels der Fehlerfunktionen $L_1$ und $L_2$ berechneten Fehler werden in einer Gesamtfehlerfunktion $L$ zu einem Gesamtfehler zusammengefasst (z.B. durch Addition mit oder ohne Gewichtung).

Modellparameter MP werden im Hinblick auf eine Reduzierung des Gesamtfehlers modifiziert. Dies kann mittels eines Optimierungsverfahrens, beispielsweise eines Gradientenverfahrens erfolgen.

Der Prozess wird für eine Vielzahl an Untersuchungsobjekten wiederholt, bis der Gesamtfehler ein vordefiniertes (gewünschtes) Minimum erreicht hat und/oder bis sich der Gesamtfehler durch Modifizieren von Modellparametern nicht weiter reduzieren lässt.

Die synthetische Repräsentation SR und die transformierte synthetische Repräsentation $SR^T$ müssen sich genauso ineinander umwandeln lassen wie die Ziel-Repräsentation TR und die transformierte Zielrepräsentation $TR^T$. Es ist möglich, eine weitere Fehlerfunktion einzuführen, die die Qualität einer solchen Umwandlung bewertet. Es ist also möglich, durch die Transformation T aus der synthetischen Repräsentation SR eine transformierte synthetische Repräsentation $SR^T$ zu erzeugen und die Abweichungen zwischen dieser durch Transformation erzeugten und der von dem Modell des maschinellen Lernens erzeugten transformierten synthetischen Repräsentation mittels einer dritten Fehler-

funktion $L_3$ zu quantifizieren. Alternativ oder zusätzlich ist es möglich, durch die inverse Transformation $T^{-1}$ aus der transformierten synthetischen Repräsentation $SR^T$ eine synthetische Repräsentation SR zu erzeugen und die Abweichungen zwischen dieser durch inverse Transformation erzeugten und der von dem Modell des maschinellen Lernens erzeugten synthetischen Repräsentation mittels einer dritten Fehlerfunktion $L_3$ zu quantifizieren. Dies ist schematisch in Fig. 6 am Beispiel der Erzeugung einer transformierten synthetischen Repräsentation $SR^{T\#}$ durch Transformation $T$ aus der synthetischen Repräsentation SR gezeigt. In dem in Fig. 6 gezeigten Beispiel quantifiziert die Fehlerfunktion $L_3$ die Abweichungen zwischen der durch Transformation erzeugten transformierten synthetischen Repräsentation $SR^{T\#}$ und der vom Modell des maschinellen Lernens MLM erzeugten transformierten synthetischen Repräsentation $SR^T$. Die Fehlerfunktionen $L_1$, $L_2$ und $L_3$ werden in einer Gesamtfehlerfunktion $L$ kombiniert (z.B. durch Addition mit oder ohne Gewichtung). In der Gesamtfehlerfunktion $L$ können die einzelnen Terme unterschiedliche Gewichte tragen, wobei die Gewichte im Verlauf des Trainings konstant gehalten oder variiert werden können.

Eine Abwandlung von den in Fig. 5 und Fig. 6 gezeigten Ausführungsformen besteht darin, dass dem Modell des maschinellen Lernens MLM nicht beide Eingangs-Repräsentationen R1 und R2 zugeführt werden, sondern nur eine der beiden. Das Modell des maschinellen Lernens kann konfiguriert sein und trainiert werden, die jeweils andere zu erzeugen.

Fig. 7 zeigt schematisch eine weitere Ausführungsform des Trainingsverfahrens. In dieser Ausführungsform werden zwei Modelle des maschinellen Lernens verwendet, ein erstes Modell MLM1 und ein zweites Modell MLM2. Das erste Modell des maschinellen Lernens MLM1 wird trainiert, aus einer Eingabe-Repräsentation R1 und/oder R2 eine Ziel-Repräsentation TR und/oder eine transformierte Ziel-Repräsentation $TR^T$ vorherzusagen. Das zweite Modell des maschinellen Lernens MLM2 wird trainiert, aus einer vorhergesagten Ziel-Repräsentation SR und/oder einer vorhergesagten transformierten Ziel-Repräsentation $SR^T$ wieder die ursprüngliche Eingabe-Repräsentation R1 und/oder R2 vorherzusagen (zu rekonstruieren). Die Modelle des maschinellen Lernens führen also einen Zyklus durch, mit dessen Hilfe die Vorhersagequalität des ersten Modells MLM1 (das bei der späteren Vorhersage verwendet wird) verbessert werden kann.

Dem ersten Modell des maschinellen Lernens MLM1 wird mindestens eine Eingabe-Repräsentation R1 und/oder mindestens eine transformierte Eingabe-Repräsentation R2 als Eingabedaten zugeführt. Das erste Modell MLM1 ist konfiguriert, eine synthetische Repräsentation SR und/oder eine transformierte synthetische Repräsentation $SR^T$ auf Basis der Eingabedaten und Modellparametern MP 1 zu erzeugen. Eine transformierte synthetische Repräsentation $SR^T$ kann auch durch Transformation T der synthetischen Repräsentation SR erzeugt werden. Eine synthetische Repräsentation SR kann auch durch inverse Transformation $T^{-1}$ der transformierten synthetischen Repräsentation $SR^T$ erzeugt werden.

Abweichungen zwischen der synthetischen Repräsentation SR und der Ziel-Repräsentation TR können mittels einer Fehlerfunktion $L_1{}^1$ quantifiziert werden. Abweichungen zwischen der transformierten synthetischen Repräsentation $SR^T$ und der transformierten Ziel-Repräsentation $TR^T$ können mittels einer Fehlerfunktion $L_2{}^1$ quantifiziert werden.

Abweichungen zwischen einer durch inverse Transformation $T^{-1}$ gewonnenen synthetischen Repräsentation SR und der durch das Modell MLM 1 erzeugten synthetischen Repräsentation SR können mittels einer Fehlerfunktion $L_3{}^1$ quantifiziert werden (in Fig. 7 nicht gezeigt). Alternativ oder zusätzlich können Abweichungen zwischen einer durch Transformation $T$ gewonnenen transformierten synthetischen Repräsentation $SR^T$ und der durch das Modell MLM1 erzeugten transformierten synthetischen Repräsentation $SR^T$ mittels einer Fehlerfunktion $L_4{}^1$ quantifiziert werden (in Fig. 6 nicht gezeigt).

Die Fehlerfunktionen $L_1{}^1$, $L_2{}^1$ und, falls vorhanden, $L_3{}^1$ und/oder $L_4{}^1$ können zu einer Gesamtfehlerfunktion kombiniert werden (in Fig. 7 nicht gezeigt).

Modellparameter MP1 können im Hinblick auf eine Reduzierung des Gesamtfehlers modifiziert werden.

Die synthetische Repräsentation SR und/oder die transformierte synthetische Repräsentation $SR^T$ wird/werden dem zweiten Modell des maschinellen Lernens MLM2 zugeführt. Das zweite Modell MLM2 ist konfiguriert, die erste Eingabe-Repräsentation R1 und/der die zweite Eingabe-Repräsentation R2 zu rekonstruieren (vorherzusagen). Das zweite Modell MLM2 ist konfiguriert, eine vorhergesagte erste Eingabe-Repräsentation R1# und/oder eine vorhergesagte zweite Eingabe-Repräsentation R2# auf Basis der synthetischen Repräsentation SR und/oder der transformierten synthetischen Repräsentation $SR^T$ und auf Basis von Modellparametern MP2 zu erzeugen. Eine zweite Eingabe-Repräsentation R2# kann auch durch Transformation $T$ der ersten Eingabe-Repräsentation R1# erzeugt werden und/oder eine erste Eingabe-Repräsentation R1# kann auch durch inverse Transformation $T^{-1}$ der zweiten Eingabe-Repräsentation R2# erzeugt werden.

Abweichungen zwischen der vorhergesagten ersten Eingabe-Repräsentation R1# und der ersten Eingabe-Repräsentation R1 können mittels einer Fehlerfunktion $L_1{}^2$ quantifiziert werden. Abweichungen zwischen der vorhergesagten zweiten Eingabe-Repräsentation R2# und der zweiten Eingabe-Repräsentation R2 können mittels einer Fehlerfunktion $L_2{}^2$ quantifiziert werden.

Abweichungen zwischen einer durch inverse Transformation $T^{-1}$ gewonnenen Eingabe-Repräsentation und der durch das Modell MLM2 erzeugten Eingabe-Repräsentation können mittels einer Fehlerfunktion $L_3{}^2$ quantifiziert werden (in Fig. 7 nicht gezeigt). Alternativ oder zusätzlich können Abweichungen zwischen einer durch Transformation gewonnenen Eingabe-Repräsentation und der durch das Modell MLM2 erzeugten Eingabe-Repräsentation mittels einer Fehlerfunktion $L_4^2$ quantifiziert werden (in Fig. 7 nicht gezeigt).

Die Fehlerfunktionen $L_1^2$, $L_2^2$ und, falls vorhanden, $L_3^2$ und/oder $L_4^2$ können zu einer Gesamtfehlerfunktion kombiniert werden (in Fig. 7 nicht gezeigt).

Modellparameter MP2 können im Hinblick auf eine Reduzierung des Gesamtfehlers modifiziert werden.

Fig. 8 zeigt schematisch ein weiteres Beispiel eines Verfahrens zum Trainieren eines Modells des maschinellen Lernens. Fig. 9 zeigt die Verwendung des trainierten Modells des maschinellen Lernens zur Vorhersage. Das in Fig. 8 und Fig. 9 gezeigte Beispiel betrifft die Beschleunigung einer magnetresonanztomographischen Untersuchung der Leber eines Untersuchungsobjekts unter Verwendung eines hepatobiliären Kontrastmittels.

Ein Beispiel eines hepatobiliären Kontrastmittels ist das Dinatriumsalz der Gadoxetsäure (Gd-EOB-DTPA Dinatrium. GD), das in dem US-Patent No. 6,039,931A beschrieben ist und unter den Markennamen Primovist® und Eovist® kommerziell erhältlich ist.

Ein hepatobiliäres Kontrastmittel kann zur Detektion von Tumoren in der Leber eingesetzt werden. Die Blutversorgung des gesunden Lebergewebes erfolgt in erster Linie über die Pfortader (Vena portae), während die Leberarterie (Arteria hepatica) die meisten Primärtumoren versorgt. Nach intravenöser Bolusinjektion eines Kontrastmittels lässt sich dementsprechend eine Zeitverzögerung zwischen der Signalanhebung des gesunden Leberparenchyms und des Tumors beobachten.

Neben malignen Tumoren findet man in der Leber häufig gutartige Läsionen wie Zysten, Hämangiome und fokal noduläre Hyperplasien (FNH). Für eine sachgerechte Therapieplanung müssen diese von den malignen Tumoren differenziert werden. Primovist® kann zur Erkennung gutartiger und bösartiger fokaler Leberläsionen eingesetzt werden. Es liefert mittels T1-gewichteter MRT Informationen über den Charakter dieser Läsionen. Bei der Differenzierung nutzt man die unterschiedliche Blutversorgung von Leber und Tumor und den zeitlichen Verlauf der Kontrastverstärkung.

Bei der durch Primovist® erzielten Kontrastverstärkung während der Anflutungsphase beobachtet man typische Perfusionsmuster, die Informationen für die Charakterisierung der Läsionen liefern. Die Darstellung der Vaskularisierung hilft, die Läsionstypen zu charakterisieren und den räumlichen Zusammenhang zwischen Tumor und Blutgefäßen zu bestimmen.

Bei T1-gewichteten MRT-Aufnahmen führt Primovist® 10-20 Minuten nach der Injektion (in der hepatobiliären Phase) zu einer deutlichen Signalverstärkung im gesunden Leberparenchym, während Läsionen, die keine oder nur wenige Hepatozyten enthalten, z.B. Metastasen oder mittelgradig bis schlecht differenzierte hepatozelluläre Karzinome (HCCs), als dunklere Bereiche auftreten.

Die zeitliche Verfolgung der Verteilung des Kontrastmittels bietet also eine gute Möglichkeit der Detektion und Differentialdiagnose fokaler Leberläsionen; allerdings zieht sich die Untersuchung über eine vergleichsweise lange Zeitspanne hin. Über diese Zeitspanne sollten Bewegungen des Patienten weitgehend vermieden werden, um Bewegungsartefakte in den MRT-Aufnahmen zu minimieren. Die lang andauernde Bewegungseinschränkung kann für einen Patienten unangenehm sein.

Dementsprechend wird bereits in WO2021/052896A1 vorgeschlagen, MRT-Aufnahmen der Leber eines Patienten während der hepatobiliären Phase nicht messtechnisch zu erzeugen, sondern auf Basis von MRT-Aufnahmen aus einer oder mehreren vorangegangenen Phasen vorherzusagen.

Fig. 8 und Fig. 9 zeigen einen gegenüber dem in WO2021/052896A1 beschriebenen Verfahren verbesserten Ansatz:

Das Training des Modells des maschinellen Lernens MLM erfolgt auf Basis von Trainingsdaten. Die Trainingsdaten umfassen, für jedes Untersuchungsobjekt einer Vielzahl an Untersuchungsobjekten, eine oder mehrere Eingabe-Repräsentationen R1,2 der Leber oder eines Teils der Leber während einer ersten Zeitspanne vor und/oder nach der Applikation eines hepatobiliären Kontrastmittels und mindestens eine Ziel-Repräsentation TR der Leber oder des Teils der Leber während einer zweiten Zeitspanne nach der Applikation des hepatobiliären Kontrastmittels.

Die Eingabe-Repräsentation(en) R1,2 und die Ziel-Repräsentation TR werden üblicherweise mit einem MRT-Scanner erzeugt. Das hepatobiliäre Kontrastmittel kann beispielsweise gewichtsadaptiert als Bolus in eine Armvene des Untersuchungsobjekts appliziert werden.

Die Eingabe-Repräsentation(en) R1,2 kann/können die Leber oder den Teil der Leber während der nativen Phase, der arteriellen Phase, der portalvenösen Phase und/oder der Übergangsphase (wie in WO2021/052896A1 und den dort aufgeführten Referenzen beschrieben) repräsentieren.

Die Ziel-Repräsentation zeigt die Leber oder den Teil der Leber während der hepatobiliären Phase, also beispielsweise 10 bis 20 Minuten nach der Applikation des hepatobiliären Kontrastmittels.

Bei der/den in Fig. 8 gezeigten Eingabe-Repräsentation(en) R1,2 und der Ziel-Repräsentation TR handelt es sich um Repräsentationen der Leber oder des Teils der Leber im Ortsraum. Bei Verwendung mehrerer Eingabe-Repräsentationen R1,2 können die Eingabe-Repräsentationen den Untersuchungsbereich in unterschiedlichen Zuständen, nämlich zu unterschiedlichen Zeitpunkten in der nativen Phase, der arteriellen Phase, der portalvenösen Phase und/oder der Übergangsphase repräsentieren. Die Ziel-Repräsentation TR repräsentiert den Untersuchungsbereich in

einem weiteren Zustand, nämlich zu einem Zeitpunkt in der hepatobiliären Phase.

Die Eingabe-Repräsentationen R1,2 werden (ggf. nach einer Vorverarbeitung, die beispielsweise eine Bewegungskorrektur, eine Co-Registrierung und/oder eine Farbraumumwandlung umfassen kann), einem Modell des maschinellen Lernens MLM zu geführt.

Das Modell des maschinellen Lernens ist konfiguriert, eine synthetische Repräsentation SR auf Basis der Eingabe-Repräsentation(en) R1,2 und auf Basis von Modellparametern MP zu erzeugen.

Von der synthetischen Repräsentation SR wird mittels einer Transformation T (z.B. einer Fourier-Transformation) eine transformierte synthetische Repräsentation $SR^T$ erzeugt.

Die transformierte synthetische Repräsentation $SR^T$ ist z.B. eine Repräsentation der Leber oder des Teils der Leber während der hepatobiliären Phase im Frequenzraum.

Die transformierte synthetische Repräsentation $SR^T$ wird mittels einer Funktion P auf einen vordefinierten Anteil reduziert, dabei wird eine anteilige transformierte synthetische Repräsentation $SR^{T,P}$ erzeugt. Die Funktion P reduziert die transformierte synthetische Repräsentation $SR^T$ auf einen vordefinierten Frequenzbereich. Im vorliegenden Beispiel werden Frequenzen außerhalb eines Kreises mit einem definierten Radius um den Ursprung der transformierten synthetischen Repräsentation $SR^T$ auf Null gesetzt, so dass nur noch die Frequenzen innerhalb des Kreises verbleiben. Mit anderen Worten, die höheren Frequenzen, in denen Feinstrukturinformationen kodiert sind, werden gelöscht, und es verbleiben die niedrigen Frequenzen, in denen Kontrastinformationen kodiert sind.

Analog wird mit der Ziel-Repräsentation TR vorgegangen:

Von der Ziel-Repräsentation TR wird mittels einer Transformation T (z.B. einer Fourier-Transformation) eine transformierte Ziel-Repräsentation $TR^T$ erzeugt. Die transformierte Ziel-Repräsentation $TR^T$ ist eine Repräsentation der Leber oder des Teils der Leber während der hepatobiliären Phase im Frequenzraum. Die transformierte Ziel-Repräsentation $TR^T$ wird mittels der Funktion P auf einen vordefinierten Anteil reduziert, dabei wird eine anteilige transformierte Ziel-Repräsentation $TR^{T,P}$ erzeugt. Die Funktion P2 reduziert die transformierte Ziel-Repräsentation $TR^T$ auf den gleichen Frequenzbereich wie die transformierte synthetische Repräsentation $SR^T$. Im vorliegenden Beispiel werden Frequenzen außerhalb eines Kreises mit einem definierten Radius um den Ursprung der transformierten Ziel-Repräsentation $TR^T$ auf Null gesetzt, so dass nur noch die Frequenzen innerhalb des Kreises verbleiben. Mit anderen Worten, die höheren Frequenzen, in denen Feinstrukturinformationen kodiert sind, werden gelöscht, und es verbleiben die niedrigen Frequenzen, in denen Kontrastinformationen kodiert sind.

Um die Vorhersagequalität des Modells des maschinellen Lernens zu bewerten, wird ein Fehler mittels einer Fehlerfunktion $L$ berechnet. Im vorliegenden Beispiel setzt sich die Fehlerfunktion $L$ aus zwei Termen zusammen, einer ersten Fehlerfunktion $L_1$ und einer zweiten Fehlerfunktion $L_2$.

Die erste Fehlerfunktion $L_1$ quantifiziert die Abweichungen zwischen der synthetischen Repräsentation SR und der Ziel-Repräsentation TR.

Die zweite Fehlerfunktion $L_2$ quantifiziert die Abweichungen zwischen der anteiligen transformierten synthetischen Repräsentation $SR^T$ und der anteiligen transformierten Ziel-Repräsentation $TR^T$.

Die Terme für $L_1$ und $L_2$ können in der Gesamtfehlerfunktion $L$ beispielsweise mit Gewichtsfaktoren versehen und addiert werden, wie in der obigen Gleichung Gl. 1 dargestellt.

In einem Optimierungsverfahren, z.B. einem Gradientenverfahren, können die Modellparameter MP im Hinblick auf eine Reduzierung des mittels der Gesamtfehlerfunktion $L$ berechneten Fehlers modifiziert werden.

Der beschriebene Prozess wird für die weiteren Untersuchungsobjekte der Vielzahl an Untersuchungsobjekten wiederholt. Das Training kann beendet werden, wenn die mittels der Gesamtfehlerfunktion $L$ errechneten Fehler ein definiertes Minimum erreichen, d.h. die Vorhersagequalität ein gewünschtes Niveau erreicht.

Fig. 9 zeigt die Verwendung des trainierten Modell des maschinellen Lernens zur Vorhersage. Das Modell kann wie in Bezug zu Fig. 8 beschrieben trainiert worden sein. Dem trainierten Modell des maschinellen Lernens $MLM^t$ werden eine oder mehrere Eingabe-Repräsentationen R1,2* der Leber oder eines Teils der Leber eines neuen Untersuchungsobjekts zugeführt. Dabei bedeutet der Begriff "neu", dass die Eingabe-Repräsentationen R1,2* nicht bereits beim Trainieren des Modells verwendet worden sind.

Die Eingabe-Repräsentation(en) R1,2* repräsentiert/repräsentieren die Leber oder den Teil der Leber des neuen Untersuchungsobjekts vor/und oder nach der Applikation eines hepatobiliären Kontrastmittels, das beispielsweise in eine Armvene des neuen Untersuchungsobjekts in Form eines Bolus appliziert worden sein kann.

Die Eingabe-Repräsentation(en) R1,2* repräsentiert/repräsentieren die Leber oder den Teil der Leber des neuen Untersuchungsobjekts in der nativen Phase, der arteriellen Phase, der portalvenösen Phase und/oder der Übergangsphase.

Die Eingabe-Repräsentation(en) R1,2* repräsentiert/repräsentieren die Leber oder den Teil der Leber des neuen Untersuchungsobjekts im Ortsraum.

Das trainierte Modell des maschinellen Lernens ist konfiguriert und trainiert, auf Basis der Eingabe-Repräsentation(en) R1,2* eine synthetische Repräsentation SR* vorherzusagen.

Die synthetische Repräsentation SR* repräsentiert die Leber oder den Teil der Leber während der hepatobiliären

Phase, also beispielsweise 10 bis 20 Minuten nach der Applikation des hepatobiliären Kontrastmittels.

Bei den Modellen des maschinellen Lernens gemäß der vorliegenden Offenbarung kann es sich beispielsweise um ein künstliches neuronales Netzwerk handeln, oder es kann ein oder mehrere solcher künstlichen neuronalen Netze umfassen.

Ein künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen der Eingabe-Repräsentationen. Üblicherweise gibt es ein Eingangsneuron für jedes Pixel oder Voxel einer Eingabe-Repräsentation, falls es sich bei der Repräsentation um eine Ortsraumdarstellung in Form einer Rastergrafik handelt, oder ein Eingangsneuron für jede Frequenz, die in der Eingabe-Repräsentation vorhanden ist, falls es sich bei der Repräsentation um eine Frequenzraumdarstellung handelt. Es können zusätzliche Eingangsneuronen für zusätzliche Eingangswerte (z.B. Informationen zum Untersuchungsbereich, zum Untersuchungsobjekt, zu Bedingungen, die bei der Erzeugung der Eingabe-Repräsentation herrschten, Informationen zu dem Zustand, den die Eingabe-Repräsentation repräsentiert, und/oder Informationen zu dem Zeitpunkt oder der Zeitspanne zu/in der die Eingabe-Repräsentation erzeugt worden ist) vorhanden sein.

Die Ausgangsneuronen können dazu dienen, eine synthetische Repräsentation, die den Untersuchungsbereich in einem anderen Zustand repräsentiert, auszugeben.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN) oder es umfasst ein solches.

Ein CNN besteht üblicherweise im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Das Trainieren des neuronalen Netzes kann beispielsweise mittels eines Backpropagation-Verfahrens durchgeführt werden. Dabei wird für das Netz eine möglichst zuverlässige Abbildung der Eingabe-Repräsentation(en) auf die synthetische Repräsentation angestrebt. Die Qualität der Vorhersage wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich der Dynamik der Beziehung zwischen der/den Eingabe-Repräsentation(en) und der synthetischen Repräsentation, die verwendet werden können, um auf Basis einer oder mehrerer Repräsentationen des Untersuchungsbereichs eines neuen Untersuchungsobjekts eine synthetische Repräsentation des Untersuchungsbereichs des neuen Untersuchungsobjekts vorherzusagen. Dabei bedeutet der Begriff "neu", dass Repräsentationen des Untersuchungsbereichs des neuen Untersuchungsobjekt nicht bereits beim Trainieren des Modells des maschinellen Lernens verwendet wurden.

Eine Kreuzvalidierungsmethode kann verwendet werden, um die Daten in Trainings- und Validierungsdatensätze aufzuteilen. Der Trainingsdatensatz wird beim Backpropagation-Training der Netzwerkgewichte verwendet. Der Validierungsdatensatz wird verwendet, um zu überprüfen, mit welcher Vorhersagegenauigkeit sich das trainierte Netzwerk auf unbekannte (neue) Daten anwenden lässt.

Das künstliche neuronale Netzwerk kann eine Autoencoder-Architektur aufweisen; z.B. kann das künstliche neuronale Netzwerk eine Architektur wie das *U-Net* aufweisen (siehe z.B. O. Ronneberger et al.: U-net: Convolutional networks for biomedical image segmentation, International Conference on Medical image computing and computer-assisted intervention, Seiten 234-241, Springer, 2015, https://doi.org/10.1007/978-3-319-24574-4_28).

Das künstliche neuronale Netzwerk kann ein *Generative Adversarial Network* (GAN) sein (siehe z.B. M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887).

Das künstliche neuronale Netz kann ein *Regularized Generative Adversarial Network* sein (siehe z.B. Q. Li et al.: RegGAN: An End-to-End Network for Building Footprint Generation with Boundary Regularization, Remote Sens. 2022, 14, 1835).

Das künstliche neuronale Netzwerk kann ein *Conditional Adversarial Network* sein (siehe z.B. P. Isola et al.: Image-to-Image Translation with Conditional Adversarial Networks, arXiv:1611.07004 [cs.CV]).

Das künstliche neuronale Netz kann ein Transformer-Netzwerk sein (siehe z.B. D. Karimi et al.: Convolution-Free Medical Image Segmentation using Transformers, arXiv:2102.13645 [eess.IV]).

Fig. 10 zeigt beispielhaft und schematisch ein Computersystem gemäß der vorliegenden Offenbarung, das zum Trainieren des Modells des maschinellen Lernens und/oder zur Verwendung des trainierten Modells des maschinellen Lernens zur Vorhersage verwendet werden kann.

Ein "Computersystem" ist ein System zur elektronischen Datenverarbeitung, das mittels programmierbarer

Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Das in Fig. 10 gezeigte Computersystem (10) umfasst eine Empfangseinheit (11), eine Steuer- und Recheneinheit (12) und eine Ausgabeeinheit (13).

Die Steuer- und Recheneinheit (12) dient der Steuerung des Computersystems (10), der Koordinierung der Datenflüsse zwischen den Einheiten des Computersystems (10) und der Durchführung von Berechnungen.

Die Steuer- und Recheneinheit (12) ist konfiguriert:

- die Empfangseinheit (11) zu veranlassen, mindestens eine Eingabe-Repräsentation eines Untersuchungsbereichs eines Untersuchungsobjekts zu empfangen,
- die empfangene mindestens eine Eingabe-Repräsentation in ein trainiertes Modell des maschinellen Lernens einzugeben, wobei das trainierte Modell des maschinellen Lernens wie in dieser Beschreibung beschrieben trainiert worden ist,
- von dem Modell des maschinellen Lernens eine synthetische Repräsentation des Untersuchungsbereichs des Untersuchungsobjekts zu empfangen,
- die Ausgabeeinheit (13) zu veranlassen, die synthetische Repräsentation des Untersuchungsbereichs des Untersuchungsobjekts auszugeben und/oder zu speichern und/oder an ein separates Computersystem zu übermitteln.

Fig. 11 zeigt beispielhaft und schematisch eine weitere Ausführungsform des erfindungsgemäßen Computersystems.

Das Computersystem (1) umfasst eine Verarbeitungseinheit (21), die mit einem Speicher (22) verbunden ist. Die Verarbeitungseinheit (21) und der Speicher (22) bilden eine Steuer- und Recheneinheit, wie sie in Fig. 10 gezeigt ist.

Die Verarbeitungseinheit (21) (engl.: *processing unit*) kann einen oder mehrere Prozessoren allein oder in Kombination mit einem oder mehreren Speichern umfassen. Bei der Verarbeitungseinheit (21) kann es sich um gewöhnliche Computerhardware handeln, die in der Lage ist, Informationen wie z.B. digitale Bildaufnahmen, Computerprogramme und/oder andere digitale Informationen zu verarbeiten. Die Verarbeitungseinheit (21) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können. Die Verarbeitungseinheit (21) kann konfiguriert sein, Computerprogramme auszuführen, die in einem Arbeitsspeicher der Verarbeitungseinheit (21) oder im Speicher (22) desselben oder eines anderen Computersystems gespeichert sein können.

Der Speicher (22) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (22) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (22) kann die Verarbeitungseinheit (21) auch mit einer oder mehreren Schnittstellen (11, 12, 31, 32, 33) verbunden sein, um Informationen anzuzeigen, zu übertragen und/oder zu empfangen. Die Schnittstellen können eine oder mehrere Kommunikationsschnittstellen (32, 33) und/oder eine oder mehrere Benutzerschnittstellen (11, 12, 31) umfassen. Die eine oder mehrere Kommunikationsschnittstellen können so konfiguriert sein, dass sie Informationen senden und/oder empfangen, z.B. zu und/oder von einer MRT-Scanner, einem CT-Scanner, einer Ultraschallkamera, anderen Computersystemen, Netzwerken, Datenspeichern oder dergleichen. Die eine oder mehrere Kommunikationsschnittstellen können so konfiguriert sein, dass sie Informationen über physische (verdrahtete) und/oder drahtlose Kommunikationsverbindungen übertragen und/oder empfangen. Die eine oder die mehreren Kommunikationsschnittstellen können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen eine oder mehrere Nahbereichskommunikationsschnittstellen umfassen, die so konfiguriert sind, dass sie Geräte mit Nahbereichskommunikationstechnologien

wie NFC, RFID, Bluetooth, Bluetooth LE, ZigBee, Infrarot (z. B. IrDA) oder Ähnlichem verbinden.

Die Benutzerschnittstellen können eine Anzeige (31) umfassen. Eine Anzeige (31) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (11, 12) kann/können verdrahtet oder drahtlos sein und kann/können so konfiguriert sein, dass sie Informationen von einem Benutzer in das Computersystem (1) empfängt/empfangen, z.B. zur Verarbeitung, Speicherung und/oder Anzeige. Geeignete Beispiele für Benutzereingabeschnittstellen sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen können ferner eine oder mehrere Schnittstellen für die Kommunikation mit Peripheriegeräten wie Druckern und dergleichen umfassen.

Ein oder mehrere Computerprogramme (40) können im Speicher (22) gespeichert sein und von der Verarbeitungseinheit (21) ausgeführt werden, die dadurch programmiert wird, die in dieser Beschreibung beschriebenen Funktionen zu erfüllen. Das Abrufen, Laden und Ausführen von Anweisungen des Computerprogramms (40) kann sequenziell erfolgen, so dass jeweils ein Befehl abgerufen, geladen und ausgeführt wird. Das Abrufen, Laden und/oder Ausführen kann aber auch parallel erfolgen.

In dem Speicher (22) kann auch das erfindungsgemäße Modell des maschinellen Lernens gespeichert sein.

Das erfindungsgemäße Computersystem kann als Laptop, Notebook, Netbook und/der Tablet-PC ausgeführt sein, es kann auch ein Bestandteil eines MRT-Scanners, eines CT-Scanners oder eines Ultraschalldiagnosegeräts sein.

Fig. 12 zeigt schematisch in Form eines Ablaufdiagramms eine Ausführungsform des Verfahrens zum Trainieren eines Modells des maschinellen Lernens. Das Trainingsverfahren (100) umfasst die Schritte:

(110) Empfangen und/oder Bereitstellen von Trainingsdaten, wobei die Trainingsdaten für jedes Untersuchungsobjekt einer Vielzahl von Untersuchungsobjekten einen Satz an Eingabedaten und Zieldaten umfasst,

- o wobei jeder Satz mindestens eine Eingabe-Repräsentation eines Untersuchungsbereichs des Untersuchungsobjekts als Eingabedaten und eine Ziel-Repräsentation des Untersuchungsbereichs des Untersuchungsobjekts sowie eine transformierte Ziel-Repräsentation als Zieldaten umfasst,
- o wobei die mindestens eine Eingabe-Repräsentation den Untersuchungsbereich in einer ersten Zeitspanne vor und/oder nach der Applikation eines Kontrastmittels repräsentiert und die synthetische Repräsentation den Untersuchungsbereich in einer zweiten Zeitspanne nach der Applikation des Kontrastmittels repräsentiert,
- o wobei die transformierte Ziel-Repräsentation zumindest einen Teil des Untersuchungsbereichs des Untersuchungsobjekts
  - ▪ im Frequenzraum repräsentiert, falls die Ziel-Repräsentation den Untersuchungsbereichs des Untersuchungsobjekts im Ortsraum repräsentiert, oder
  - ▪ im Ortsraum repräsentiert, falls die Ziel-Repräsentation den Untersuchungsbereich des Untersuchungsobjekts im Frequenzraum repräsentiert,

(120) Trainieren eines Modells des maschinellen Lernens, wobei das Modell des maschinellen Lernens konfiguriert ist, auf Basis mindestens einer Eingabe-Repräsentation eines Untersuchungsbereichs eines Untersuchungsobjekts und Modellparametern eine synthetische Repräsentation des Untersuchungsbereichs des Untersuchungsobjekts zu erzeugen,
wobei das Trainieren für jedes Untersuchungsobjekt der Vielzahl an Untersuchungsobjekten umfasst:

- (121) Zuführen der mindestens einen Eingabe-Repräsentation dem Modell des maschinellen Lernens,
- (122) Empfangen einer synthetischen Repräsentation des Untersuchungsbereichs des Untersuchungsobjekts von dem Modell des maschinellen Lernens,
- (123) Erzeugen und/oder Empfangen einer transformierten synthetischen Repräsentation auf Basis der synthetischen Repräsentation und/oder zu der synthetischen Repräsentation, wobei die transformierte synthetische Repräsentation zumindest einen Teil des Untersuchungsbereichs des Untersuchungsobjekts
  - ▪ im Frequenzraum repräsentiert, falls die synthetische Repräsentation den Untersuchungsbereich des Untersuchungsobjekts im Ortsraum repräsentiert, oder

(fortgesetzt)

- im Ortsraum repräsentiert, falls die synthetische Repräsentation den Untersuchungsbereich des Untersuchungsobjekts im Frequenzraum repräsentiert,

(124) Quantifizieren der Abweichungen i) zwischen zumindest einem Teil der synthetischen Repräsentation und zumindest einem Teil der Ziel-Repräsentation und ii) zwischen zumindest einem Teil der transformierten synthetischen Repräsentation und zumindest einem Teil der transformierten Ziel-Repräsentation mittels einer Fehlerfunktion,

(125) Reduzieren der Abweichungen durch Modifizieren von Modellparametern,

(130) Ausgeben und/oder Speichern des trainierten Modells des maschinellen Lernens und/oder der Modellparameter und/oder Übermitteln des trainierten Modells des maschinellen Lernens und/oder der Modellparameter an ein separates Computersystem und/oder Verwenden des trainierten Modells des maschinellen Lernens zum Erzeugen einer synthetischen radiologischen Aufnahme des Untersuchungsbereichs eines neuen Untersuchungsobjekts.

Fig. 13 zeigt schematisch in Form eines Ablaufdiagramms eine Ausführungsform des Verfahrens zum Erzeugen einer synthetischen Repräsentation eines Untersuchungsbereichs eines Untersuchungsobjekts mit Hilfe des trainierten Modells des maschinellen Lernens. Das Vorhersageverfahren (200) umfasst die Schritte:

(210) Bereitstellen eines trainieren Modells des maschinellen Lernens,
  - o wobei das trainierte Modell des maschinellen Lernens anhand von Trainingsdaten trainiert worden ist, auf Basis mindestens einer Eingabe-Repräsentation eines Untersuchungsbereichs eines Untersuchungsobjekts eine synthetische Repräsentation des Untersuchungsbereichs des Untersuchungsobjekts zu erzeugen,
  - o wobei die Trainingsdaten für jedes Untersuchungsobjekt einer Vielzahl von Untersuchungsobjekten i) mindestens eine Eingabe-Repräsentation des Untersuchungsbereichs des Untersuchungsobjekts, ii) eine Ziel-Repräsentation des Untersuchungsbereichs des Untersuchungsobjekts und iii) eine transformierte Ziel-Repräsentation umfassen,
    - wobei die mindestens eine Eingabe-Repräsentation den Untersuchungsbereich des jeweiligen Untersuchungsobjekts in einer ersten Zeitspanne vor oder nach einer Applikation eines Kontrastmittels repräsentiert,
    - wobei die Ziel-Repräsentation den Untersuchungsbereich des jeweiligen Untersuchungsobjekts in einer zweiten Zeitspanne nach der Applikation des Kontrastmittels repräsentiert,
    - wobei die transformierte Ziel-Repräsentation zumindest einen Teil des Untersuchungsbereichs des jeweiligen Untersuchungsobjekts im Frequenzraum repräsentiert, falls die Ziel-Repräsentation den Untersuchungsbereichs des jeweiligen Untersuchungsobjekts im Ortsraum repräsentiert, oder im Ortsraum repräsentiert, falls die Ziel-Repräsentation den Untersuchungsbereich des jeweiligen Untersuchungsobjekts im Frequenzraum repräsentiert,
  - o wobei das Trainieren des Modells des maschinellen Lernens ein Reduzieren von Abweichungen zwischen i) zumindest einem Teil der synthetischen Repräsentation und zumindest einem Teil der Ziel-Repräsentation und ii) zwischen zumindest einem Teil einer transformierten synthetischen Repräsentation und zumindest einem Teil der transformierten Ziel-Repräsentation umfasst,

(220) Empfangen mindestens einer Eingabe-Repräsentation des Untersuchungsbereichs eines neuen Untersuchungsobjekts, wobei die mindestens eine Eingabe-Repräsentation des Untersuchungsbereichs des neuen Untersuchungsobjekts den Untersuchungsbereich in einer ersten Zeitspanne vor und/oder nach einer Applikation eines Kontrastmittels repräsentiert,

(230) Eingeben der mindestens einen Eingabe-Repräsentation des Untersuchungsbereichs des neuen Untersuchungsobjekts in das trainierte Modell des maschinellen Lernens,

(240) Empfangen einer synthetischen Repräsentation des Untersuchungsbereichs des neuen Untersuchungsobjekts von dem Modell des maschinellen Lernens,

(250) Ausgeben und/oder Speichern der synthetischen Repräsentation des Untersuchungsbereichs des neuen Untersuchungsobjekts und/oder Übermitteln der synthetischen Repräsentation des Untersuchungsbereichs des neuen Untersuchungsobjekts an ein separates Computersystem.

## Patentansprüche

1. Computer-implementiertes Verfahren zum Erzeugen einer synthetischen radiologischen Aufnahme umfassend:

   - Bereitstellen eines trainieren Modells ($MLM^t$) des maschinellen Lernens,

     ◦ wobei das trainierte Modell ($MLM^t$) des maschinellen Lernens anhand von Trainingsdaten (TD) trainiert worden ist, auf Basis mindestens einer Eingabe-Repräsentation (R1, R2) eines Untersuchungsbereichs eines Untersuchungsobjekts eine synthetische Repräsentation (SR) des Untersuchungsbereichs des Untersuchungsobjekts zu erzeugen,
     ◦ wobei die Trainingsdaten (TD) für jedes Untersuchungsobjekt einer Vielzahl von Untersuchungsobjekten i) mindestens eine Eingabe-Repräsentation (R1, R2) des Untersuchungsbereichs des Untersuchungsobjekts, ii) eine Ziel-Repräsentation (TR) des Untersuchungsbereichs des Untersuchungsobjekts und iii) eine transformierte Ziel-Repräsentation ($TR^T$) umfassen,

       ▪ wobei die mindestens eine Eingabe-Repräsentation (R1, R2) den Untersuchungsbereich des jeweiligen Untersuchungsobjekts in einer ersten Zeitspanne vor oder nach einer Applikation eines Kontrastmittels repräsentiert,
       ▪ wobei die Ziel-Repräsentation (TR) den Untersuchungsbereich des jeweiligen Untersuchungsobjekts in einer zweiten Zeitspanne nach der Applikation des Kontrastmittels repräsentiert, wobei zweite Zeitspanne der ersten Zeitspanne zeitlich nachgelagert ist,
       ▪ wobei die transformierte Ziel-Repräsentation ($TR^T$) zumindest einen Teil des Untersuchungsbereichs des jeweiligen Untersuchungsobjekts im Frequenzraum repräsentiert, falls die Ziel-Repräsentation (TR) den Untersuchungsbereichs des jeweiligen Untersuchungsobjekts im Ortsraum repräsentiert, oder im Ortsraum repräsentiert, falls die Ziel-Repräsentation (TR) den Untersuchungsbereich des jeweiligen Untersuchungsobjekts im Frequenzraum repräsentiert,

     ◦ wobei das Trainieren des Modells ($MLM^t$) des maschinellen Lernens ein Reduzieren von Abweichungen zwischen i) zumindest einem Teil der synthetischen Repräsentation (SR) und zumindest einem Teil der Ziel-Repräsentation (TR) und ii) zwischen zumindest einem Teil einer transformierten synthetischen Repräsentation ($SR^T$) und zumindest einem Teil der transformierten Ziel-Repräsentation ($TR^T$) umfasst,

   - Empfangen mindestens einer Eingabe-Repräsentation (R1*, R2*) des Untersuchungsbereichs eines neuen Untersuchungsobjekts, wobei die mindestens eine Eingabe-Repräsentation (R1*, R2*) des Untersuchungsbereichs des neuen Untersuchungsobjekts den Untersuchungsbereich in einer ersten Zeitspanne vor und/oder nach einer Applikation eines Kontrastmittels repräsentiert,
   - Eingeben der mindestens einen Eingabe-Repräsentation (R1*, R2*) des Untersuchungsbereichs des neuen Untersuchungsobjekts in das trainierte Modell ($MLM^t$) des maschinellen Lernens,
   - Empfangen einer synthetischen Repräsentation (SR*) des Untersuchungsbereichs des neuen Untersuchungsobjekts von dem Modell ($MLM^t$) des maschinellen Lernens,
   - Ausgeben und/oder Speichern der synthetischen Repräsentation (SR*) des Untersuchungsbereichs des neuen Untersuchungsobjekts und/oder Übermitteln der synthetischen Repräsentation (SR*) des Untersuchungsbereichs des neuen Untersuchungsobjekts an ein separates Computersystem.

2. Verfahren gemäß Anspruch 1, wobei das Trainieren des Modells ($MLM^t$) des maschinellen Lernens umfasst:

   - Empfangen und/oder Bereitstellen der Trainingsdaten (TD), wobei die Trainingsdaten (TD) für jedes Untersuchungsobjekt der Vielzahl von Untersuchungsobjekten einen Satz an Eingabedaten und Zieldaten umfasst,

     ◦ wobei jeder Satz die mindestens eine Eingabe-Repräsentation (R1, R2) des Untersuchungsbereichs des Untersuchungsobjekts als Eingabedaten und die Ziel-Repräsentation (TR) des Untersuchungsbereichs des Untersuchungsobjekts sowie die transformierte Ziel-Repräsentation ($TR^T$) als Zieldaten umfasst,
     ◦ wobei die mindestens eine Eingabe-Repräsentation (R1, R2) den Untersuchungsbereich in der ersten Zeitspanne vor und/oder nach der Applikation des Kontrastmittels repräsentiert und die Ziel-Repräsentation (TR) den Untersuchungsbereich in der zweiten Zeitspanne nach der Applikation des Kontrastmittels repräsentiert,
     ◦ wobei die transformierte Ziel-Repräsentation ($TR^T$) zumindest einen Teil des Untersuchungsbereichs des Untersuchungsobjekts

- im Frequenzraum repräsentiert, falls die Ziel-Repräsentation (TR) den Untersuchungsbereichs des Untersuchungsobjekts im Ortsraum repräsentiert, oder
- im Ortsraum repräsentiert, falls die Ziel-Repräsentation (TR) den Untersuchungsbereich des Untersuchungsobjekts im Frequenzraum repräsentiert,

- Trainieren eines Modells (MLM) des maschinellen Lernens, wobei das Modell (MLM) des maschinellen Lernens konfiguriert ist, auf Basis mindestens einer Eingabe-Repräsentation (R1, R2) eines Untersuchungsbereichs eines Untersuchungsobjekts und Modellparametern (MP) eine synthetische Repräsentation (SR) des Untersuchungsbereichs des Untersuchungsobjekts zu erzeugen,
wobei das Trainieren für jedes Untersuchungsobjekt der Vielzahl an Untersuchungsobjekten umfasst:

◦ Zuführen der mindestens einen Eingabe-Repräsentation (R1, R2) dem Modell des maschinellen Lernens,
◦ Empfangen einer synthetischen Repräsentation (SR) des Untersuchungsbereichs des Untersuchungsobjekts von dem Modell (MLM) des maschinellen Lernens,
◦ Erzeugen und/oder Empfangen einer transformierten synthetischen Repräsentation ($SR^T$) auf Basis der synthetischen Repräsentation (SR) und/oder zu der synthetischen Repräsentation (SR), wobei die transformierte synthetische Repräsentation ($SR^T$) zumindest einen Teil des Untersuchungsbereichs des Untersuchungsobjekts

  - im Frequenzraum repräsentiert, falls die synthetische Repräsentation (SR) den Untersuchungsbereich des Untersuchungsobjekts im Ortsraum repräsentiert, oder
  - im Ortsraum repräsentiert, falls die synthetische Repräsentation (SR) den Untersuchungsbereich des Untersuchungsobjekts im Frequenzraum repräsentiert,

◦ Quantifizieren der Abweichungen i) zwischen zumindest einem Teil der synthetischen Repräsentation (SR) und zumindest einem Teil der Ziel-Repräsentation (TR) und ii) zwischen zumindest einem Teil der transformierten synthetischen Repräsentation ($SR^T$) und zumindest einem Teil der transformierten Ziel-Repräsentation ($TR^T$) mittels einer Fehlerfunktion ($L$),
◦ Reduzieren der Abweichungen durch Modifizieren von Modellparametern (MP),

- Ausgeben und/oder Speichern des trainierten Modells ($MLM^t$) des maschinellen Lernens und/oder der Modellparameter (MP) und/oder Übermitteln des trainierten Modells ($MLM^t$) des maschinellen Lernens und/oder der Modellparameter (MP) an ein separates Computersystem.

**3.** Verfahren gemäß Anspruch 2,

wobei das Empfangen und/oder Bereitstellen von Trainingsdaten (TD) umfasst:

- Erzeugen einer anteiligen transformierten Ziel-Repräsentation ($TR^{T,P}$), wobei die anteilige transformierte Ziel-Repräsentation ($TR^{T,P}$) auf einen Teil oder mehrere Teile der transformierten Ziel-Repräsentation ($TR^T$) reduziert wird,

wobei das Erzeugen und/oder Empfangen einer transformierten synthetischen Repräsentation ($SR^T$) auf Basis und/oder zu der synthetischen Repräsentation (SR) umfasst:

- Erzeugen einer anteiligen transformierten synthetischen Repräsentation ($SR^{T,P}$), wobei die anteilige transformierte synthetische Repräsentation ($SR^{T,P}$) auf einen Teil oder mehrere Teile der transformierten synthetischen Repräsentation ($SR^T$) reduziert wird,

wobei das Quantifizieren der Abweichungen zwischen der transformierten synthetischen Repräsentation ($SR^T$) und der transformierten Ziel-Repräsentation ($TR^T$) umfasst:

- Quantifizieren der Abweichungen zwischen der anteiligen transformierten synthetischen Repräsentation ($SR^{T,P}$) und der anteiligen transformierten Ziel-Repräsentation ($TR^{T,P}$).

**4.** Verfahren gemäß einem der Ansprüche 1 bis 3,
wobei das Trainieren für jedes Untersuchungsobjekt der Vielzahl an Untersuchungsobjekten umfasst:

◦ Zuführen der mindestens einen Eingabe-Repräsentation (R1, R2) dem Modell des maschinellen Lernens (MLM),

◦ Empfangen der synthetischen Repräsentation (SR) und einer ersten transformierten synthetischen Repräsentation ($SR^T$) des Untersuchungsbereichs des Untersuchungsobjekts von dem Modell (MLM) des maschinellen Lernens, wobei die erste transformierte synthetische Repräsentation ($SR^T$) zumindest einen Teil des Untersuchungsbereichs des Untersuchungsobjekts

▪ im Frequenzraum repräsentiert, falls die synthetische Repräsentation (SR) den Untersuchungsbereichs des Untersuchungsobjekts im Ortsraum repräsentiert, oder

▪ im Ortsraum repräsentiert, falls die synthetische Repräsentation (SR) den Untersuchungsbereich des Untersuchungsobjekts im Frequenzraum repräsentiert,

◦ Erzeugen einer zweiten transformierten synthetischen Repräsentation ($SR^{T\#}$) auf Basis der synthetischen Repräsentation (SR) mittels einer Transformation (*T*), wobei die zweite transformierte synthetische Repräsentation ($SR^{T\#}$) zumindest einen Teil des Untersuchungsbereichs des Untersuchungsobjekts

▪ im Frequenzraum repräsentiert, falls die synthetische Repräsentation (SR) den Untersuchungsbereichs des Untersuchungsobjekts im Ortsraum repräsentiert, oder

▪ im Ortsraum repräsentiert, falls die synthetische Repräsentation (SR) den Untersuchungsbereich des Untersuchungsobjekts im Frequenzraum repräsentiert,

- Quantifizieren der Abweichungen i) zwischen zumindest einem Teil der synthetischen Repräsentation (SR) und zumindest einem Teil der Ziel-Repräsentation (TR), ii) zwischen zumindest einem Teil der ersten transformierten synthetischen Repräsentation ($SR^T$) und zumindest einem Teil der transformierten Ziel-Repräsentation ($TR^T$) und iii) zwischen zumindest einem Teil der ersten transformierten synthetischen Repräsentation ($SR^T$) und zumindest einem Teil der zweiten transformierten synthetischen Repräsentation ($SR^{T\#}$) mittels einer Fehlerfunktion (L),
- Reduzieren der Abweichungen durch Modifizieren von Modellparametern (MP).

**5.** Verfahren nach einem der Ansprüche 1 bis 4,

wobei das Modell (MLM) des maschinellen Lernens beim Trainieren ein erstes Modell (MLM1) des maschinellen Lernens und ein zweites Modell (MLM2) des maschinellen Lernens umfasst,

wobei das erste Modell (MLM1) des maschinellen Lernens konfiguriert ist, auf Basis mindestens einer Eingabe-Repräsentation (R1, R2) und Modellparametern (MP1) eine synthetische Repräsentation (SR) des Untersuchungsbereichs des Untersuchungsobjekts zu erzeugen,

wobei das zweite Modell (MLM2) des maschinellen Lernens konfiguriert ist, auf Basis der synthetischen Repräsentation (SR) des Untersuchungsbereichs des Untersuchungsobjekts und Modellparametern (MP2) mindestens eine Eingabe-Repräsentation (R1, R2) zu rekonstruieren,

wobei das Trainieren für jedes Untersuchungsobjekt der Vielzahl an Untersuchungsobjekten umfasst:

◦ Erzeugen einer transformierten Eingabe-Repräsentation (R2) auf Basis mindestens einer Eingabe-Repräsentation (R1) mittels einer Transformation (*T*), wobei die transformierte Eingabe-Repräsentation (R2) zumindest einen Teil des Untersuchungsbereichs des Untersuchungsobjekts

▪ im Frequenzraum repräsentiert, falls die mindestens eine Eingabe-Repräsentation (R1) den Untersuchungsbereichs des Untersuchungsobjekts im Ortsraum repräsentiert, oder

▪ im Ortsraum repräsentiert, falls die Eingabe-Repräsentation (R1) den Untersuchungsbereich des Untersuchungsobjekts im Frequenzraum repräsentiert,

◦ Zuführen der mindestens einen Eingabe-Repräsentation (R1) und/oder der transformierten Eingabe-Repräsentation (R2) dem ersten Modell (MLM1) des maschinellen Lernens,

◦ Empfangen einer synthetischen Repräsentation (SR) des Untersuchungsbereichs des Untersuchungsobjekts von dem ersten Modell (MLM1) des maschinellen Lernens,

◦ Erzeugen und/oder Empfangen einer transformierten synthetischen Repräsentation ($SR^T$) auf Basis und/oder zu der synthetischen Repräsentation (SR), wobei die transformierte synthetische Repräsentation ($SR^T$) zumindest einen Teil des Untersuchungsbereichs des Untersuchungsobjekts

- im Frequenzraum repräsentiert, falls die synthetische Repräsentation (SR) den Untersuchungsbereichs des Untersuchungsobjekts im Ortsraum repräsentiert, oder
- im Ortsraum repräsentiert, falls die synthetische Repräsentation (SR) den Untersuchungsbereich des Untersuchungsobjekts im Frequenzraum repräsentiert,

◦ Zuführen der synthetischen Repräsentation (SR) und/oder der transformierten synthetischen Repräsentation ($SR^T$) dem zweiten Modell (MLM2) des maschinellen Lernens,
◦ Empfangen einer vorhergesagten Eingabe-Repräsentation (R1#) von dem zweiten Modell (MLM2) des maschinellen Lernens,
◦ Erzeugen und/oder Empfangen einer transformierten vorhergesagten Eingabe-Repräsentation (R2#) auf Basis und/oder zu der vorhergesagten Eingabe-Repräsentation (R1#), wobei die transformierte vorhergesagte Eingabe-Repräsentation (R2#) zumindest einen Teil des Untersuchungsbereichs des Untersuchungsobjekts

- im Frequenzraum repräsentiert, falls die vorhergesagte Eingabe-Repräsentation (R1#) den Untersuchungsbereichs des Untersuchungsobjekts im Ortsraum repräsentiert, oder
- im Ortsraum repräsentiert, falls die vorhergesagte Eingabe-Repräsentation (R1#) den Untersuchungsbereich des Untersuchungsobjekts im Frequenzraum repräsentiert,

◦ Quantifizieren der Abweichungen i) zwischen zumindest einem Teil der synthetischen Repräsentation (SR) und zumindest einem Teil der Ziel-Repräsentation (TR), ii) zwischen zumindest einem Teil der transformierten synthetischen Repräsentation ($SR^T$) und zumindest einem Teil der transformierten Ziel-Repräsentation ($TR^T$), iii) zwischen zumindest einem Teil der Eingabe-Repräsentation (R1) und zumindest einem Teil der vorhergesagten Eingabe-Repräsentation (R1#) und iv) zwischen zumindest einem Teil der transformierten Eingabe-Repräsentation (R2) und zumindest einem Teil der transformierten vorhergesagten Eingabe-Repräsentation (R2#) mittels einer Fehlerfunktion (L),
◦ Reduzieren der Abweichungen durch Modifizieren von Modellparametern (MP).

**6.** Verfahren gemäß einem der Ansprüche 1 bis 5, wobei das Untersuchungsobjekt ein Säugetier, vorzugsweise ein Mensch ist.

**7.** Verfahren gemäß einem der Ansprüche 1 bis 6, wobei der Untersuchungsbereich eine Leber, ein Gehirn, ein Herz, eine Niere, eine Lunge, einen Magen, einen Darm, eine Bauchspeicheldrüse, eine Schilddrüse, eine Prostata, eine Brust eines Menschen ist oder umfasst.

**8.** Verfahren gemäß einem der Ansprüche 1 bis 7, wobei der Untersuchungsbereich eine Leber oder ein Teil einer Leber eines Menschen ist.

**9.** Verfahren gemäß einem der Ansprüche 1 bis 8, wobei jede der mindestens einen Eingabe-Repräsentation (R1, R2, R1*, R2*) eine Repräsentation des Untersuchungsbereichs im Ortsraum ist, die Ziel-Repräsentation (TR) eine Repräsentation des Untersuchungsbereichs im Ortsraum ist und die synthetische Repräsentation (SR, SR*) eine Repräsentation des Untersuchungsbereichs im Ortsraum ist.

**10.** Verfahren gemäß einem der Ansprüche 3 bis 9, wobei die anteilige transformierte synthetische Repräsentation ($SR^{T,P}$) den Untersuchungsbereich im Frequenzraum repräsentiert, wobei die anteilige transformierte synthetische Repräsentation ($SR^{T,P}$) auf einen Frequenzbereich der transformierten synthetischen Repräsentation ($SR^T$) reduziert wird, wobei in dem Frequenzbereich Kontrastinformationen kodiert sind.

**11.** Verfahren gemäß einem der Ansprüche 3 bis 9, wobei die anteilige transformierte synthetische Repräsentation ($SR^{T,P}$) den Untersuchungsbereich im Frequenzraum repräsentiert, wobei die anteilige transformierte synthetische Repräsentation ($SR^{T,P}$) auf einen Frequenzbereich der transformierten synthetischen Repräsentation ($SR^T$) reduziert wird, wobei in dem Frequenzbereich Informationen über Feinstrukturen kodiert sind.

**12.** Computersystem (10) zum Erzeugen einer synthetischen radiologischen Aufnahme umfassend

- eine Empfangseinheit (11),
- eine Steuer- und Recheneinheit (12) und
- eine Ausgabeeinheit (13),

- wobei die Steuer- und Recheneinheit (12) konfiguriert ist, ein trainiertes Modell ($MLM^t$) des maschinellen Lernens bereitzustellen,

◦ wobei das trainierte Modell ($MLM^t$) des maschinellen Lernens anhand von Trainingsdaten (TD) trainiert worden ist, auf Basis mindestens einer Eingabe-Repräsentation (R1, R2) eines Untersuchungsbereichs eines Untersuchungsobjekts eine synthetische Repräsentation (SR) des Untersuchungsbereichs des Untersuchungsobjekts zu erzeugen,
◦ wobei die Trainingsdaten (TD) für jedes Untersuchungsobjekt einer Vielzahl von Untersuchungsobjekten i) mindestens eine Eingabe-Repräsentation (R1, R2) des Untersuchungsbereichs des Untersuchungsobjekts, ii) eine Ziel-Repräsentation (TR) des Untersuchungsbereichs des Untersuchungsobjekts und iii) eine transformierte ($TR^T$) Ziel-Repräsentation umfassen,

▪ wobei die mindestens eine Eingabe-Repräsentation (R1, R2) den Untersuchungsbereich des jeweiligen Untersuchungsobjekts in einer ersten Zeitspanne vor oder nach einer Applikation eines Kontrastmittels repräsentiert,
▪ wobei die Ziel-Repräsentation (TR) den Untersuchungsbereich des jeweiligen Untersuchungsobjekts in einer zweiten Zeitspanne nach der Applikation des Kontrastmittels repräsentiert, wobei zweite Zeitspanne der ersten Zeitspanne zeitlich nachgelagert ist,
▪ wobei die transformierte Ziel-Repräsentation ($TR^T$) zumindest einen Teil des Untersuchungsbereichs des jeweiligen Untersuchungsobjekts im Frequenzraum repräsentiert, falls die Ziel-Repräsentation (TR) den Untersuchungsbereichs des jeweiligen Untersuchungsobjekts im Ortsraum repräsentiert, oder im Ortsraum repräsentiert, falls die Ziel-Repräsentation (TR) den Untersuchungsbereich des jeweiligen Untersuchungsobjekts im Frequenzraum repräsentiert,

◦ wobei das Trainieren des Modells ($MLM^t$) des maschinellen Lernens ein Reduzieren von Abweichungen zwischen i) zumindest einem Teil der synthetischen Repräsentation (SR) und zumindest einem Teil der Ziel-Repräsentation (TR) und ii) zwischen zumindest einem Teil einer transformierten synthetischen Repräsentation ($SR^T$) und zumindest einem Teil der transformierten Ziel-Repräsentation ($TR^T$) umfasst,

- wobei die Steuer- und Recheneinheit (12) konfiguriert ist, die Empfangseinheit (11) zu veranlassen, mindestens eine Eingabe-Repräsentation (R1*, R2*) eines Untersuchungsbereichs eines neuen Untersuchungsobjekts zu empfangen, wobei die mindestens eine Eingabe-Repräsentation (R1*, R2*) des Untersuchungsbereichs des neuen Untersuchungsobjekts den Untersuchungsbereich in einer ersten Zeitspanne vor und/oder nach einer Applikation eines Kontrastmittels repräsentiert,
- wobei die Steuer- und Recheneinheit (12) konfiguriert ist, die mindestens eine Eingabe-Repräsentation (R1*, R2*) des Untersuchungsbereichs des neuen Untersuchungsobjekts in ein trainiertes Modell ($MLM^t$) des maschinellen Lernens einzugeben,
- wobei die Steuer- und Recheneinheit (12) konfiguriert ist, von dem Modell ($MLM^t$) des maschinellen Lernens eine synthetische Repräsentation (SR*) des Untersuchungsbereichs des neuen Untersuchungsobjekts zu empfangen,
- wobei die Steuer- und Recheneinheit (12) konfiguriert ist, die Ausgabeeinheit (13) zu veranlassen, die synthetische Repräsentation (SR*) des Untersuchungsbereichs des neuen Untersuchungsobjekts auszugeben und/oder zu speichern und/oder an ein separates Computersystem zu übermitteln.

**13.** Computerprogrammprodukt zum Erzeugen einer synthetischen radiologischen Aufnahme umfassend ein Computerprogramm (40), das in einen Arbeitsspeicher (22) eines Computersystems (1) geladen werden kann und dort das Computersystem (1) dazu veranlasst, folgende Schritte ausführen:

- Bereitstellen eines trainieren Modells ($MLM^t$) des maschinellen Lernens,

◦ wobei das trainierte Modell ($MLM^t$) des maschinellen Lernens anhand von Trainingsdaten (TD) trainiert worden ist, auf Basis mindestens einer Eingabe-Repräsentation (R1, R2) eines Untersuchungsbereichs eines Untersuchungsobjekts eine synthetische Repräsentation (SR) des Untersuchungsbereichs des Untersuchungsobjekts zu erzeugen,
◦ wobei die Trainingsdaten (TD) für jedes Untersuchungsobjekt einer Vielzahl von Untersuchungsobjekten i) mindestens eine Eingabe-Repräsentation (R1, R2) des Untersuchungsbereichs des Untersuchungsobjekts, ii) eine Ziel-Repräsentation (TR) des Untersuchungsbereichs des Untersuchungsobjekts und iii) eine transformierte Ziel-Repräsentation ($TR^T$) umfassen,

▪ wobei die mindestens eine Eingabe-Repräsentation (R1, R2) den Untersuchungsbereich des jeweiligen Untersuchungsobjekts in einer ersten Zeitspanne vor oder nach einer Applikation eines Kontrastmittels repräsentiert,

▪ wobei die Ziel-Repräsentation (TR) den Untersuchungsbereich des jeweiligen Untersuchungsobjekts in einer zweiten Zeitspanne nach der Applikation des Kontrastmittels repräsentiert, wobei zweite Zeitspanne der ersten Zeitspanne zeitlich nachgelagert ist,

▪ wobei die transformierte Ziel-Repräsentation ($TR^T$) zumindest einen Teil des Untersuchungsbereichs des jeweiligen Untersuchungsobjekts im Frequenzraum repräsentiert, falls die Ziel-Repräsentation (TR) den Untersuchungsbereichs des jeweiligen Untersuchungsobjekts im Ortsraum repräsentiert, oder im Ortsraum repräsentiert, falls die Ziel-Repräsentation (TR) den Untersuchungsbereich des jeweiligen Untersuchungsobjekts im Frequenzraum repräsentiert,

◦ wobei das Trainieren des Modells ($MLM^t$) des maschinellen Lernens ein Reduzieren von Abweichungen zwischen i) zumindest einem Teil der synthetischen Repräsentation (SR) und zumindest einem Teil der Ziel-Repräsentation (TR) und ii) zwischen zumindest einem Teil einer transformierten synthetischen Repräsentation ($SR^T$) und zumindest einem Teil der transformierten Ziel-Repräsentation ($TR^T$) umfasst,

- Empfangen mindestens einer Eingabe-Repräsentation (R1*, R2*) des Untersuchungsbereichs eines neuen Untersuchungsobjekts, wobei die mindestens eine Eingabe-Repräsentation (R1*, R2*) des Untersuchungsbereichs des neuen Untersuchungsobjekts den Untersuchungsbereich in einer ersten Zeitspanne vor und/oder nach einer Applikation eines Kontrastmittels repräsentiert,
- Eingeben der mindestens einen Eingabe-Repräsentation (R1*, R2*) des Untersuchungsbereichs des neuen Untersuchungsobjekts in das trainierte Modell ($MLM^t$) des maschinellen Lernens,
- Empfangen einer synthetischen Repräsentation (SR*) des Untersuchungsbereichs des neuen Untersuchungsobjekts von dem Modell ($MLM^t$) des maschinellen Lernens,
- Ausgeben und/oder Speichern der synthetischen Repräsentation (SR*) des Untersuchungsbereichs des neuen Untersuchungsobjekts und/oder Übermitteln der synthetischen Repräsentation (SR*) des Untersuchungsbereichs des neuen Untersuchungsobjekts an ein separates Computersystem.

**14.** Verwendung eines Kontrastmittels in einem radiologischen Untersuchungsverfahren, wobei das radiologische Untersuchungsverfahren umfasst:

- Bereitstellen eines trainieren Modells ($MLM^t$) des maschinellen Lernens,

◦ wobei das trainierte Modell ($MLM^t$) des maschinellen Lernens anhand von Trainingsdaten (TD) trainiert worden ist, auf Basis mindestens einer Eingabe-Repräsentation (R1, R2) eines Untersuchungsbereichs eines Untersuchungsobjekts eine synthetische Repräsentation (SR) des Untersuchungsbereichs des Untersuchungsobjekts zu erzeugen,

◦ wobei die Trainingsdaten (TD) für jedes Untersuchungsobjekt einer Vielzahl von Untersuchungsobjekten i) mindestens eine Eingabe-Repräsentation (R1, R2) des Untersuchungsbereichs des Untersuchungsobjekts, ii) eine Ziel-Repräsentation (TR) des Untersuchungsbereichs des Untersuchungsobjekts und iii) eine transformierte Ziel-Repräsentation ($TR^T$) umfassen,

▪ wobei die mindestens eine Eingabe-Repräsentation (R1, R2) den Untersuchungsbereich des jeweiligen Untersuchungsobjekts in einer ersten Zeitspanne vor oder nach einer Applikation des Kontrastmittels repräsentiert,

▪ wobei die Ziel-Repräsentation (TR) den Untersuchungsbereich des jeweiligen Untersuchungsobjekts in einer zweiten Zeitspanne nach der Applikation des Kontrastmittels repräsentiert, wobei zweite Zeitspanne der ersten Zeitspanne zeitlich nachgelagert ist,

▪ wobei die transformierte Ziel-Repräsentation ($TR^T$) zumindest einen Teil des Untersuchungsbereichs des jeweiligen Untersuchungsobjekts im Frequenzraum repräsentiert, falls die Ziel-Repräsentation (TR) den Untersuchungsbereichs des jeweiligen Untersuchungsobjekts im Ortsraum repräsentiert, oder im Ortsraum repräsentiert, falls die Ziel-Repräsentation (TR) den Untersuchungsbereich des jeweiligen Untersuchungsobjekts im Frequenzraum repräsentiert,

◦ wobei das Trainieren des Modells ($MLM^t$) des maschinellen Lernens ein Reduzieren von Abweichungen zwischen i) zumindest einem Teil der synthetischen Repräsentation (SR) und zumindest einem Teil der Ziel-Repräsentation (TR) und ii) zwischen zumindest einem Teil einer transformierten synthetischen Repräsentation ($SR^T$) und zumindest einem Teil der transformierten Ziel-Repräsentation ($TR^T$) umfasst,

- Empfangen mindestens einer Eingabe-Repräsentation (R1*, R2*) des Untersuchungsbereichs eines neuen Untersuchungsobjekts, wobei die mindestens eine Eingabe-Repräsentation (R1*, R2*) des Untersuchungsbereichs des neuen Untersuchungsobjekts den Untersuchungsbereich in einer ersten Zeitspanne vor und/oder nach einer Applikation eines Kontrastmittels repräsentiert,
- Eingeben der mindestens einen Eingabe-Repräsentation (R1*, R2*) des Untersuchungsbereichs des neuen Untersuchungsobjekts in das trainierte Modell ($MLM^t$) des maschinellen Lernens,
- Empfangen einer synthetischen Repräsentation (SR*) des Untersuchungsbereichs des neuen Untersuchungsobjekts von dem Modell ($MLM^t$) des maschinellen Lernens,
- Ausgeben und/oder Speichern der synthetischen Repräsentation (SR*) des Untersuchungsbereichs des neuen Untersuchungsobjekts und/oder Übermitteln der synthetischen Repräsentation (SR*) des Untersuchungsbereichs des neuen Untersuchungsobjekts an ein separates Computersystem.

**15.** Kit umfassend ein Kontrastmittel und ein Computerprogrammprodukt umfassend ein Computerprogramm (40), das in einen Arbeitsspeicher (22) eines Computersystems (1) geladen werden kann und dort das Computersystem (1) dazu veranlasst, folgende Schritte ausführen:

- Bereitstellen eines trainieren Modells ($MLM^t$) des maschinellen Lernens,

  ◦ wobei das trainierte Modell ($MLM^t$) des maschinellen Lernens anhand von Trainingsdaten (TD) trainiert worden ist, auf Basis mindestens einer Eingabe-Repräsentation (R1, R2) eines Untersuchungsbereichs eines Untersuchungsobjekts eine synthetische Repräsentation (SR) des Untersuchungsbereichs des Untersuchungsobjekts zu erzeugen,
  ◦ wobei die Trainingsdaten (TD) für jedes Untersuchungsobjekt einer Vielzahl von Untersuchungsobjekten i) mindestens eine Eingabe-Repräsentation (R1, R2) des Untersuchungsbereichs des Untersuchungsobjekts, ii) eine Ziel-Repräsentation (TR) des Untersuchungsbereichs des Untersuchungsobjekts und iii) eine transformierte Ziel-Repräsentation ($TR^T$) umfassen,

    ▪ wobei die mindestens eine Eingabe-Repräsentation (R1, R2) den Untersuchungsbereich des jeweiligen Untersuchungsobjekts in einer ersten Zeitspanne vor oder nach einer Applikation des Kontrastmittels repräsentiert,
    ▪ wobei die Ziel-Repräsentation (TR) den Untersuchungsbereich des jeweiligen Untersuchungsobjekts in einer zweiten Zeitspanne nach der Applikation des Kontrastmittels repräsentiert, wobei zweite Zeitspanne der ersten Zeitspanne zeitlich nachgelagert ist,
    ▪ wobei die transformierte Ziel-Repräsentation ($TR^T$) zumindest einen Teil des Untersuchungsbereichs des jeweiligen Untersuchungsobjekts im Frequenzraum repräsentiert, falls die Ziel-Repräsentation (TR) den Untersuchungsbereichs des jeweiligen Untersuchungsobjekts im Ortsraum repräsentiert, oder im Ortsraum repräsentiert, falls die Ziel-Repräsentation (TR) den Untersuchungsbereich des jeweiligen Untersuchungsobjekts im Frequenzraum repräsentiert,

  ◦ wobei das Trainieren des Modells ($MLM^t$) des maschinellen Lernens ein Reduzieren von Abweichungen zwischen i) zumindest einem Teil der synthetischen Repräsentation (SR) und zumindest einem Teil der Ziel-Repräsentation (TR) und ii) zwischen zumindest einem Teil einer transformierten synthetischen Repräsentation ($SR^T$) und zumindest einem Teil der transformierten Ziel-Repräsentation ($TR^T$) umfasst,

- Empfangen mindestens einer Eingabe-Repräsentation (R1*, R2*) des Untersuchungsbereichs eines neuen Untersuchungsobjekts, wobei die mindestens eine Eingabe-Repräsentation (R1*, R2*) des Untersuchungsbereichs des neuen Untersuchungsobjekts den Untersuchungsbereich in einer ersten Zeitspanne vor und/oder nach einer Applikation eines Kontrastmittels repräsentiert,
- Eingeben der mindestens einen Eingabe-Repräsentation (R1*, R2*) des Untersuchungsbereichs des neuen Untersuchungsobjekts in das trainierte Modell ($MLM^t$) des maschinellen Lernens,
- Empfangen einer synthetischen Repräsentation (SR*) des Untersuchungsbereichs des neuen Untersuchungsobjekts von dem Modell ($MLM^t$) des maschinellen Lernens,
- Ausgeben und/oder Speichern der synthetischen Repräsentation (SR*) des Untersuchungsbereichs des neuen Untersuchungsobjekts und/oder Übermitteln der synthetischen Repräsentation (SR*) des Untersuchungsbereichs des neuen Untersuchungsobjekts an ein separates Computersystem.

**16.** Kit gemäß Anspruch 15, wobei das Kontrastmittel eines oder mehrere Kontrastmittel ausgewählt aus der folgenden Liste ist oder umfasst:

Gadoxetat-Dinatrium,
Gadolinium(III) 2-[4,7,10-tris(carboxymethyl)-1,4,7,10-tetrazacyclododec-1-yl]essigsäure,
Gadolinium(III) Ethoxybenzyl-diethylenetriaminepentaessigsäure,
Gadolinium(III) 2-[3,9-bis[1-carboxylato-4-(2,3-dihydroxypropylamino)-4-oxobutyl]-3,6,9,15-tetrazabicyclo [9.3.1]pentadeca-1(15),11,13-trien-6-yl]-5-(2,3-dihydroxypropylamino)-5-oxopentanoat,
Dihydrogen[(+)-4-carboxy-5,8,11-tris(carboxymethyl)-1-phenyl-2-oxa-5,8,11-triazatridecan-13-oato(5-)]gadolinat(2-),
Tetragadolinium-[4,10-bis(carboxylatomethyl)-7-13,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetat, Gadolinium 2,2',2"-(10-{1-carboxy-2-[2-(4-ethoxyphenyl)ethoxy]ethyl}-1,4,7,10-tetraazacyclododecan-1,4,7-triyl)triacetat,
Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat,
Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat,
Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat),
Gadolinium 2,2',2"-{10-[(1S)-4-(4-butoxyphenyl)-1-carboxybutyl]-1,4,7,10-tetraazacyclododecan-1,4,7-triyl}triacetat,
Gadolinium-2,2',2"-{(2S)-10-(carboxymethyl)-2-[4-(2-ethoxyethoxy)benzyl]-1,4,7,10-tetraazacyclododecane-1,4,7-triyl}triacetat,
Gadolinium-2,2',2"-[10-(carboxymethyl)-2-(4-ethoxybenzyl)-1,4,7,10-tetraazacyclododecane-1,4,7-triyl]triacetat,
Gadolinium(III) 5,8-bis(carboxylatomethyl)-2-[2-(methylamino)-2-oxoethyl]-10-oxo-2,5,8,11-tetraazadodecan-1-carboxylat-hydrat,
Gadolinium(III) 2-[4-(2-hydroxypropyl)-7,10-bis(2-oxido-2-oxoethyl)-1,4,7,10-tetrazacyclododec-1-yl]acetat,
Gadolinium(III) 2,2',2"-(10-((2R,3S)-1,3,4-trihydroxybutan-2-yl)-1,4,7,10-tetraazacyclododecan-1,4,7-triyl)triacetat,
ein $Gd^{3+}$-Komplex einer Verbindung der Formel (I)

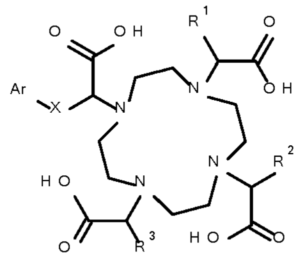

(I) ,

wobei
Ar eine Gruppe ausgewählt aus

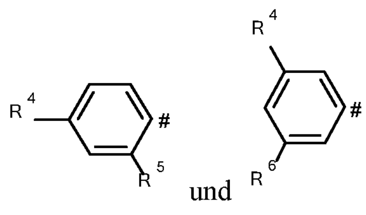

darstellt,
wobei # die Anknüpfung zu X darstellt,

X eine Gruppe darstellt, die aus
$CH_2$, $(CH_2)_2$, $(CH_2)_3$, $(CH_2)_4$ und $*-(CH_2)_2-O-CH_2{}^{\#}$ ausgewählt wird,
wobei * die Anknüpfung zu Ar darstellt und # die Anknüpfung zum Essigsäurerest darstellt, $R^1$, $R^2$ and $R^3$ unabhängig voneinander ein Wasserstoffatom oder eine Gruppe ausgewählt aus $C_1$-$C_3$-Alkyl, $-CH_2OH$, $-(CH_2)_2OH$ und $-CH_2OCH_3$ darstellen,
$R^4$ eine Gruppe ausgewählt aus $C_2$-$C_4$-Alkoxy, $(H_3C-CH_2)-O-(CH_2)_2-O-$, $(H_3C-CH_2)-O-(CH_2)_2-O-(CH_2)_2-O-$ und $(H_3C-CH_2)-O-(CH_2)_2-O-(CH_2)_2-O-(CH_2)_2-O-$ darstellt,
$R^5$ ein Wasserstoffatom darstellt,
und
$R^6$ ein Wasserstoffatom darstellt,
oder ein Stereoisomer, Tautomer, Hydrat, Solvat oder Salz davon, oder eine Mischung davon,
ein $Gd^{3+}$-Komplex einer Verbindung der Formel (II)

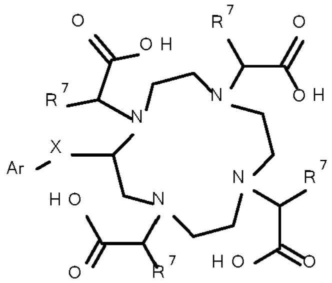

(II) ,

wobei
Ar eine Gruppe ausgewählt aus

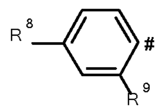
and
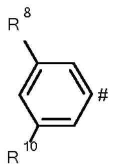

darstellt,
wobei # die Anknüpfung zu X darstellt,
X eine Gruppe darstellt, die aus $CH_2$, $(CH_2)_2$, $(CH_2)_3$, $(CH_2)_4$ and $*-(CH_2)_2-O-CH_2-{}^{\#}$ ausgewählt wird, wobei * die Anknüpfung zu Ar darstellt und # die Anknüpfung zum Essigsäurerest darstellt,
$R^7$ ein Wasserstoffatom oder eine Gruppe ausgewählt aus $C_1$-$C_3$-Alkyl, $-CH_2OH$, $-(CH_2)_2OH$ und $-CH_2OCH_3$ darstellt;
$R^8$ eine Gruppe ausgewählt aus $C_2$-$C_4$-Alkoxy, $(H_3C-CH_2O)-(CH_2)_2-O-$, $(H_3C-H_2O)-(CH_2)_2-O-(CH_2)_2-O-$ und $(H_3C-CH_2O)-(CH_2)_2-O-(CH_2)_2-O-(CH_2)_2-O-$ darstellt;
$R^9$ und $R^{10}$ unabhängig voneinander ein Wasserstoffatom darstellen;
oder ein Stereoisomer, Tautomer, Hydrat, Solvat oder Salz davon, oder eine Mischung davon.

## Claims

**1.** Computer-implemented method for generating a synthetic radiological image, comprising:

- providing a trained machine-learning model ($MLM^t$),

  ◦ the trained machine-learning model ($MLM^t$) having been trained by means of training data (TD) to generate on the basis of at least one input representation (R1, R2) of an examination region of an examination object a synthetic representation (SR) of the examination region of the examination object,
  ◦ the training data (TD) comprising for each examination object of a multiplicity of examination objects i) at

least one input representation (R1, R2) of the examination region of the examination object, ii) a target representation (TR) of the examination region of the examination object and iii) a transformed target representation ($TR^T$),

- ▪ the at least one input representation (R1, R2) representing the examination region of the respective examination object in a first period of time before or after administration of a contrast agent,
- ▪ the target representation (TR) representing the examination region of the respective examination object in a second period of time after the administration of the contrast agent, the second period of time following the first period of time,
- ▪ the transformed target representation ($TR^T$) representing at least part of the examination region of the respective examination object in frequency space, if the target representation (TR) represents the examination region of the respective examination object in real space, or in real space, if the target representation (TR) represents the examination region of the respective examination object in frequency space,

◦ the training of the machine-learning model ($MLM^t$) comprising reducing differences between i) at least part of the synthetic representation (SR) and at least part of the target representation (TR) and ii) between at least part of a transformed synthetic representation ($SR^T$) and at least part of the transformed target representation ($TR^T$),

- receiving at least one input representation (R1*, R2*) of the examination region of a new examination object, the at least one input representation (R1*, R2*) of the examination region of the new examination object representing the examination region in a first period of time before and/or after administration of a contrast agent,
- inputting the at least one input representation (R1*, R2*) of the examination region of the new examination object into the trained machine-learning model ($MLM^t$),
- receiving a synthetic representation (SR*) of the examination region of the new examination object from the machine-learning model ($MLM^t$),
- outputting and/or storing the synthetic representation (SR*) of the examination region of the new examination object and/or transmitting the synthetic representation (SR*) of the examination region of the new examination object to a separate computer system.

**2.** Method according to Claim 1, wherein the training of the machine-learning model ($MLM^t$) comprises:

- receiving and/or providing the training data (TD), the training data (TD) comprising a set of input data and target data for each examination object of the multiplicity of examination objects,

◦ each set comprising the at least one input representation (R1, R2) of the examination region of the examination object as input data and the target representation (TR) of the examination region of the examination object and the transformed target representation ($TR^T$) as target data,
◦ the at least one input representation (R1, R2) representing the examination region in the first period of time before and/or after the administration of the contrast agent and the target representation (TR) representing the examination region in the second period of time after the administration of the contrast agent,
◦ the transformed target representation ($TR^T$) representing at least part of the examination region of the examination object

- ▪ in frequency space, if the target representation (TR) represents the examination region of the examination object in real space, or
- ▪ in real space, if the target representation (TR) represents the examination region of the examination object in frequency space,

- training a machine-learning model (MLM), the machine-learning model (MLM) being configured to generate on the basis of at least one input representation (R1, R2) of an examination region of an examination object and model parameters (MP) a synthetic representation (SR) of the examination region of the examination object, wherein the training comprises for each examination object of the multiplicity of examination objects:

◦ feeding the at least one input representation (R1, R2) to the machine-learning model,
◦ receiving a synthetic representation (SR) of the examination region of the examination object from the machine-learning model (MLM),

◦ generating and/or receiving a transformed synthetic representation ($SR^T$) on the basis of the synthetic representation (SR) and/or in relation to the synthetic representation (SR), the transformed synthetic representation ($SR^T$) representing at least part of the examination region of the examination object

▪ in frequency space, if the synthetic representation (SR) represents the examination region of the examination object in real space, or
▪ in real space, if the synthetic representation (SR) represents the examination region of the examination object in frequency space,

◦ quantifying the differences i) between at least part of the synthetic representation (SR) and at least part of the target representation (TR) and ii) between at least part of the transformed synthetic representation ($SR^T$) and at least part of the transformed target representation ($TR^T$) by means of a loss function (L),
◦ reducing the differences by modifying model parameters (MP),

- outputting and/or storing the trained machine-learning model ($MLM^t$) and/or the model parameters (MP) and/or transmitting the trained machine-learning model ($MLM^t$) and/or the model parameters (MP) to a separate computer system.

**3.** Method according to Claim 2,

wherein the receiving and/or providing of training data (TD) comprises:

- generating a partial transformed target representation ($TR^{T,P}$), the partial transformed target representation ($TR^{T,P}$) being reduced to one part or multiple parts of the transformed target representation ($TR^T$),

wherein the generating and/or receiving of a transformed synthetic representation ($SR^T$) on the basis of and/or in relation to the synthetic representation (SRT) comprises:

- generating a partial transformed synthetic representation ($SR^{T,P}$), the partial transformed synthetic representation ($SR^{T,P}$) being reduced to one part or multiple parts of the transformed synthetic representation ($SR^T$),

wherein the quantifying of the differences between the transformed synthetic representation ($SR^T$) and the transformed target representation ($TR^T$) comprises:

- quantifying the differences between the partial transformed synthetic representation ($SR^{T,P}$) and the partial transformed target representation ($TR^{T,P}$).

**4.** Method according to any of Claims 1 to 3,

wherein the training comprises for each examination object of the multiplicity of examination objects:

◦ feeding the at least one input representation (R1, R2) to the machine-learning model (MLM),
◦ receiving the synthetic representation (SR) and a first transformed synthetic representation ($SR^T$) of the examination region of the examination object from the machine-learning model (MLM), the first transformed synthetic representation ($SR^T$) representing at least part of the examination region of the examination object

▪ in frequency space, if the synthetic representation (SR) represents the examination region of the examination object in real space, or
▪ in real space, if the synthetic representation (SR) represents the examination region of the examination object in frequency space,

◦ generating a second transformed synthetic representation ($SR^{T\#}$) on the basis of the synthetic representation (SR) by means of a transform (T), the second transformed synthetic representation ($SR^{T\#}$) representing at least part of the examination region of the examination object

▪ in frequency space, if the synthetic representation (SR) represents the examination region of the examination object in real space, or

- in real space, if the synthetic representation (SR) represents the examination region of the examination object in frequency space,

- quantifying the differences i) between at least part of the synthetic representation (SR) and at least part of the target representation (TR), ii) between at least part of the first transformed synthetic representation ($SR^T$) and at least part of the transformed target representation ($TR^T$) and iii) between at least part of the first transformed synthetic representation ($SR^T$) and at least part of the second transformed synthetic representation ($SR^{T\#}$) by means of a loss function (L),
- reducing the differences by modifying model parameters (MP).

**5.** Method according to any of Claims 1 to 4,

wherein the machine-learning model (MLM) undergoing training comprises a first machine-learning model (MLM1) and a second machine-learning model (MLM2),
wherein the first machine-learning model (MLM1) is configured to generate on the basis of at least one input representation (R1, R2) and model parameters (MP1) a synthetic representation (SR) of the examination region of the examination object,
wherein the second machine-learning model (MLM2) is configured to reconstruct on the basis of the synthetic representation (SR) of the examination region of the examination object and model parameters (MP2) at least one input representation (R1, R2),
wherein the training comprises for each examination object of the multiplicity of examination objects:

◦ generating a transformed input representation (R2) on the basis of at least one input representation (R1) by means of a transform (T), the transformed input representation (R2) representing at least part of the examination region of the examination object

- in frequency space, if the at least one input representation (R1) represents the examination region of the examination object in real space, or
- in real space, if the input representation (R1) represents the examination region of the examination object in frequency space,

◦ feeding the at least one input representation (R1) and/or the transformed input representation (R2) to the first machine-learning model (MLM1),
◦ receiving a synthetic representation (SR) of the examination region of the examination object from the first machine-learning model (MLM1),
◦ generating and/or receiving a transformed synthetic representation ($SR^T$) on the basis of and/or in relation to the synthetic representation (SR), the transformed synthetic representation ($SR^T$) representing at least part of the examination region of the examination object

- in frequency space, if the synthetic representation (SR) represents the examination region of the examination object in real space, or
- in real space, if the synthetic representation (SR) represents the examination region of the examination object in frequency space,

◦ feeding the synthetic representation (SR) and/or the transformed synthetic representation ($SR^T$) to the second machine-learning model (MLM2),
◦ receiving a predicted input representation (R1#) from the second machine-learning model (MLM2),
◦ generating and/or receiving a transformed predicted input representation (R2#) on the basis of and/or in relation to the predicted input representation (R1#), the transformed predicted input representation (R2#) representing at least part of the examination region of the examination object

- in frequency space, if the predicted input representation (R1#) represents the examination region of the examination object in real space, or
- in real space, if the predicted input representation (R1#) represents the examination region of the examination object in frequency space,

◦ quantifying the differences i) between at least part of the synthetic representation (SR) and at least part of the target representation (TR), ii) between at least part of the transformed synthetic representation ($SR^T$) and at

least part of the transformed target representation ($TR^T$), iii) between at least part of the input representation (R1) and at least part of the predicted input representation (R1#) and iv) between at least part of the transformed input representation (R2) and at least part of the transformed predicted input representation (R2#) by means of a loss function (L),
◦ reducing the differences by modifying model parameters (MP).

**6.** Method according to any of Claims 1 to 5, wherein the examination object is a mammal, preferably a human.

**7.** Method according to any of Claims 1 to 6, wherein the examination region is or includes a liver, brain, heart, kidney, lung, stomach, intestine, pancreas, thyroid gland, prostate or breast of a human.

**8.** Method according to any of Claims 1 to 7, wherein the examination region is a liver or part of a liver of a human.

**9.** Method according to any of Claims 1 to 8, wherein each input representation of the at least one input representation (R1, R2, R1*, R2*) is a representation of the examination region in real space, the target representation (TR) is a representation of the examination region in real space, and the synthetic representation (SR, SR*) is a representation of the examination region in real space.

**10.** Method according to any of Claims 3 to 9, wherein the partial transformed synthetic representation ($SR^{T,P}$) represents the examination region in frequency space, the partial transformed synthetic representation ($SR^{T,P}$) being reduced to a frequency range of the transformed synthetic representation ($SR^T$), contrast information being encoded in the frequency range.

**11.** Method according to any of Claims 3 to 9, wherein the partial transformed synthetic representation ($SR^{T,P}$) represents the examination region in frequency space, the partial transformed synthetic representation ($SR^{T,P}$) being reduced to a frequency range of the transformed synthetic representation ($SR^T$), information about fine structures being encoded in the frequency range.

**12.** Computer system (10) for generating a synthetic radiological image, comprising

- a receiving unit (11),
- a control and calculation unit (12) and
- an output unit (13),

- wherein the control and calculation unit (12) is configured to provide a trained machine-learning model ($MLM^t$),

◦ the trained machine-learning model ($MLM^t$) having been trained by means of training data (TD) to generate on the basis of at least one input representation (R1, R2) of an examination region of an examination object a synthetic representation (SR) of the examination region of the examination object,
◦ the training data (TD) comprising for each examination object of a multiplicity of examination objects i) at least one input representation (R1, R2) of the examination region of the examination object, ii) a target representation (TR) of the examination region of the examination object and iii) a transformed target representation ($TR^T$),

▪ the at least one input representation (R1, R2) representing the examination region of the respective examination object in a first period of time before or after administration of a contrast agent,
▪ the target representation (TR) representing the examination region of the respective examination object in a second period of time after the administration of the contrast agent, the second period of time following the first period of time,
▪ the transformed target representation ($TR^T$) representing at least part of the examination region of the respective examination object in frequency space, if the target representation (TR) represents the examination region of the respective examination object in real space, or in real space, if the target representation (TR) represents the examination region of the respective examination object in frequency space,

◦ the training of the machine-learning model ($MLM^t$) comprising reducing differences between i) at least

part of the synthetic representation (SR) and at least part of the target representation (TR) and ii) between at least part of a transformed synthetic representation ($SR^T$) and at least part of the transformed target representation ($TR^T$),

- wherein the control and calculation unit (12) is configured to cause the receiving unit (11) to receive at least one input representation (R1*, R2*) of an examination region of a new examination object, the at least one input representation (R1*, R2*) of the examination region of the new examination object representing the examination region in a first period of time before and/or after administration of a contrast agent,
- wherein the control and calculation unit (12) is configured to input the at least one input representation (R1*, R2*) of the examination region of the new examination object into a trained machine-learning model ($MLM^t$),
- wherein the control and calculation unit (12) is configured to receive from the machine-learning model ($MLM^t$) a synthetic representation (SR*) of the examination region of the new examination object,
- wherein the control and calculation unit (12) is configured to cause the output unit (13) to output the synthetic representation (SR*) of the examination region of the new examination object and/or to store it and/or to transmit it to a separate computer system.

**13.** Computer program product for generating a synthetic radiological image, comprising a computer program (40) that can be loaded into a working memory (22) of a computer system (1), where it causes the computer system (1) to execute the following steps:

- providing a trained machine-learning model ($MLM^t$),

  ◦ the trained machine-learning model ($MLM^t$) having been trained by means of training data (TD) to generate on the basis of at least one input representation (R1, R2) of an examination region of an examination object a synthetic representation (SR) of the examination region of the examination object,
  ◦ the training data (TD) comprising for each examination object of a multiplicity of examination objects i) at least one input representation (R1, R2) of the examination region of the examination object, ii) a target representation (TR) of the examination region of the examination object and iii) a transformed target representation ($TR^T$),

    ▪ the at least one input representation (R1, R2) representing the examination region of the respective examination object in a first period of time before or after administration of a contrast agent,
    ▪ the target representation (TR) representing the examination region of the respective examination object in a second period of time after the administration of the contrast agent, the second period of time following the first period of time,
    ▪ the transformed target representation ($TR^T$) representing at least part of the examination region of the respective examination object in frequency space, if the target representation (TR) represents the examination region of the respective examination object in real space, or in real space, if the target representation (TR) represents the examination region of the respective examination object in frequency space,

  ◦ the training of the machine-learning model ($MLM^t$) comprising reducing differences between i) at least part of the synthetic representation (SR) and at least part of the target representation (TR) and ii) between at least part of a transformed synthetic representation ($SR^T$) and at least part of the transformed target representation ($TR^T$),

- receiving at least one input representation (R1*, R2*) of the examination region of a new examination object, the at least one input representation (R1*, R2*) of the examination region of the new examination object representing the examination region in a first period of time before and/or after administration of a contrast agent,
- inputting the at least one input representation (R1*, R2*) of the examination region of the new examination object into the trained machine-learning model ($MLM^t$),
- receiving a synthetic representation (SR*) of the examination region of the new examination object from the machine-learning model ($MLM^t$),
- outputting and/or storing the synthetic representation (SR*) of the examination region of the new examination object and/or transmitting the synthetic representation (SR*) of the examination region of the new examination object to a separate computer system.

**14.** Use of a contrast agent in a radiological examination method, the radiological examination method comprising:

- providing a trained machine-learning model ($MLM^t$),

  ◦ the trained machine-learning model ($MLM^t$) having been trained by means of training data (TD) to generate on the basis of at least one input representation (R1, R2) of an examination region of an examination object a synthetic representation (SR) of the examination region of the examination object,
  ◦ the training data (TD) comprising for each examination object of a multiplicity of examination objects i) at least one input representation (R1, R2) of the examination region of the examination object, ii) a target representation (TR) of the examination region of the examination object and iii) a transformed target representation ($TR^T$),

    ▪ the at least one input representation (R1, R2) representing the examination region of the respective examination object in a first period of time before or after administration of the contrast agent,
    ▪ the target representation (TR) representing the examination region of the respective examination object in a second period of time after the administration of the contrast agent, the second period of time following the first period of time,
    ▪ the transformed target representation ($TR^T$) representing at least part of the examination region of the respective examination object in frequency space, if the target representation (TR) represents the examination region of the respective examination object in real space, or in real space, if the target representation (TR) represents the examination region of the respective examination object in frequency space,

  ◦ the training of the machine-learning model ($MLM^t$) comprising reducing differences between i) at least part of the synthetic representation (SR) and at least part of the target representation (TR) and ii) between at least part of a transformed synthetic representation ($SR^T$) and at least part of the transformed target representation ($TR^T$),

- receiving at least one input representation (R1*, R2*) of the examination region of a new examination object, the at least one input representation (R1*, R2*) of the examination region of the new examination object representing the examination region in a first period of time before and/or after administration of a contrast agent,
- inputting the at least one input representation (R1*, R2*) of the examination region of the new examination object into the trained machine-learning model ($MLM^t$),
- receiving a synthetic representation (SR*) of the examination region of the new examination object from the machine-learning model ($MLM^t$),
- outputting and/or storing the synthetic representation (SR*) of the examination region of the new examination object and/or transmitting the synthetic representation (SR*) of the examination region of the new examination object to a separate computer system.

**15.** Kit comprising a contrast agent and a computer program product comprising a computer program (40) that can be loaded into a working memory (22) of a computer system (1), where it causes the computer system (1) to execute the following steps:

- providing a trained machine-learning model ($MLM^t$),

  ◦ the trained machine-learning model ($MLM^t$) having been trained by means of training data (TD) to generate on the basis of at least one input representation (R1, R2) of an examination region of an examination object a synthetic representation (SR) of the examination region of the examination object,
  ◦ the training data (TD) comprising for each examination object of a multiplicity of examination objects i) at least one input representation (R1, R2) of the examination region of the examination object, ii) a target representation (TR) of the examination region of the examination object and iii) a transformed target representation ($TR^T$),

    ▪ the at least one input representation (R1, R2) representing the examination region of the respective examination object in a first period of time before or after administration of the contrast agent,
    ▪ the target representation (TR) representing the examination region of the respective examination object in a second period of time after the administration of the contrast agent, the second period of time following the first period of time,
    ▪ the transformed target representation ($TR^T$) representing at least part of the examination region of the respective examination object in frequency space, if the target representation (TR) represents the

examination region of the respective examination object in real space, or in real space, if the target representation (TR) represents the examination region of the respective examination object in frequency space,

◦ the training of the machine-learning model ($MLM^t$) comprising reducing differences between i) at least part of the synthetic representation (SR) and at least part of the target representation (TR) and ii) between at least part of a transformed synthetic representation ($SR^T$) and at least part of the transformed target representation ($TR^T$),

- receiving at least one input representation (R1*, R2*) of the examination region of a new examination object, the at least one input representation (R1*, R2*) of the examination region of the new examination object representing the examination region in a first period of time before and/or after administration of a contrast agent,
- inputting the at least one input representation (R1*, R2*) of the examination region of the new examination object into the trained machine-learning model ($MLM^t$),
- receiving a synthetic representation (SR*) of the examination region of the new examination object from the machine-learning model ($MLM^t$),
- outputting and/or storing the synthetic representation (SR*) of the examination region of the new examination object and/or transmitting the synthetic representation (SR*) of the examination region of the new examination object to a separate computer system.

**16.** Kit according to Claim 15, wherein the contrast agent is or comprises one or more contrast agents selected from the following list:

gadoxetate disodium,
gadolinium(III) 2-[4,7,10-tris(carboxymethyl)-1,4,7,10-tetrazacyclododec-1-yl]acetic acid,
gadolinium(III)
ethoxybenzyldiethylenetriaminepentaacetic acid,
gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoate,
dihydrogen [(±)-4-carboxy-5,8,11-tris(carboxymethyl)-1-phenyl-2-oxa-5,8,11-triazatridecan-13-oato(5-)]gadolinate(2-),
tetragadolinium [4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]amino}methyl)-4,7,11,14-tetraazaheptadecan-2-yl}-1,4,7,10-tetraazacyclododecan-1-yl]acetate, gadolinium 2,2',2"-(10-{1-carboxy-2-[2-(4-ethoxyphenyl)ethoxy]ethyl}-1,4,7,10-tetraazacyclododecane-1,4,7-triyl)triacetate,
gadolinium 2,2',2"-{10-[1-carboxy-2-{4-[2-(2-ethoxyethoxy)ethoxy]phenyl}ethyl]-1,4,7,10-tetraazacyclododecane-1,4,7-triyl}triacetate,
gadolinium 2,2',2"-{10-[(1R)-1-carboxy-2-{4-[2-(2-ethoxyethoxy)ethoxy]phenyl}ethyl]-1,4,7,10-tetraazacyclododecane-1,4,7-triyl}triacetate,
gadolinium (2S,2'S,2"S)-2,2',2"-{10-[(1S)-1-carboxy-4-{4-[2-(2-ethoxyethoxy)ethoxy]phenyl}butyl]-1,4,7,10-tetraazacyclododecane-1,4,7-triyl}tris(3-hydroxypropanoate),
gadolinium 2,2',2"-{10-[(1S)-4-(4-butoxyphenyl)-1-carboxybutyl]-1,4,7,10-tetraazacyclododecane-1,4,7-triyl}triacetate,
gadolinium-2,2',2"-{(2S)-10-(carboxymethyl)-2-[4-(2-ethoxyethoxy)benzyl]-1,4,7,10-tetraazacyclododecane-1,4,7-triyl}triacetate,
gadolinium-2,2',2"-[10-(carboxymethyl)-2-(4-ethoxybenzyl)-1,4,7,10-tetraazacyclododecane-1,4,7-triyl]triacetate,
gadolinium(III) 5,8-bis(carboxylatomethyl)-2-[2-(methylamino)-2-oxoethyl]-10-oxo-2,5,8,11-tetraazadodecane-1-carboxylate hydrate,
gadolinium(III) 2-[4-(2-hydroxypropyl)-7,10-bis(2-oxido-2-oxoethyl)-1,4,7,10-tetrazacyclododec-1-yl]acetate,
gadolinium(III) 2,2',2"-(10-((2R,3S)-1,3,4-trihydroxybutan-2-yl)-1,4,7,10-tetraazacyclododecane-1,4,7-triyl)triacetate,
a $Gd^{3+}$ complex of a compound of the formula (I)

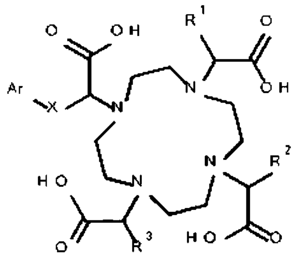

**(I)**,

where
Ar is a group selected from

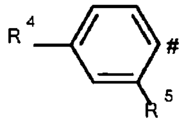
and
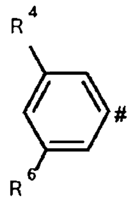
,

where # is the linkage to X,
X is a group selected from
$CH_2$, $(CH_2)_2$, $(CH_2)_3$, $(CH_2)_4$ and $*-(CH_2)_2-O-CH_2-^{\#}$,
where * is the linkage to Ar and # is the linkage to the acetic acid residue,
$R^1$, $R^2$ and $R^3$ are each independently a hydrogen atom or a group selected from $C_1$-$C_3$ alkyl, $-CH_2OH$, $-(CH_2)_2OH$ and - $CH_2OCH_3$,
$R^4$ is a group selected from $C_2$-$C_4$ alkoxy, $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,
$R^5$ is a hydrogen atom,
and
$R^6$ is a hydrogen atom,
or a stereoisomer, tautomer, hydrate, solvate or salt thereof, or a mixture thereof,
a $Gd^{3+}$ complex of a compound of the formula (II)

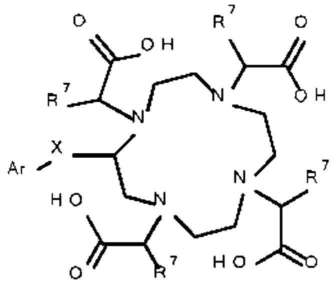

**(II)**,

where
Ar is a group selected from

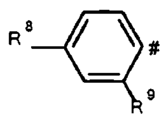 and 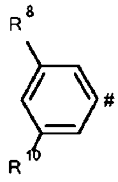 ,

where # is the linkage to X,
X is a group selected from $CH_2$, $(CH_2)_2$, $(CH_2)_3$, $(CH_2)_4$ and *- $(CH_2)_2$-O-$CH_2$-#, where * is the linkage to Ar and # is the linkage to the acetic acid residue,
$R^7$ is a hydrogen atom or a group selected from $C_1$-$C_3$ alkyl, -$CH_2$OH, - $(CH_2)_2$OH and -$CH_2OCH_3$;
$R^8$ is a group selected from $C_2$-$C_4$ alkoxy, ($H_3$C-$CH_2$O)-$(CH_2)_2$-O-, ($H_3$C-$CH_2$O)-$(CH_2)_2$-O-$(CH_2)_2$-O- and ($H_3$C-$CH_2$O)-$(CH_2)_2$-O-$(CH_2)_2$-O-$(CH_2)_2$-O-;
$R^9$ and $R^{10}$ are each independently a hydrogen atom;
or a stereoisomer, tautomer, hydrate, solvate or salt thereof, or a mixture thereof.

**Revendications**

1. Procédé mis en œuvre par ordinateur pour générer une image radiologique synthétique comprenant :

- fourniture d’un modèle entraîné ($MLM^t$) de l’apprentissage automatique,

  ◦ le modèle entraîné ($MLM^t$) de l’apprentissage automatique ayant été entraîné à l’aide de données d’entraînement (TD) afin de générer, sur la base d’au moins une représentation d’entrée (R1, R2) d’une zone d’examen d’un objet d’examen, une représentation synthétique (SR) de la zone d’examen de l’objet d’examen,
  ◦ les données d’entraînement (TD) comprenant, pour chaque objet d’examen d’une pluralité d’objets d’examen, i) au moins une représentation d’entrée (R1, R2) de la zone d’examen de l’objet d’examen, ii) une représentation cible (TR) de la zone d’examen de l’objet d’examen et iii) une représentation cible transformée ($TR^T$),

    ▪ l’au moins une représentation d’entrée (R1, R2) représentant la zone d’examen de l’objet d’examen respectif dans un premier intervalle de temps avant ou après l’administration d’un produit de contraste,
    ▪ la représentation cible (TR) représentant la zone d’examen de l’objet d’examen respectif dans un deuxième intervalle de temps après l’administration du produit de contraste, le deuxième intervalle de temps étant postérieur au premier intervalle de temps,
    ▪ la représentation cible transformée ($TR^T$) représentant au moins une partie de la zone d’examen de l’objet d’examen dans le domaine fréquentiel, dans le cas où la représentation cible (TR) représente la zone d’examen de l’objet d’examen respectif dans le domaine spatial, ou la représentant dans le domaine spatial, dans le cas où la représentation cible (TR) représente la zone d’examen de l’objet d’examen respectif dans le domaine fréquentiel,

  ◦ l’entraînement du modèle ($MLM^t$) de l’apprentissage automatique comprenant une réduction des écarts entre i) au moins une partie de la représentation synthétique (SR) et au moins une partie de la représentation cible (TR) et ii) entre au moins une partie d’une représentation synthétique transformée ($SR^T$) et au moins une partie de la représentation cible transformée ($TR^T$),

- réception d’au moins une représentation d’entrée (R1*, R2*) de la zone d’examen d’un nouvel objet d’examen, l’au moins une représentation d’entrée (R1*, R2*) de la zone d’examen du nouvel objet d’examen représentant la zone d’examen dans un premier intervalle de temps avant et/ou après une administration d’un produit de contraste,
- application en entrée de l’au moins une représentation d’entrée (R1*, R2*) de la zone d’examen du nouvel objet d’examen dans le modèle entraîné ($MLM^t$) de l’apprentissage automatique,
- réception d’une représentation synthétique (SR*) de la zone d’examen du nouvel objet d’examen en provenance du modèle ($MLM^t$) de l’apprentissage automatique,
- délivrance en sortie et/ou mémorisation de la représentation synthétique (SR*) de la zone d’examen du nouvel objet d’examen et/ou transmission de la représentation synthétique (SR*) de la zone d’examen du nouvel objet

d'examen à un système informatique séparé.

**2.** Procédé selon la revendication 1, l'entraînement du modèle ($MLM^t$) de l'apprentissage automatique comprenant :

- réception et/ou la fourniture de données d'entraînement (TD), les données d'entraînement (TD), pour chaque objet d'examen d'une pluralité d'objets d'examen, comprenant un ensemble de données d'entrée et de données cibles,

  ◦ chaque ensemble comprenant au moins une représentation d'entrée (R1, R2) de la région d'examen de l'objet d'examen en tant que données d'entrée et la représentation cible (TR) de la région d'examen de l'objet d'examen ainsi que la représentation cible transformée ($TR^T$) en tant que données cibles,
  ◦ l'au moins une représentation d'entrée (R1, R2) représentant la région d'examen dans le premier intervalle de temps avant et/ou après l'administration du produit de contraste et la représentation cible (TR) représentant la zone d'examen dans le deuxième intervalle de temps après l'administration du produit de contraste,
  ◦ la représentation cible transformée ($TR^T$) représentant au moins une partie de la zone d'examen de l'objet d'examen

    ▪ dans le domaine fréquentiel, dans le cas où la représentation cible (TR) représente la zone d'examen de l'objet d'examen dans le domaine spatial, ou
    ▪ dans le domaine spatial, dans le cas où la représentation cible (TR) représente la zone d'examen de l'objet d'examen dans le domaine fréquentiel,

- entraînement d'un modèle (MLM) de l'apprentissage automatique, le modèle (MLM) de l'apprentissage automatique étant configuré pour générer une représentation synthétique (SR) de la zone d'examen de l'objet d'examen sur la base d'au moins une représentation d'entrée (R1, R2) d'une zone d'examen d'un objet d'examen et de paramètres de modèle (MP),
l'entraînement comprenant, pour chaque objet d'examen de la pluralité d'objets d'examen :

  ◦ amenée de l'au moins une représentation d'entrée (R1, R2) au modèle de l'apprentissage automatique,
  ◦ réception, en provenance du modèle (MLM) de l'apprentissage automatique, d'une représentation synthétique (SR) de la zone d'examen de l'objet d'examen,
  ◦ génération et/ou réception d'une représentation synthétique transformée ($SR^T$) basée sur, et/ou en relation avec, la représentation synthétique (SR), la représentation synthétique transformée ($SR^T$) représentant au moins une partie de la zone d'examen de l'objet d'examen

    ▪ dans le domaine fréquentiel, dans le cas où la représentation synthétique (SR) représente la zone d'examen de l'objet d'examen dans le domaine spatial, ou
    ▪ dans le domaine spatial, dans le cas où la représentation synthétique (SR) représente la zone d'examen de l'objet d'examen dans le domaine fréquentiel,

  ◦ quantification des écarts i) entre au moins une partie de la représentation synthétique (SR) et au moins une partie de la représentation cible (TR) et ii) entre au moins une partie de la représentation synthétique transformée ($SR^T$) et au moins une partie de la représentation cible transformée ($TR^T$) au moyen d'une fonction d'erreur (L),
  ◦ réduction des écarts par modification des paramètres de modèle (MP),

- délivrance en sortie et/ou mémorisation du modèle entraîné ($MLM^t$) de l'apprentissage automatique et/ou des paramètres de modèle (MP) et/ou transmission du modèle entraîné ($MLM^t$) de l'apprentissage automatique et/ou des paramètres de modèle (MP) à un système informatique séparé.

**3.** Procédé selon la revendication 2,

la réception et/ou la fourniture de données d'entraînement (TD) comprenant :

  - génération d'une représentation cible transformée partielle ($TR^{T,P}$), la représentation cible transformée partielle ($TR^{T,P}$) étant réduite à une partie ou plusieurs parties de la représentation cible transformée ($TR^T$),

la génération et/ou la réception d'une représentation synthétique transformée ($SR^T$) basée sur, et/ou en relation avec, la représentation synthétique (SR) comprenant :

- génération d'une représentation synthétique transformée partielle ($SR^{T,P}$), la représentation synthétique transformée partielle ($SR^{T,P}$) étant réduite à une partie ou plusieurs parties de la représentation synthétique transformée ($SR^T$),

la quantification des écarts entre la représentation synthétique transformée ($SR^T$) et la représentation cible transformée ($TR^T$) comprenant :

- quantification des écarts entre la représentation synthétique transformée partielle ($SR^{T,P}$) et la représentation cible transformée partielle ($TR^{T,P}$).

**4.** Procédé selon l'une des revendications 1 à 3, l'entraînement comprenant, pour chaque objet d'examen de la pluralité d'objets d'examen :

◦ amenée de l'au moins une représentation d'entrée (R1, R2) au modèle (MLM) de l'apprentissage automatique,
◦ réception, en provenance du modèle (MLM) de l'apprentissage automatique, de la représentation synthétique (SR) et d'une première représentation synthétique transformée ($SR^T$) de la zone d'examen de l'objet d'examen, la première représentation synthétique transformée ($SR^T$) représentant au moins une partie de la zone d'examen de l'objet d'examen

▪ dans le domaine fréquentiel, dans le cas où la représentation synthétique (SR) représente la zone d'examen de l'objet d'examen dans le domaine spatial, ou
▪ dans le domaine spatial, dans le cas où la représentation synthétique (SR) représente la zone d'examen de l'objet d'examen dans le domaine fréquentiel,

◦ génération d'une deuxième représentation synthétique transformée ($SR^{T\#}$) sur la base de la représentation synthétique (SR) au moyen d'une transformation (T), la deuxième représentation synthétique transformée ($SR^{T\#}$) représentant au moins une partie de la zone d'examen de l'objet d'examen

▪ dans le domaine fréquentiel, dans le cas où la représentation synthétique (SR) représente la zone d'examen de l'objet d'examen dans le domaine spatial, ou
▪ dans le domaine spatial, dans le cas où la représentation synthétique (SR) représente la zone d'examen de l'objet d'examen dans le domaine fréquentiel,

- quantification des écarts i) entre au moins une partie de la représentation synthétique (SR) et au moins une partie de la représentation cible (TR), ii) entre au moins une partie de la première représentation synthétique transformée ($SR^T$) et au moins une partie de la représentation cible transformée ($TR^T$) et iii) entre au moins une partie de la première représentation synthétique transformée ($SR^T$) et au moins une partie de la deuxième représentation synthétique transformée ($SR^{T\#}$) au moyen d'une fonction d'erreur (L),
- réduction des écarts par modification des paramètres de modèle (MP),

**5.** Procédé selon l'une des revendications 1 à 4,

le modèle (MLM) de l'apprentissage automatique comprenant, lors de l'entraînement, un premier modèle (MLM1) de l'apprentissage automatique et un deuxième modèle (MLM2) de l'apprentissage automatique,
le premier modèle (MLM1) de l'apprentissage automatique étant configuré pour générer, sur la base d'au moins une représentation d'entrée (R1, R2) et de paramètres de modèle (MP1), une représentation synthétique (SR) de la zone d'examen de l'objet d'examen,
le deuxième modèle (MLM2) de l'apprentissage automatique étant configuré pour reconstruire, sur la base de la représentation synthétique (SR) de la zone d'examen de l'objet d'examen et de paramètres de modèle (MP2), au moins une représentation d'entrée (R1, R2), l'entraînement comprenant, pour chaque objet d'examen de la pluralité d'objets d'examen :

◦ génération d'une représentation d'entrée transformée (R2) sur la base d'au moins une représentation d'entrée (R1) au moyen d'une transformation (T), la représentation d'entrée transformée (R2) représentant au moins une partie de la zone d'examen de l'objet d'examen

- dans le domaine fréquentiel, dans le cas où la représentation d'entrée (R1) représente la zone d'examen de l'objet d'examen dans le domaine spatial, ou
- dans le domaine spatial, dans le cas où la représentation d'entrée (R1) représente la zone d'examen de l'objet d'examen dans le domaine fréquentiel,

◦ amenée de l'au moins une représentation d'entrée (R1) et/ou de la représentation d'entrée transformée (R2) au premier modèle (MLM1) de l'apprentissage automatique,
◦ réception, en provenance du premier modèle (MLM1) de l'apprentissage automatique, d'une représentation synthétique (SR) de la zone d'examen de l'objet d'examen,
◦ génération et/ou réception d'une représentation synthétique transformée ($SR^T$) basée sur, et/ou en relation avec, la représentation synthétique (SR), la représentation synthétique transformée ($SR^T$) représentant au moins une partie de la zone d'examen de l'objet d'examen

- dans le domaine fréquentiel, dans le cas où la représentation synthétique (SR) représente la zone d'examen de l'objet d'examen dans le domaine spatial, ou
- dans le domaine spatial, dans le cas où la représentation synthétique (SR) représente la zone d'examen de l'objet d'examen dans le domaine fréquentiel,

◦ amenée de la représentation synthétique (SR) et/ou de la représentation synthétique transformée ($SR^T$) au deuxième modèle (MLM2) de l'apprentissage automatique,
◦ réception d'une représentation d'entrée prédite (R1#) en provenance du deuxième modèle (MLM2) de l'apprentissage automatique,
◦ génération et/ou réception d'une représentation d'entrée prédite transformée (R2#) basée sur, et/ou en relation avec la représentation d'entrée prédite (R1#), la représentation d'entrée prédite transformée (R2#) représentant au moins une partie de la zone d'examen de l'objet d'examen

- dans le domaine fréquentiel, dans le cas où la représentation d'entrée prédite (R1#) représente la zone d'examen de l'objet d'examen dans le domaine spatial, ou
- dans le domaine spatial, dans le cas où la représentation d'entrée prédite (R1#) représente la zone d'examen de l'objet d'examen dans le domaine fréquentiel,

◦ quantification des écarts i) entre au moins une partie de la représentation synthétique (SR) et au moins une partie de la représentation cible (TR), ii) entre au moins une partie de la représentation synthétique transformée ($SR^T$) et au moins une partie de la représentation cible transformée ($TR^T$), iii) entre au moins une partie de la représentation d'entrée (R1) et au moins une partie de la représentation d'entrée prédite (R1#) et iv) entre au moins une partie de la représentation d'entrée transformée (R2) et au moins une partie de la représentation d'entrée prédite transformée (R2#) au moyen d'une fonction d'erreur (L),
◦ réduction des écarts par modification des paramètres de modèle (MP).

**6.** Procédé selon l'une des revendications 1 à 5, l'objet d'examen étant un mammifère, de préférence un être humain.

**7.** Procédé selon l'une des revendications 1 à 6, la zone d'examen étant ou comprenant un foie, un cerveau, un cœur, un rein, un poumon, un estomac, un intestin, un pancréas, une glande thyroïde, une prostate ou un sein d'un être humain.

**8.** Procédé selon l'une des revendications 1 à 7, la zone d'examen étant un foie ou une partie d'un foie d'un être humain.

**9.** Procédé selon l'une des revendications 1 à 8, chacune des au moins une représentation d'entrée (R1, R2, R1*, R2*) étant une représentation de la zone d'examen dans le domaine spatial, la représentation cible (TR) étant une représentation de la zone d'examen dans le domaine spatial et la représentation synthétique (SR, SR*) étant une représentation de la zone d'examen dans le domaine spatial.

**10.** Procédé selon l'une des revendications 3 à 9, la représentation synthétique transformée partielle ($SR^{T,P}$) représentant la zone d'examen dans le domaine fréquentiel, la représentation synthétique transformée partielle ($SR^{T,P}$) étant réduite à une plage de fréquences de la représentation synthétique transformée ($SR^T$), des informations de contraste étant codées dans la plage de fréquences.

**11.** Procédé selon l'une des revendications 3 à 9, la représentation synthétique transformée partielle ($SR^{T,P}$) représentant la zone d'examen dans le domaine fréquentiel, la représentation synthétique transformée partielle ($SR^{T,P}$)

étant réduite à une plage de fréquences de la représentation synthétique transformée ($SR^T$), des informations sur les structures fines étant codées dans la plage de fréquences.

**12.** Système informatique (10) destiné à générer une image radiologique synthétique, comprenant

• une unité de réception (11),
• une unité de commande et de calcul (12), et
• une unité de sortie (13),

- l'unité de commande et de calcul (12) étant configurée pour fournir un modèle entraîné ($MLM^t$) de l'apprentissage automatique,

◦ le modèle entraîné ($MLM^t$) de l'apprentissage automatique ayant été entraîné à l'aide de données d'entraînement (TD) afin de générer, sur la base d'au moins une représentation d'entrée (R1, R2) d'une zone d'examen d'un objet d'examen, une représentation synthétique (SR) de la zone d'examen de l'objet d'examen,
◦ les données d'entraînement (TD) comprenant, pour chaque objet d'examen d'une pluralité d'objets d'examen, i) au moins une représentation d'entrée (R1, R2) de la zone d'examen de l'objet d'examen, ii) une représentation cible (TR) de la zone d'examen de l'objet d'examen et iii) une représentation cible transformée ($TR^T$),

▪ l'au moins une représentation d'entrée (R1, R2) représentant la zone d'examen de l'objet d'examen respectif dans un premier intervalle de temps avant ou après l'administration d'un produit de contraste,
▪ la représentation cible (TR) représentant la zone d'examen de l'objet d'examen respectif dans un deuxième intervalle de temps après l'administration du produit de contraste, le deuxième intervalle de temps étant postérieur au premier intervalle de temps,
▪ la représentation cible transformée ($TR^T$) représentant au moins une partie de la zone d'examen de l'objet d'examen dans le domaine fréquentiel, dans le cas où la représentation cible (TR) représente la zone d'examen de l'objet d'examen respectif dans le domaine spatial, ou la représentant dans le domaine spatial, dans le cas où la représentation cible (TR) représente la zone d'examen de l'objet d'examen respectif dans le domaine fréquentiel,

◦ l'entraînement du modèle ($MLM^t$) de l'apprentissage automatique comprenant une réduction des écarts entre i) au moins une partie de la représentation synthétique (SR) et au moins une partie de la représentation cible (TR) et ii) entre au moins une partie d'une représentation synthétique transformée ($SR^T$) et au moins une partie de la représentation cible transformée ($TR^T$),

- l'unité de commande et de calcul (12) étant configurée pour amener l'unité de réception (11) à recevoir au moins une représentation d'entrée (R1*, R2*) d'une zone d'examen d'un nouvel objet d'examen, l'au moins une représentation d'entrée (R1*, R2*) de la zone d'examen du nouvel objet d'examen représentant la zone d'examen pendant un premier intervalle de temps avant et/ou après l'administration d'un produit de contraste,
- l'unité de commande et de calcul (12) étant configurée pour fournir en entrée l'au moins une représentation d'entrée (R1*, R2*) de la zone d'examen du nouvel objet d'examen à un modèle entraîné ($MLM^t$) de l'apprentissage automatique,
- l'unité de commande et de calcul (12) étant configurée pour recevoir une représentation synthétique (SR*) de la zone d'examen du nouvel objet d'examen en provenance du modèle ($MLM^t$) de l'apprentissage automatique,
- l'unité de commande et de calcul (12) étant configurée pour amener l'unité de sortie (13) à délivrer en sortie et/ou à mémoriser et/ou à transmettre à un système informatique séparé la représentation synthétique (SR*) de la zone d'examen du nouvel objet d'examen.

**13.** Produit de programme informatique destiné à générer une image radiologique synthétique, comprenant un programme informatique (40) qui peut être chargé dans une mémoire de travail (22) d'un système informatique (1) et qui y amène le système informatique (1) à exécuter les étapes suivantes :

- fourniture d'un modèle entraîné ($MLM^t$) de l'apprentissage automatique,

◦ le modèle entraîné ($MLM^t$) de l'apprentissage automatique ayant été entraîné à l'aide de données d'entraînement (TD) afin de générer, sur la base d'au moins une représentation d'entrée (R1, R2) d'une zone d'examen d'un objet d'examen, une représentation synthétique (SR) de la zone d'examen de l'objet d'examen,
◦ les données d'entraînement (TD) comprenant, pour chaque objet d'examen d'une pluralité d'objets d'examen, i) au moins une représentation d'entrée (R1, R2) de la zone d'examen de l'objet d'examen, ii) une représentation cible (TR) de la zone d'examen de l'objet d'examen et iii) une représentation cible transformée ($TR^T$),

▪ l'au moins une représentation d'entrée (R1, R2) représentant la zone d'examen de l'objet d'examen respectif dans un premier intervalle de temps avant ou après l'administration d'un produit de contraste,
▪ la représentation cible (TR) représentant la zone d'examen de l'objet d'examen respectif dans un deuxième intervalle de temps après l'administration du produit de contraste, le deuxième intervalle de temps étant postérieur au premier intervalle de temps,
▪ la représentation cible transformée ($TR^T$) représentant au moins une partie de la zone d'examen de l'objet d'examen dans le domaine fréquentiel, dans le cas où la représentation cible (TR) représente la zone d'examen de l'objet d'examen respectif dans le domaine spatial, ou la représentant dans le domaine spatial, dans le cas où la représentation cible (TR) représente la zone d'examen de l'objet d'examen respectif dans le domaine fréquentiel,

◦ l'entraînement du modèle ($MLM^t$) de l'apprentissage automatique comprenant une réduction des écarts entre i) au moins une partie de la représentation synthétique (SR) et au moins une partie de la représentation cible (TR) et ii) entre au moins une partie d'une représentation synthétique transformée ($SR^T$) et au moins une partie de la représentation cible transformée ($TR^T$),

- réception d'au moins une représentation d'entrée (R1*, R2*) de la zone d'examen d'un nouvel objet d'examen, l'au moins une représentation d'entrée (R1*, R2*) de la zone d'examen du nouvel objet d'examen représentant la zone d'examen dans un premier intervalle de temps avant et/ou après une administration d'un produit de contraste,
- application en entrée de l'au moins une représentation d'entrée (R1*, R2*) de la zone d'examen du nouvel objet d'examen dans le modèle entraîné ($MLM^t$) de l'apprentissage automatique,
- réception d'une représentation synthétique (SR*) de la zone d'examen du nouvel objet d'examen en provenance du modèle ($MLM^t$) de l'apprentissage automatique,
- délivrance en sortie et/ou mémorisation de la représentation synthétique (SR*) de la zone d'examen du nouvel objet d'examen et/ou transmission de la représentation synthétique (SR*) de la zone d'examen du nouvel objet d'examen à un système informatique séparé.

**14.** Utilisation d'un produit de contraste dans un procédé d'examen radiologique, le procédé d'examen radiologique comprenant les étapes suivantes :

- fourniture d'un modèle entraîné ($MLM^t$) de l'apprentissage automatique,

◦ le modèle entraîné ($MLM^t$) de l'apprentissage automatique ayant été entraîné à l'aide de données d'entraînement (TD) afin de générer, sur la base d'au moins une représentation d'entrée (R1, R2) d'une zone d'examen d'un objet d'examen, une représentation synthétique (SR) de la zone d'examen de l'objet d'examen,
◦ les données d'entraînement (TD) comprenant, pour chaque objet d'examen d'une pluralité d'objets d'examen, i) au moins une représentation d'entrée (R1, R2) de la zone d'examen de l'objet d'examen, ii) une représentation cible (TR) de la zone d'examen de l'objet d'examen et iii) une représentation cible transformée ($TR^T$),

▪ l'au moins une représentation d'entrée (R1, R2) représentant la zone d'examen de l'objet d'examen respectif dans un premier intervalle de temps avant ou après l'administration d'un produit de contraste,
▪ la représentation cible (TR) représentant la zone d'examen de l'objet d'examen respectif dans un deuxième intervalle de temps après l'administration du produit de contraste, le deuxième intervalle de temps étant postérieur au premier intervalle de temps,
▪ la représentation cible transformée ($TR^T$) représentant au moins une partie de la zone d'examen de l'objet d'examen dans le domaine fréquentiel, dans le cas où la représentation cible (TR) représente la

zone d'examen de l'objet d'examen respectif dans le domaine spatial, ou la représentant dans le domaine spatial, dans le cas où la représentation cible (TR) représente la zone d'examen de l'objet d'examen respectif dans le domaine fréquentiel,

◦ l'entraînement du modèle ($MLM^t$) de l'apprentissage automatique comprenant une réduction des écarts entre i) au moins une partie de la représentation synthétique (SR) et au moins une partie de la représentation cible (TR) et ii) entre au moins une partie d'une représentation synthétique transformée ($SR^T$) et au moins une partie de la représentation cible transformée ($TR^T$),

- réception d'au moins une représentation d'entrée (R1*, R2*) de la zone d'examen d'un nouvel objet d'examen, l'au moins une représentation d'entrée (R1*, R2*) de la zone d'examen du nouvel objet d'examen représentant la zone d'examen dans un premier intervalle de temps avant et/ou après une administration d'un produit de contraste,
- application en entrée de l'au moins une représentation d'entrée (R1*, R2*) de la zone d'examen du nouvel objet d'examen dans le modèle entraîné ($MLM^t$) de l'apprentissage automatique,
- réception d'une représentation synthétique (SR*) de la zone d'examen du nouvel objet d'examen en provenance du modèle ($MLM^t$) de l'apprentissage automatique,
- délivrance en sortie et/ou mémorisation de la représentation synthétique (SR*) de la zone d'examen du nouvel objet d'examen et/ou transmission de la représentation synthétique (SR*) de la zone d'examen du nouvel objet d'examen à un système informatique séparé.

**15.** Kit comprenant un produit de contraste et un produit de programme informatique comprenant un programme informatique (40) qui peut être chargé dans une mémoire de travail (22) d'un système informatique (1) et qui y amène le système informatique (1) à exécuter les étapes suivantes :

- fourniture d'un modèle entraîné ($MLM^t$) de l'apprentissage automatique,

◦ le modèle entraîné ($MLM^t$) de l'apprentissage automatique ayant été entraîné à l'aide de données d'entraînement (TD) afin de générer, sur la base d'au moins une représentation d'entrée (R1, R2) d'une zone d'examen d'un objet d'examen, une représentation synthétique (SR) de la zone d'examen de l'objet d'examen,
◦ les données d'entraînement (TD) comprenant, pour chaque objet d'examen d'une pluralité d'objets d'examen, i) au moins une représentation d'entrée (R1, R2) de la zone d'examen de l'objet d'examen, ii) une représentation cible (TR) de la zone d'examen de l'objet d'examen et iii) une représentation cible transformée ($TR^T$),

▪ l'au moins une représentation d'entrée (R1, R2) représentant la zone d'examen de l'objet d'examen respectif dans un premier intervalle de temps avant ou après l'administration d'un produit de contraste,
▪ la représentation cible (TR) représentant la zone d'examen de l'objet d'examen respectif dans un deuxième intervalle de temps après l'administration du produit de contraste, le deuxième intervalle de temps étant postérieur au premier intervalle de temps,
▪ la représentation cible transformée ($TR^T$) représentant au moins une partie de la zone d'examen de l'objet d'examen dans le domaine fréquentiel, dans le cas où la représentation cible (TR) représente la zone d'examen de l'objet d'examen respectif dans le domaine spatial, ou la représentant dans le domaine spatial, dans le cas où la représentation cible (TR) représente la zone d'examen de l'objet d'examen respectif dans le domaine fréquentiel,

◦ l'entraînement du modèle ($MLM^t$) de l'apprentissage automatique comprenant une réduction des écarts entre i) au moins une partie de la représentation synthétique (SR) et au moins une partie de la représentation cible (TR) et ii) entre au moins une partie d'une représentation synthétique transformée ($SR^T$) et au moins une partie de la représentation cible transformée ($TR^T$),

- réception d'au moins une représentation d'entrée (R1*, R2*) de la zone d'examen d'un nouvel objet d'examen, l'au moins une représentation d'entrée (R1*, R2*) de la zone d'examen du nouvel objet d'examen représentant la zone d'examen dans un premier intervalle de temps avant et/ou après une administration d'un produit de contraste,
- application en entrée de l'au moins une représentation d'entrée (R1*, R2*) de la zone d'examen du nouvel objet d'examen dans le modèle entraîné ($MLM^t$) de l'apprentissage automatique,

- réception d'une représentation synthétique (SR*) de la zone d'examen du nouvel objet d'examen en provenance du modèle ($MLM^t$) de l'apprentissage automatique,
- délivrance en sortie et/ou mémorisation de la représentation synthétique (SR*) de la zone d'examen du nouvel objet d'examen et/ou transmission de la représentation synthétique (SR*) de la zone d'examen du nouvel objet d'examen à un système informatique séparé.

**16.** Kit selon la revendication 15, le produit de contraste étant ou comprenant un ou plusieurs produits de contraste choisis dans la liste suivante :

gadoxétate disodique,
acide gadolinium(III) 2-[4,7,10-tris(carboxyméthyl)-1,4,7,10-tétrazacyclododéc-1-yl]acétique,
acide gadolinium(III) éthoxybenzyl-diéthylènetriaminepentaacétique,
2-[3,9-bis[1-carboxylato-4-(2,3-dihydroxypropylamino)-4-oxobutyl]-3,6,9,15-tétrazabicyclo[9.3.1]pentadéca-1(15),11,13-trién-6-yl]-5-(2,3-dihydroxypropylamino)-5-oxopentanoate de gadolinium(III),
dihydrogène[(±)-4-carboxy-5,8,11-tris(carboxyméthyl)-1-phényl-2-oxa-5,8,11-triazatridécan-13-oato(5-)]gadolinate(2-),
acétate de tétragadolinium-[4,10-bis(carboxylatométhyl)-7-{3,6,12,15-tétraoxo-16-[4,7,10-tris-(carboxylatométhyl)-1,4,7,10-tétraazacyclododécan-1-yl]-9,9-bis({ [({2-[4,7,10-tris-(carboxylatométhyl)-1,4,7,10-tétraazacyclododécan-1-yl]propanoyl}amino)acétyl]-amino}méthyl)-4,7,11,14-tétraazahepta-décan-2-yl}-1,4,7,10-tétraazacyclododécan-1-yle], 2,2',2''-(10-{1-carboxy-2-[2-(4-éthoxyphényl)éthoxy]éthyl}-1,4,7,10-tétraazacyclododécan-1,4,7-triyl)triacétate de gadolinium,
2,2',2"-[10-[1-carboxy-2-{4-[2-(2-éthoxyéthoxy)éthoxy]phényl}éthyl]-1,4,7,10-tétraazacyclododécan-1,4,7-triyl}triacétate de gadolinium,
2,2',2"-[10-[(1R)-1-carboxy-2-{4-[2-(2-éthoxyéthoxy)éthoxy]phényl}éthyl]-1,4,7,10-tétraazacyclododécan-1,4,7-triyl}triacétate de gadolinium,
(2S,2'S,2"S)-2,2',2"-{10-[(1S)-1-carboxy-4-{4-[2-(2-éthoxyéthoxy)éthoxy]phényl}butyl]-1,4,7,10-tétraazacyclododécan-1,4,7-triyl}tris(3-hydroxypropanoate) de gadolinium,
2,2',2"-{10-[(1S)-4-(4-butoxyphenyl)-1-carboxybutyl]-1,4,7,10-tétraazacyclododécan-1,4,7-triyl}triacétate de gadolinium,
triacétate de gadolinium-2,2',2"-{(2S)-10-(carboxyméthyl)-2-[4-(2-éthoxyéthoxy)benzyl]-1,4,7,10-tétraazacyclododécane-1,4,7-triyle},
triacétate de gadolinium-2,2',2"-[10-(carboxyméthyl)-2-(4-éthoxybenzyl)-1,4,7,10-tétraazacyclododécane-1,4,7-triyle],
5,8-bis(carboxylatométhyl)-2-[2-(méthylamino)-2-oxoéthyl]-10-oxo-2,5,8,11-tétraazadodécan-1-carboxylate-hydrate de gadolinium (III),
2-[4-(2-hydroxypropyl)-7,10-bis(2-oxido-2-oxoéthyl)-1,4,7,10-tétrazacyclododéc-1-yl]acétate de gadolinium (III),
2,2',2"-(10-((2R,3S)-1,3,4-trihydroxybutan-2-yl)-1,4,7,10-tétraazacyclododécan-1,4,7-triyl)triacétate de gadolinium (III),
un complexe de $Gd^{3+}$ d'un composé de formule (I),

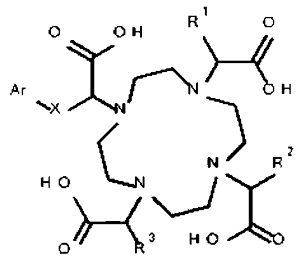

**(I)** ,

Ar représentant un groupe choisi parmi

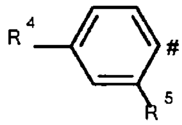 et 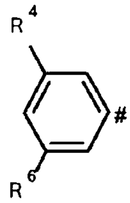 ,

# représentant la liaison à X,

X représentant un groupe qui est choisi parmi $CH_2$, $(CH_2)_2$, $(CH_2)_3$, $(CH_2)_4$ et $*-(CH_2)_2-O-CH_2-^{\#}$,

* représentant la liaison à Ar et # représentant la liaison au radical d'acide acétique,

$R^1$, $R^2$ et $R^3$ représentant, indépendamment les uns des autres, un atome d'hydrogène ou un groupe choisi parmi un alkyle en $C_1$-$C_3$, $-CH_2OH$, $-(CH_2)_2OH$ et $-CH_2OCH_3$,

$R^4$ représentant un groupe choisi parmi un alcoxy en $C_2$-$C_4$, $(H_3C-CH_2)-O-(CH_2)_2-O-$, $(H_3C-CH_2)-O-(CH_2)_2-O-(CH_2)_2-O-$ et $(H_3C-CH_2)-O-(CH_2)_2-O-(CH_2)_2-O-(CH_2)_2-O-$,

$R^5$ représentant un atome d'hydrogène,

et

$R^6$ représentant un atome d'hydrogène,

ou un stéréoisomère, un tautomère, un hydrate, un solvate ou un sel de celui-ci, ou un mélange de ceux-ci,

un complexe de $Gd^{3+}$ d'un composé de formule (II),

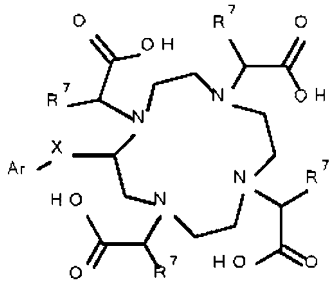

**(II)**,

Ar représentant un groupe choisi parmi

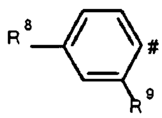 et 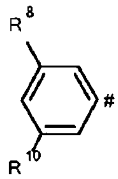 ,

# représentant la liaison à X,

X représentant un groupe qui est choisi parmi $CH_2$, $(CH_2)_2$, $(CH_2)_3$, $(CH_2)_4$ et $*-(CH_2)_2-O-CH_2-^{\#}$, * représentant la liaison à Ar et # représentant la liaison au radical d'acide acétique,

$R^7$ représentant un atome d'hydrogène ou un groupe choisi parmi un alkyle en $C_1$-$C_3$, $-CH_2OH$, $-(CH_2)_2OH$ et $-CH_2OCH_3$ ;

$R^8$ représentant un groupe choisi parmi un alcoxy en $C_2$-$C_4$, $(H_3C-CH_2O)-(CH_2)_2-O-$, $(H_{3C}-CH_2O)-(CH_2)_2-O-(CH_2)_2-O-$ et $(H_3C-CH_2O)-(CH_2)_2-O-(CH_2)_2-O-(CH_2)_2-O-$ ;

$R^9$ et $R^{10}$ représentant, indépendamment l'un de l'autre, un atome d'hydrogène ;

ou un stéréoisomère, un tautomère, un hydrate, un solvate ou un sel de celui-ci, ou un mélange de ceux-ci.

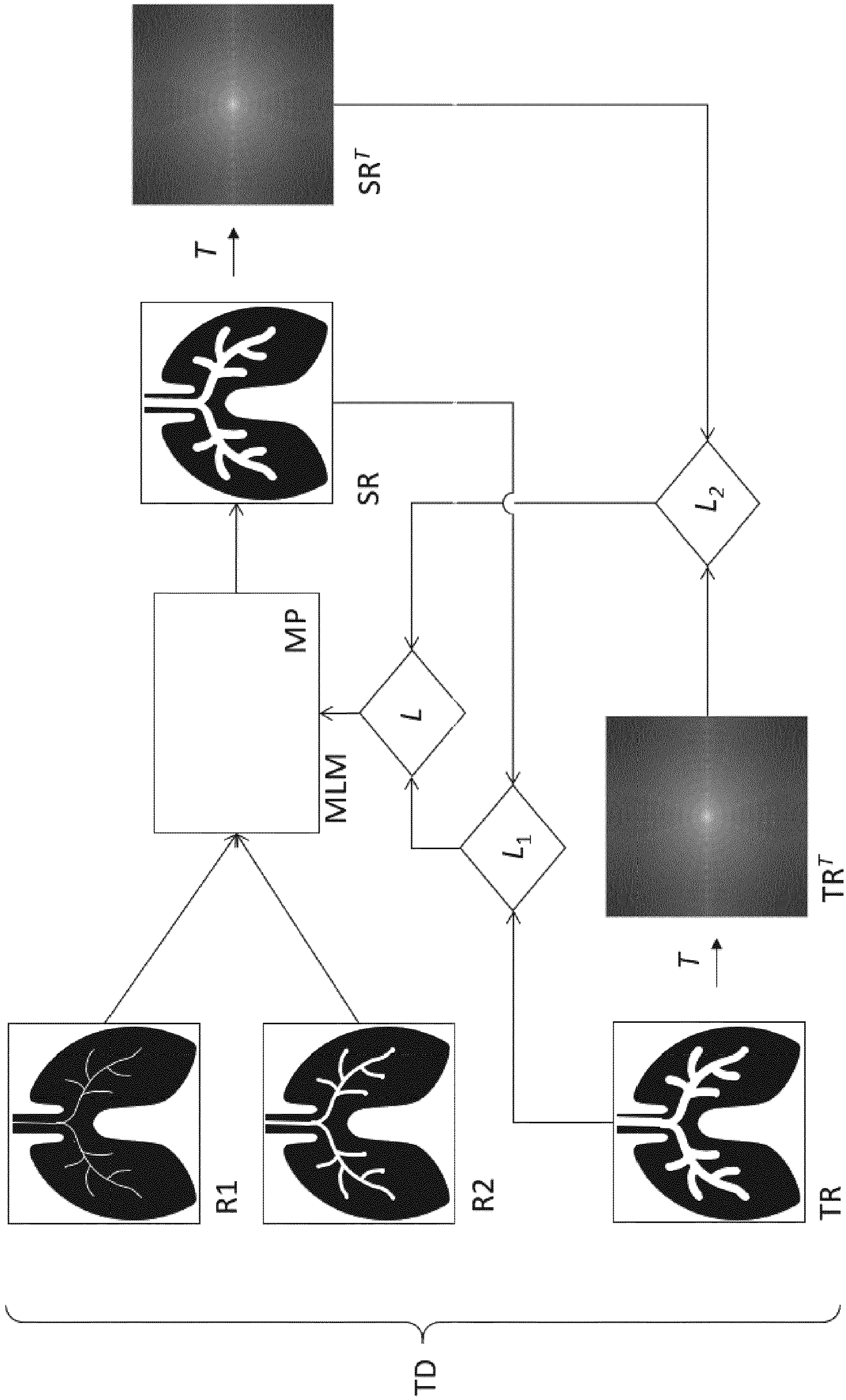


Fig. 1

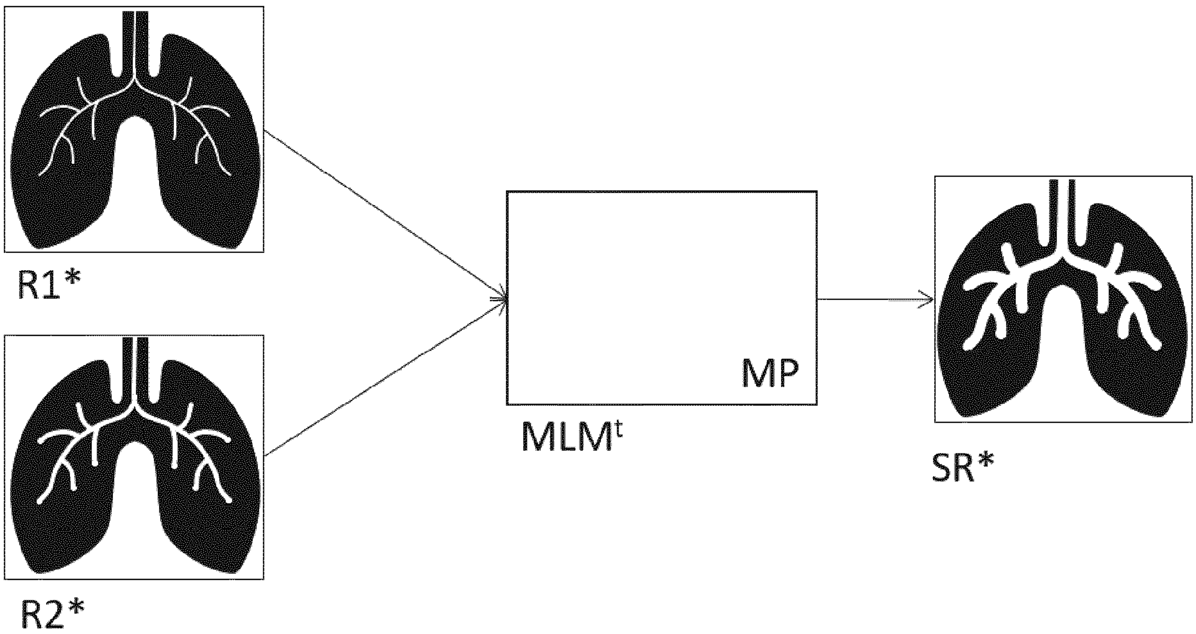


Fig. 2

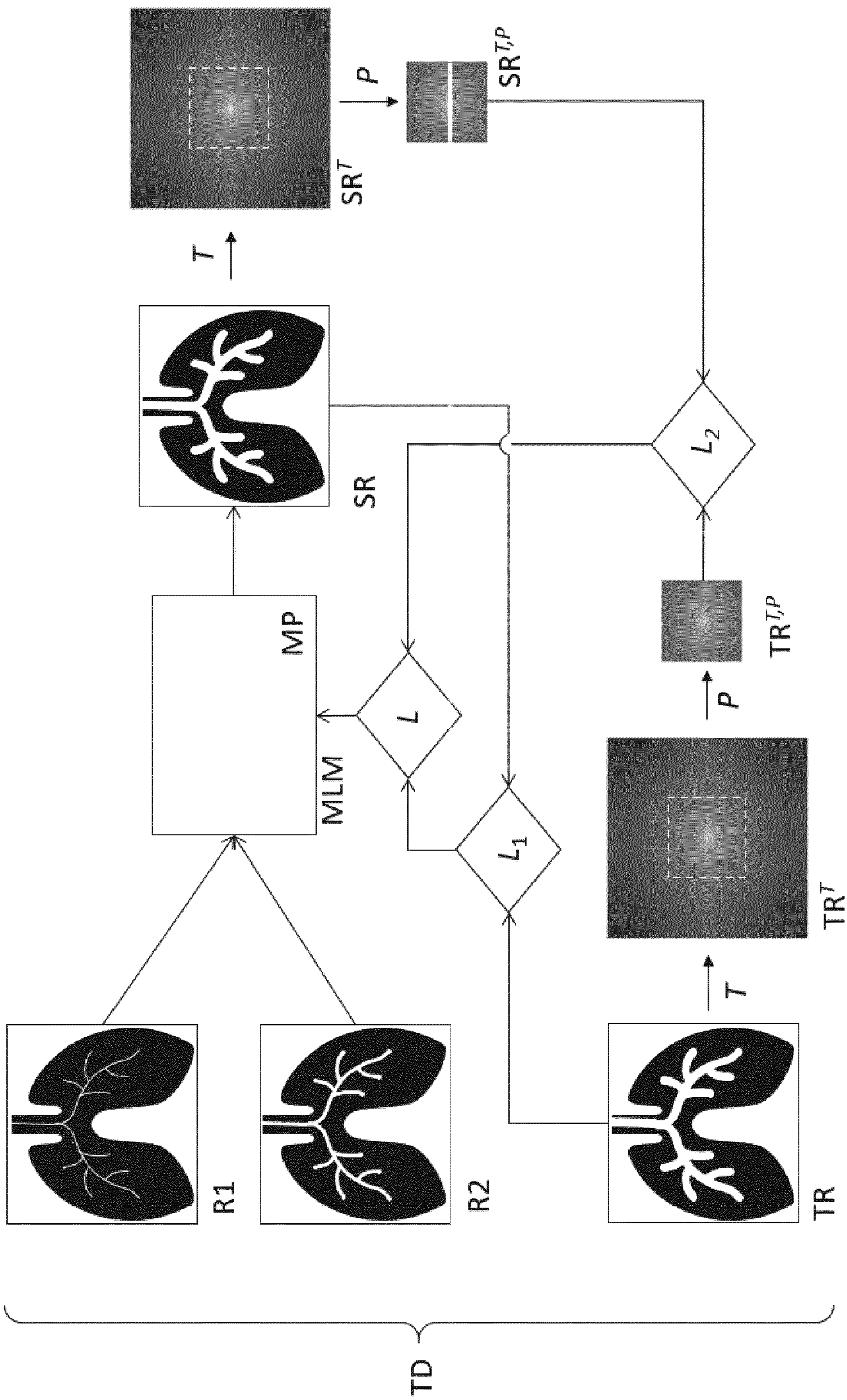


Fig. 3

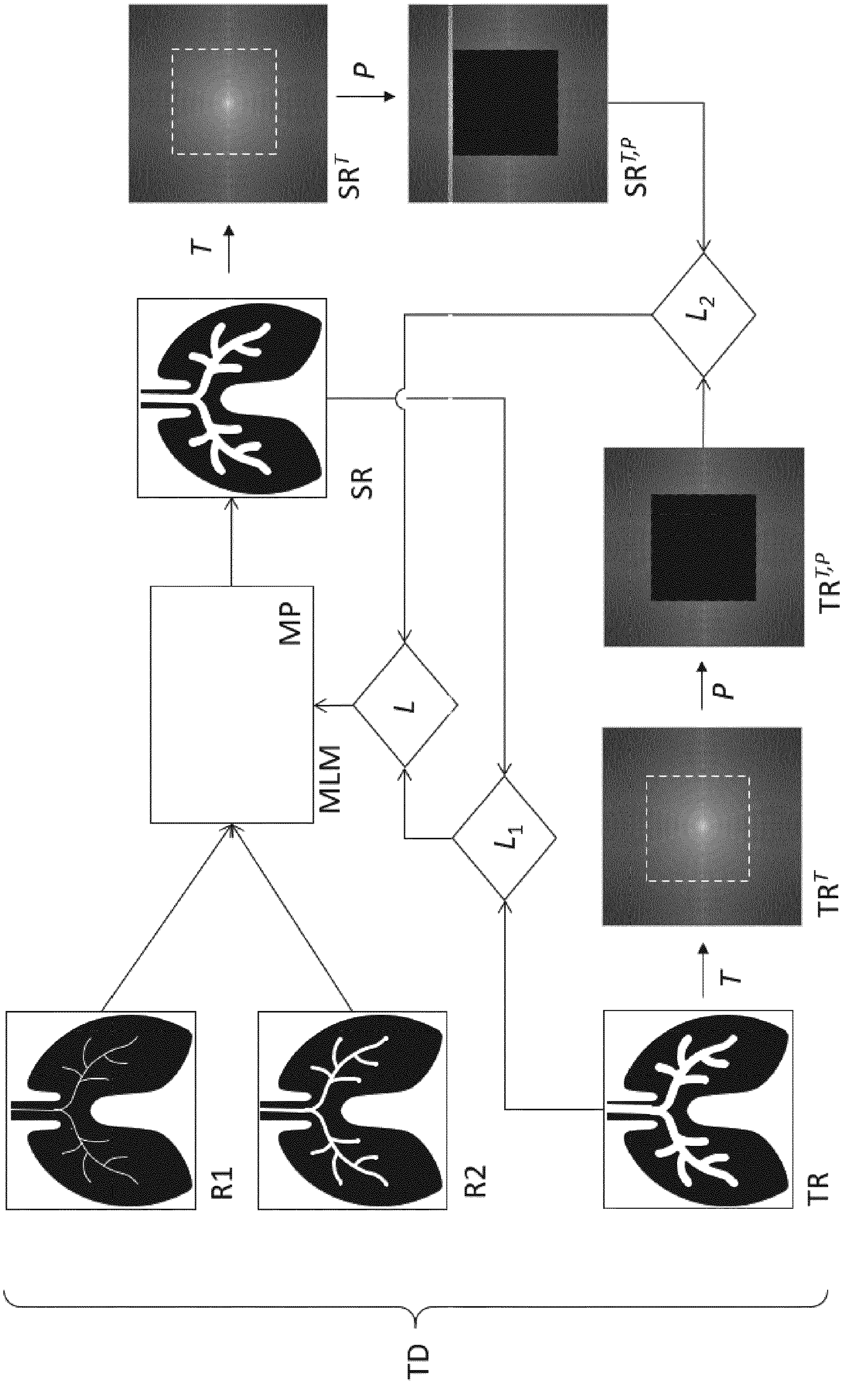


Fig. 4

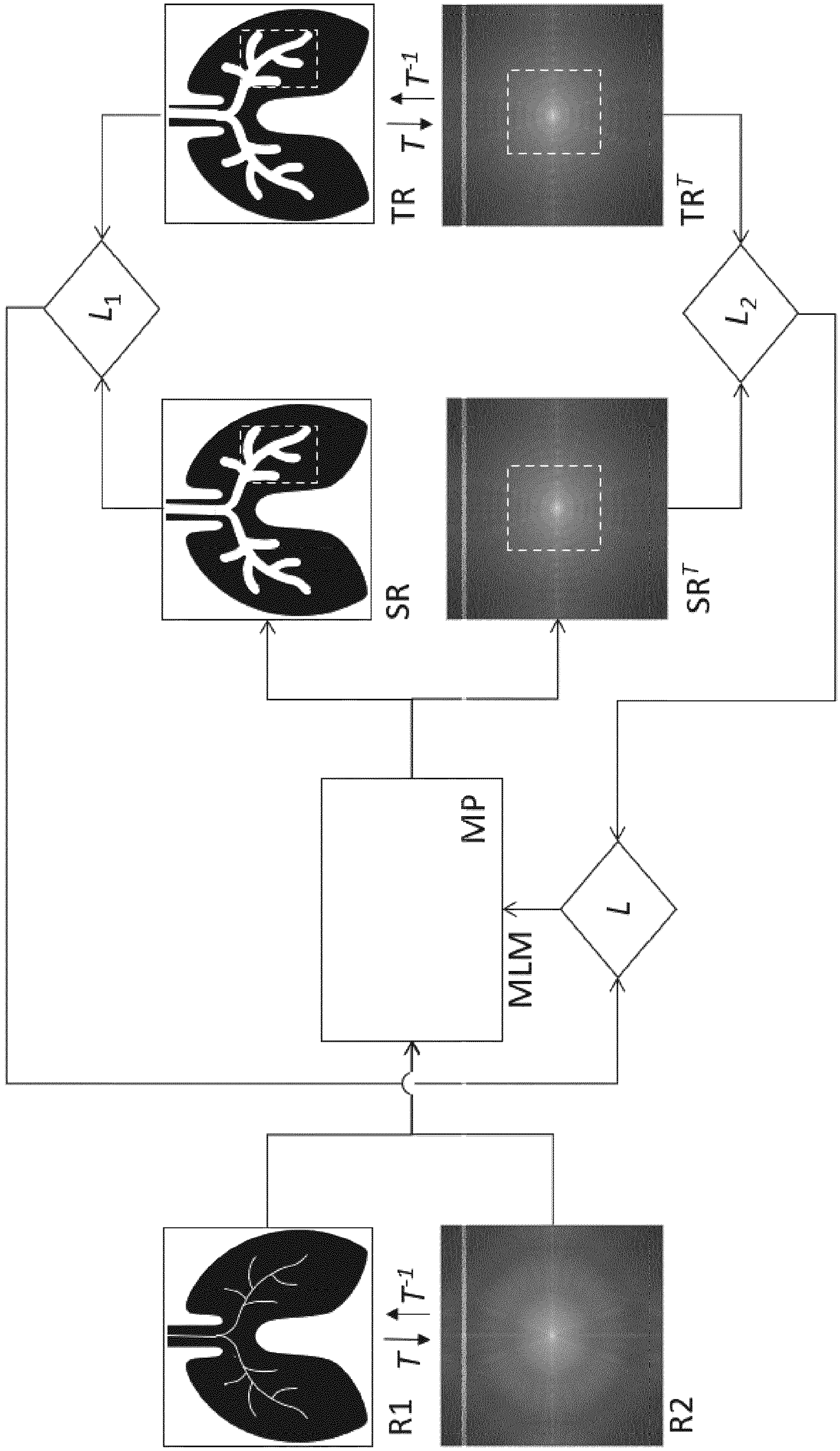


Fig. 5

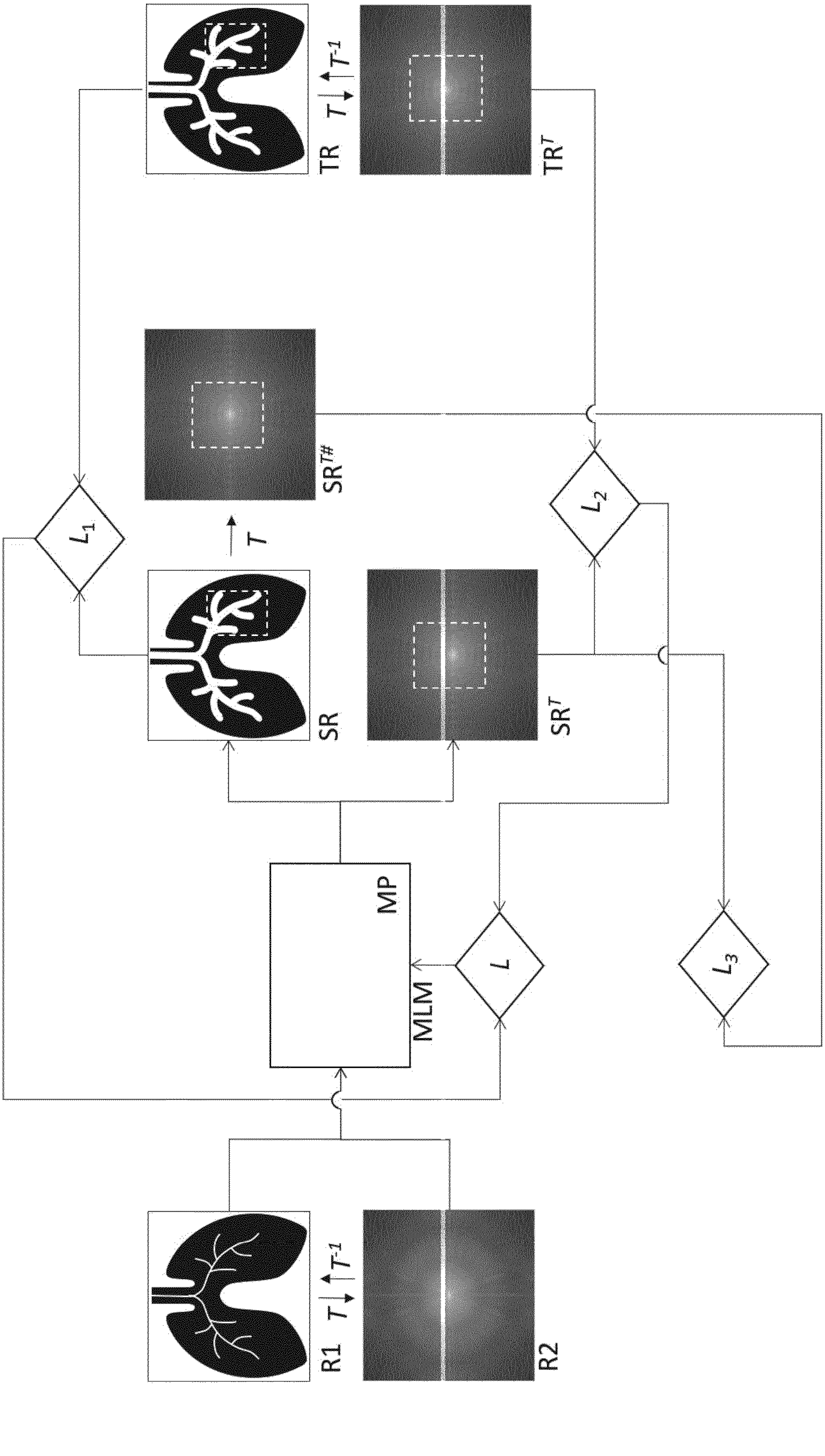


Fig. 6

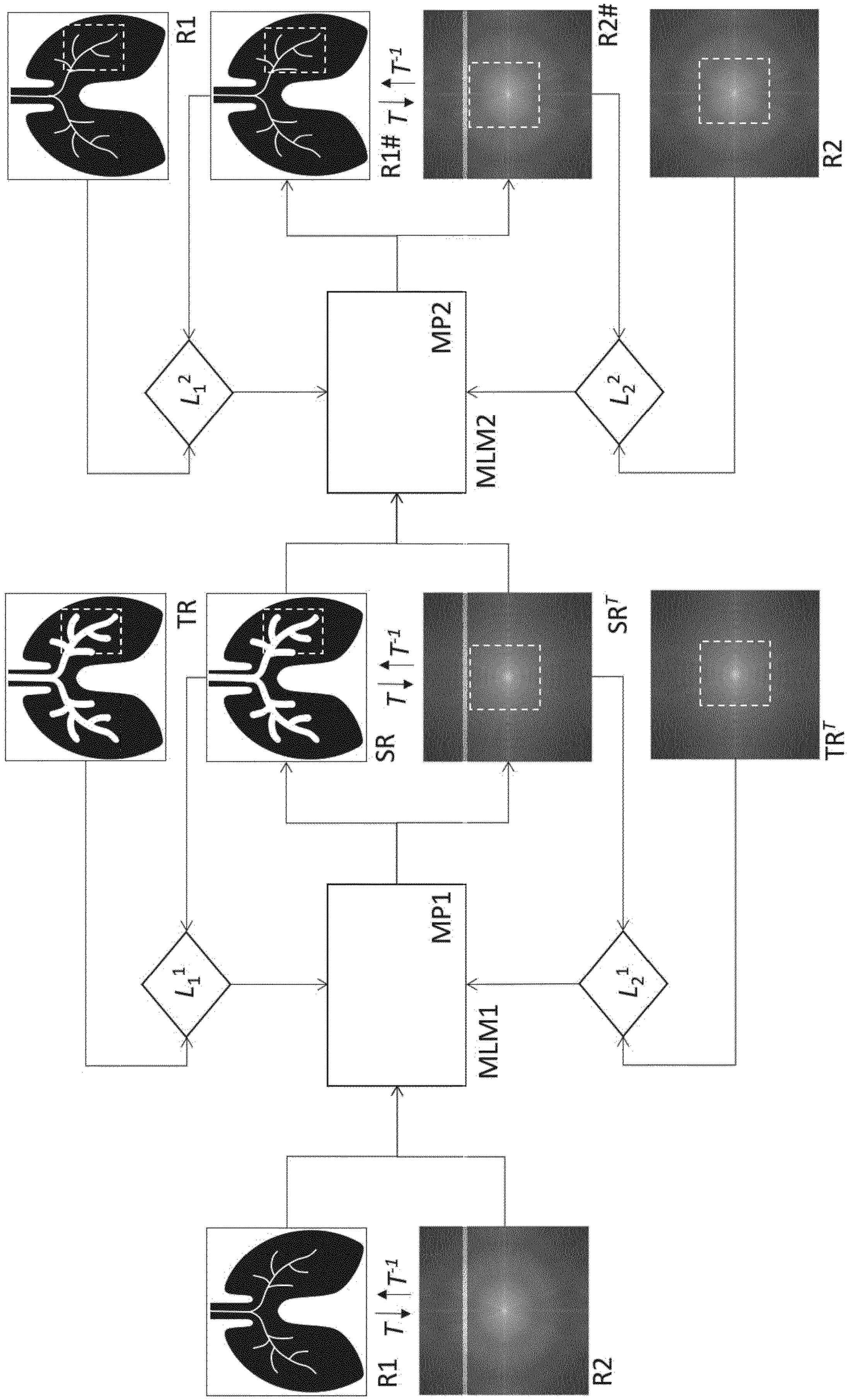


Fig. 7

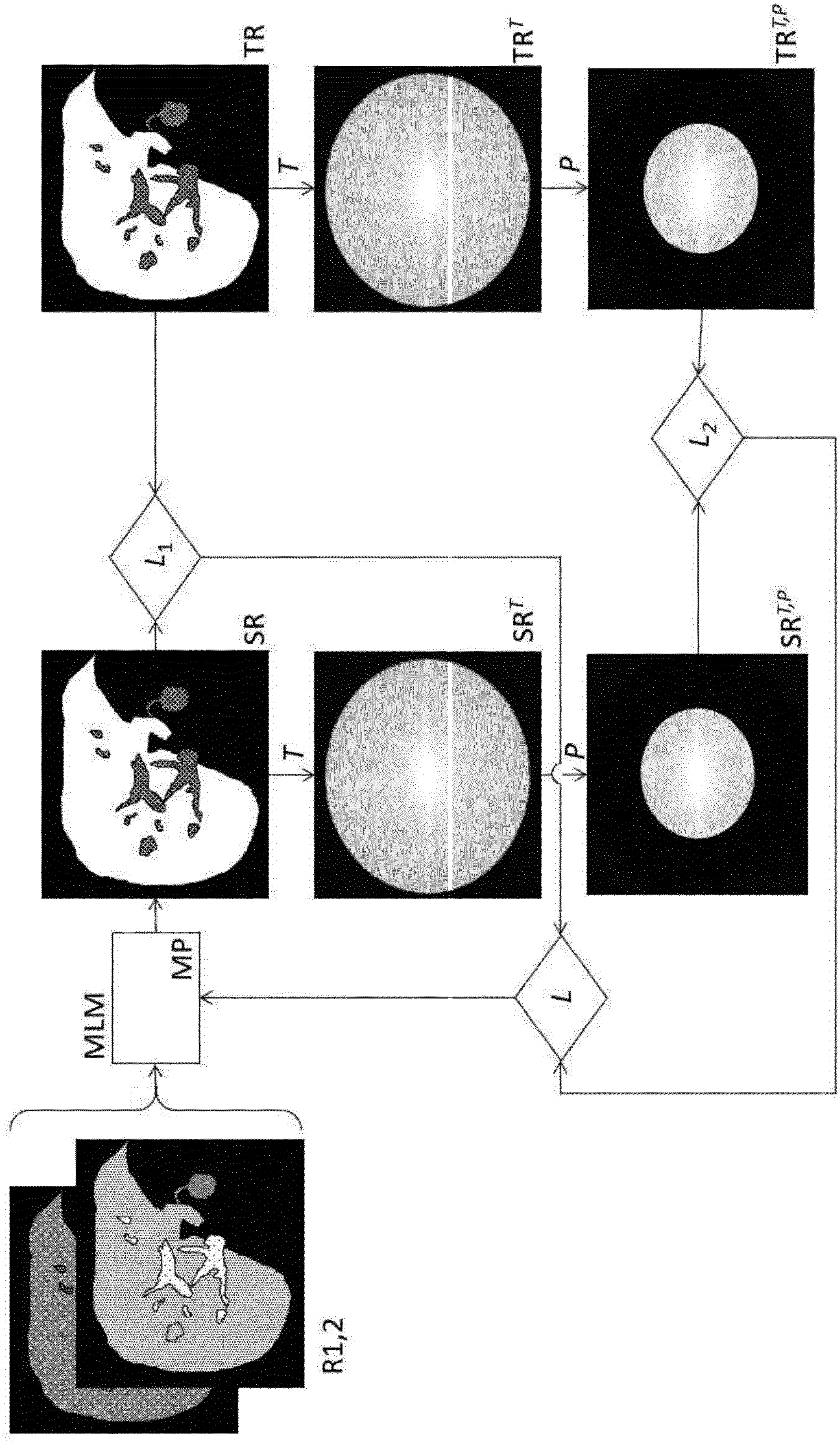


Fig. 8

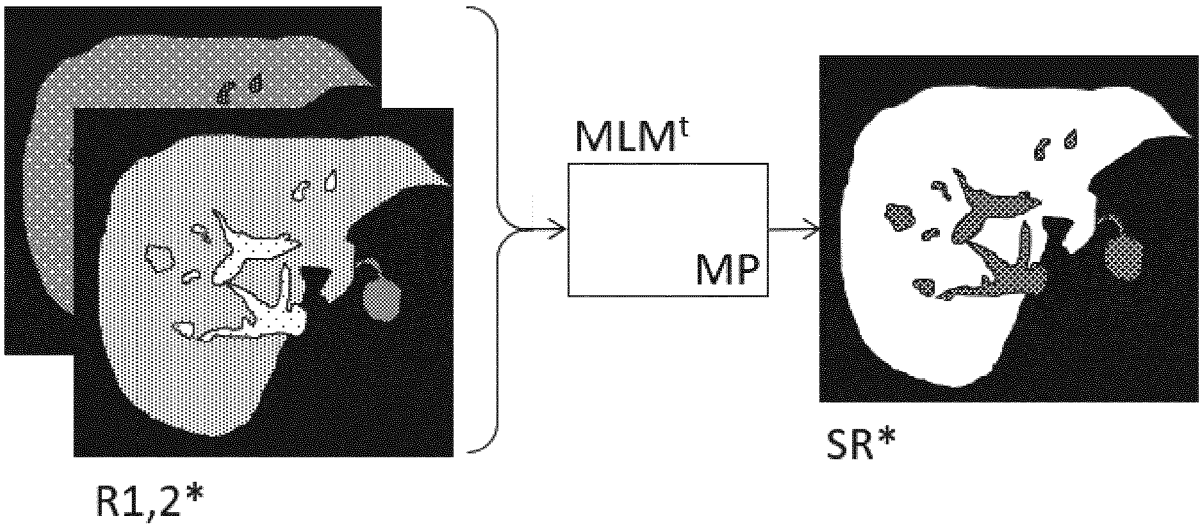


Fig. 9

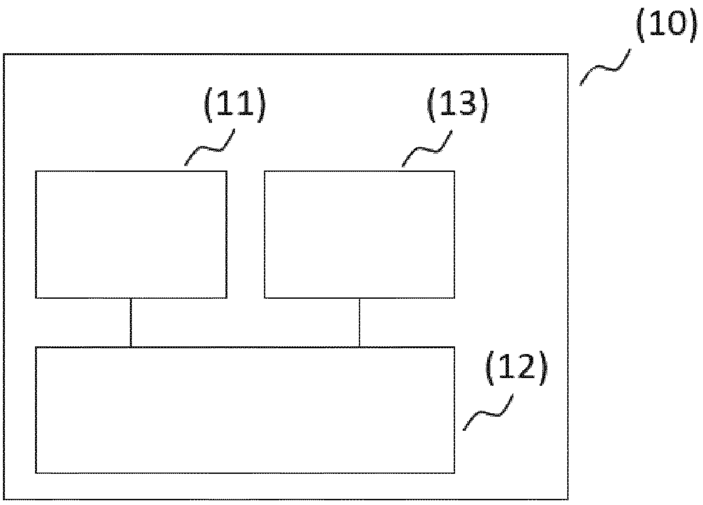


Fig. 10

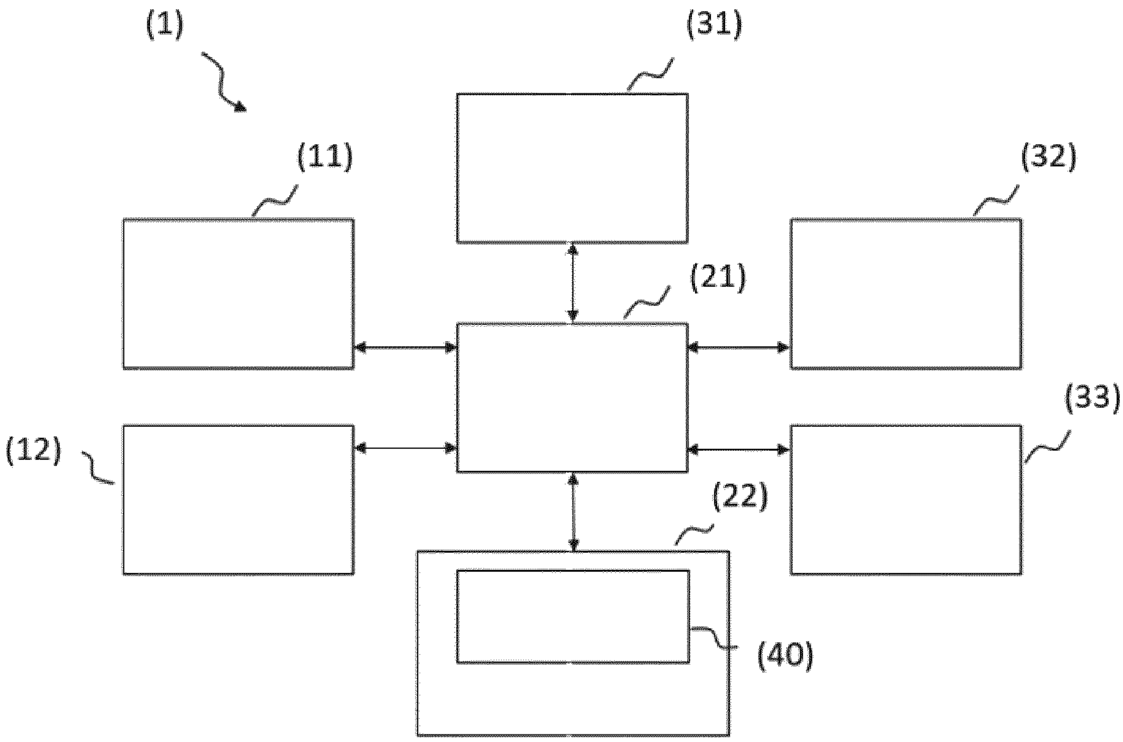


Fig. 11

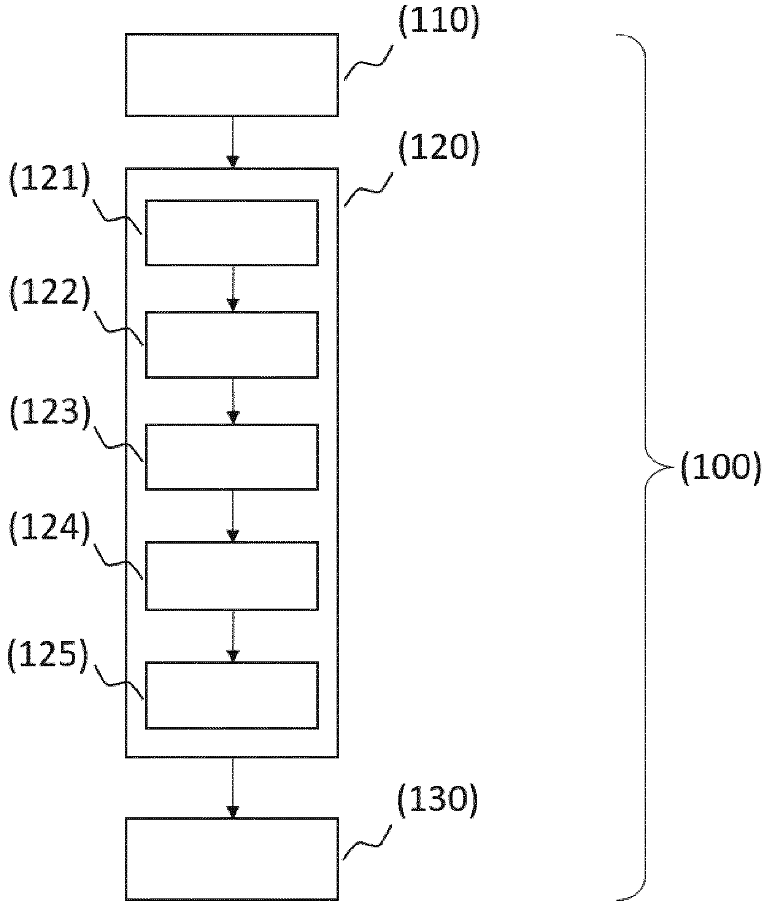


Fig. 12

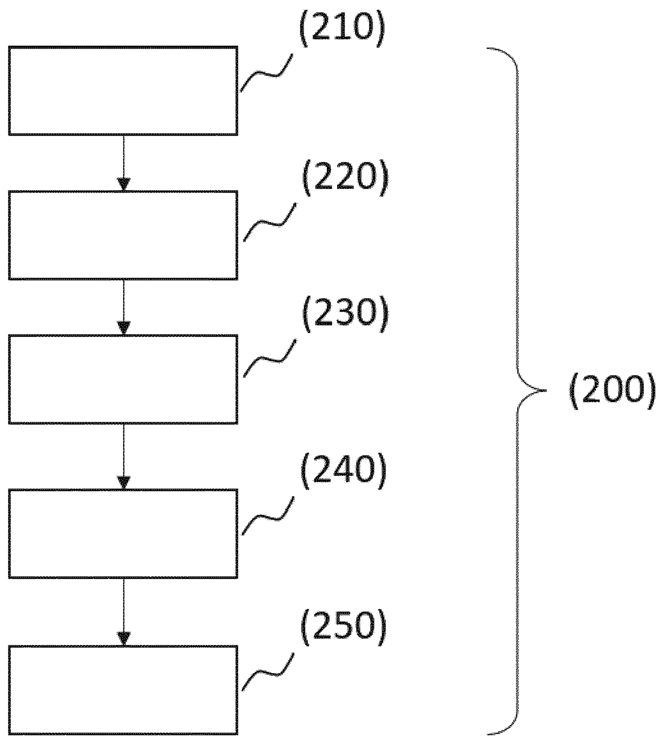


Fig. 13

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2021197996 A1
- US 10997716 B2
- EP 3875979 A1
- WO 2021052896 A1
- US 6039931 A
- WO 2007042504 A
- WO 2020030618 A
- WO 2022013454 A
- WO 2016193190 A
- WO 2022194777 A


### In der Beschreibung aufgeführte Nicht-Patentliteratur


- **K. SCHWARZ et al.** *On the Frequency Bias of Generative Models*, https://doi.org/10.48550/arXiv.2111.02447
- **A. S. L. JASCINTH et al.** Contrast Agents in computed tomography: A Review. *Journal of Applied Dental and Medical Sciences*, 2016, vol. 2 (2), 143-149
- **H. LUSIC et al.** X-ray-Computed Tomography Contrast Agents. *Chem. Rev.*, 2013, vol. 113 (3), 1641-1666, https://www.radiology.wisc.edu/wpcontent/uploads/2017/10/contrast-agents-tutorial.pdf
- **M. R. NOUGH et al.** Radiographie and magnetic resonances contrast agents: Essentials and tips for safe practices. *World J Radiol.*, 28 September 2017, vol. 9 (9), 339-349
- **L. C. ABONYI et al.** Intravascular Contrast Media in Radiography: Historical Development & Review of Risk Factors for Adverse Reactions. *South American Journal of Clinical Research*, 2016, vol. 3 (1), 1-10
- *ACR Manual on Contrast Media*, 2020, ISBN 978-1-55903-012-0
- **A. IGNEE et al.** Ultrasound contrast agents. *Endosc Ultrasound.*, November 2016, vol. 5 (6), 355-362
- **J. LOHRKE et al.** *Preclinical Profile of Gadoquatrane: A Novel Tetrameric, Macrocyclic High Relaxivity Gadolinium-Based Contrast Agent. Invest Radiol*, 2022, vol. 57 (10), 629-638
- *J. Magn. Reson. Imaging*, 2012, vol. 35 (3), 492-511
- *Clujul Medical*, 2015, vol. 88 (4), 438-448
- *Journal of Hepatology*, 2019, vol. 71, 534-542, http://dx.doi.org/10.1016/j.jhep.2019.05.005
- **R. MECHREZ et al.** The Contextual Loss for Image Transformation with Non-Aligned Data. *arXiv:1803.02077v4*, 2018
- **H. ZHAO et al.** Loss Functions for Image Restoration with Neural Networks. *arXiv:1511.08861v3*, 2018
- **D. FUOLI et al.** Fourier Space Losses for Efficient Perceptual Image Super-Resolution. *arXiv:2106.00783v1*
- U-net: Convolutional networks for biomedical image segmentation. **O. RONNEBERGER et al.** International Conference on Medical image computing and computer-assisted intervention. Springer, 2015, 234-241
- **M.-Y. LIU et al.** Generative Adversarial Networks for Image and Video Synthesis: Algorithms and Applications. *arXiv:2008.02793*
- **J. HENRY et al.** *Pix2Pix GAN for Image-to-Image Translation*
- **Q. LI et al.** RegGAN: An End-to-End Network for Building Footprint Generation with Boundary Regularization. *Remote Sens.*, 2022, vol. 14, 1835
- **P. ISOLA et al.** Image-to-Image Translation with Conditional Adversarial Networks. *arXiv:1611.07004 [cs.CV*
- **D. KARIMI et al.** Convolution-Free Medical Image Segmentation using Transformers. *arXiv:2102.13645 [eess.IV*